# EUROPEAN PATENT SPECIFICATION

(11) **EP 0 560 794 B1**
(45) Date of publication and mention of the grant of the patent: **18.09.1996**
(21) Application number: 91919496.9
(22) Date of filing: 12.11.1991
(51) Int. Cl.: C07D 411/04, C07D 473/02, A61K 31/505, C07F 7/18

(54) **SUBSTITUTED 1,3-OXATHIOLANES WITH ANTIVIRAL PROPERTIES**
SUBSTITUIERTE 1,3-OXATHIOLANE MIT ANTIVIRALEN EIGENSCHAFTEN
1,3-OXATHIOLANES SUBSTITUES AYANT DES PROPRIETES ANTIVIRALES

(30) Priority: 13.11.1990 US 612840
(43) Date of publication of application: 22.09.1993
(73) Proprietor: BIOCHEM PHARMA INC., Laval, Quebec H7V 4A7 (CA)
(72) Inventor: BELLEAU, Bernard, deceased (US); BRASILI, Livio, I-63023 Fermo (IT); NGUYEN-BA, Nghe, LaPrairie, Quebec J5R 5M5 (CA); LAVAL, Chan, Chun, Kong, Kirkland, Quebec H9J 3X8 (CA)
(74) Representative: Ritter, Stephen David
(86) International application number: CA9100407
(87) International publication number: WO9208717

(56) References cited:
- EP-A- 337 713
- EP-A- 0 382 526
- WO-A-88/08001

## Description

The present invention relates to novel substituted 1,3-oxathiolane compounds having pharmacological activity, to intermediates useful in their preparation, to pharmaceutical compositions containing them, and to the use of these compounds in the antiviral treatment of mammals.

Retroviral infections are a serious cause of disease, most notably, the acquired immunodeficiency syndrome (AIDS). The human immunodeficiency virus (HIV) has been recognized as the etiologic agent of AIDS. Compounds having an inhibitory effect on HIV multiplication or otherwise effective in the therapy of retroviral infections are being actively sought.

H. Mitsuya et al., "3'-Azido-3'-deoxythymidine (BW A509U): An antiviral agent that inhibits the infectivity and cytopathic effect of human T-lymphotropic virus type III/lymphadenopathy-associated virus in vitro", Proc. Nat. Acad. Sci. U.S.A., 82, pp. 7096-7100 (1985), refers to 3'-azido-2',3'-dideoxythymidine of formula (A), commonly referred to as AZT. This compound is said to be useful in providing some protection for AIDS carriers against the cytopathogenic effect of immunodeficiency virus (HIV).

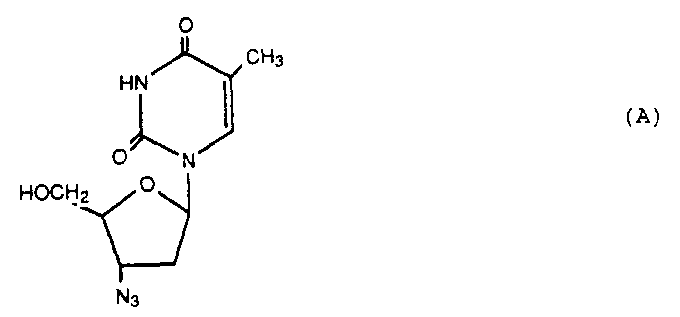

H. Mitsuya and S. Broder, "Inhibition of the in vitro infectivity and cytopathic effect of human T-lymphotrophic virus type III/lymphadenopathy-associated virus (HTLV-III/LAV) by 2',3'-dideoxynucleosides", Proc. Natl. Acad. Sci. U.S.A., 83, pp. 1911-15 (1986), have also referred to a group of 2',3'-dideoxynucleosides shown in formula (B) which are said to possess protective activity against HIV-induced cytopathogenicity.

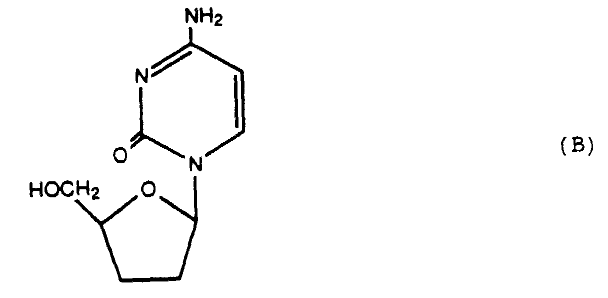

P. Herdewijn et al., "3'-Substituted 2',3'-dideoxynucleoside analogues as potential anti-HIV(HTLV-III/LAV) agents", J. Med. Chem., 30, pp. 1270-1278 (1987), describe the anti-HIV activity of a series of 3'-substituted nucleoside analogues. While 3'-fluoro analogues of 2',3'-dideoxythymidine and 2',3'-dideoxycytidine shown in formulas (C) and (D) are found to possess potent antiretroviral activity, substituents linked to the 3'-carbon via a thio or oxygen bridge did not yield active products.

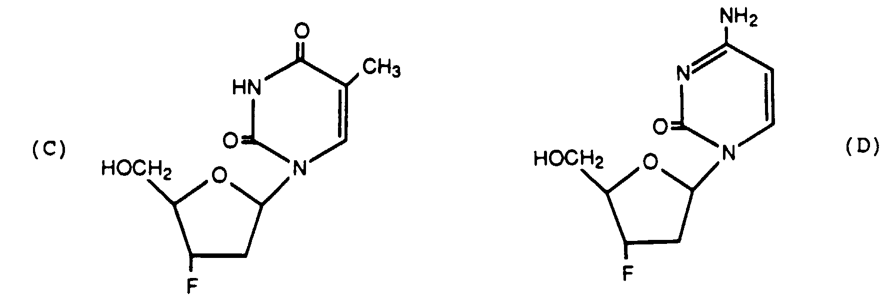

Analysis of molecular conformation studies in P. Van Roey et al., "Correlation between preferred sugar ring corformation and activity of nucleoside analogues against human immunodeficiency virus", Proc. Natl. Acad. Sci. U.S.A., 86(10), pp. 3929-3933 (1989), indicate that active anti-HIV nucleoside analogues have 3' carbon conformations on the side opposite to the base.

D. Huryn et al., "Synthesis of iso-ddA, member of a novel class of anti-HIV agents", Tetrahedron Lett., 30(46), pp. 6259-6262 (1989), refer to the iso-nucleoside analogue of formula (E) as a stable inhibitor of HIV replication.

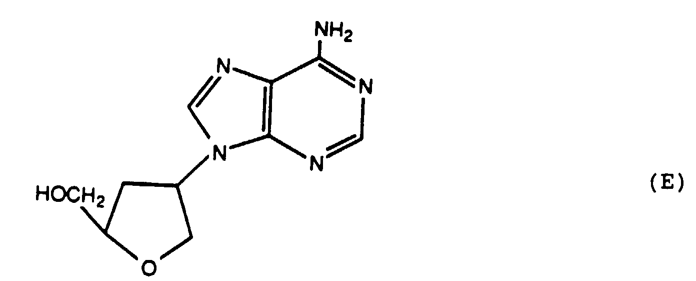

R. Vince and M. Hua, "Synthesis and anti-HIV activity of carbocyclic 2',3'-didehydro-2',3'-dideoxy 2,6-disubstituted purine nucleosides", J. Med. Chem., 33(1), pp. 17-21 (1990), describe the analogues shown in formulas (F) and (G) as having anti-HIV activity. The unsaturated analogue (F) shows greater selectivity and potency as an inhibitor of HIV replication than the saturated analog (G).

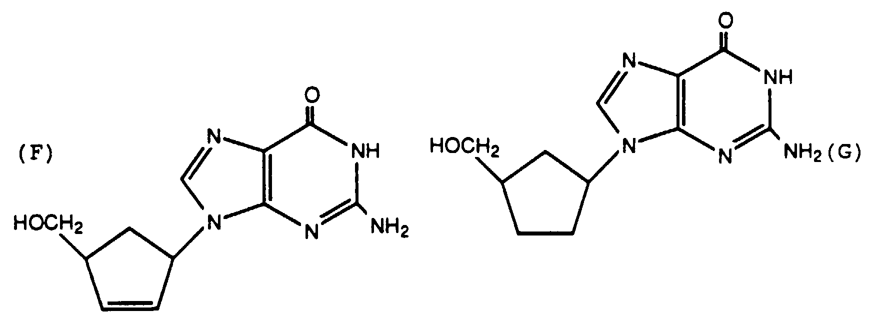

C. Chu et al., "Synthesis and structure-activity relationships of 6-substituted 2',3'-dideoxypurine nucleosides as potential anti-human immunodeficiency virus agents", J. Med. Chem., 33(6), pp. 1553-1561 (1990), describe the N₆-methyl derivative shown in formula (H) as having greater potency against HIV than unmethylated 2',3'-dideoxyadenosine.

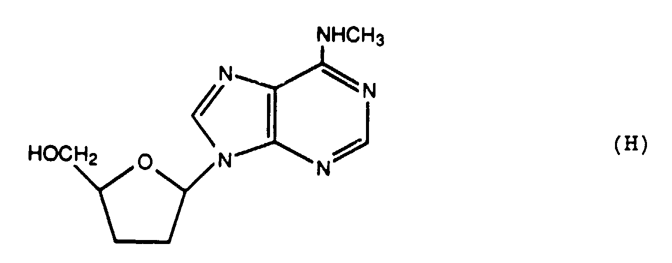

Finally, B. Belleau et al., "Design and activity of a novel class of nucleoside analogues effective against HIV-1", Abstracts of papers, Fifth International Conference on AIDS, Montreal, T.C.O. 1, p. 515 (1989), refer to dioxolanes and oxathiolanes of formulas (J) and (K) as having potent anti-HIV activity. These compounds are also the subject of a European patent application by Belleau et al. (EP-A-382 526). European patent application 337,713 also discloses dioxolanes similar to formula (J). PCT publication WO 88/08001 discloses that nucleoside analogues with an α-trans configuration can inhibit HIV multiplication.

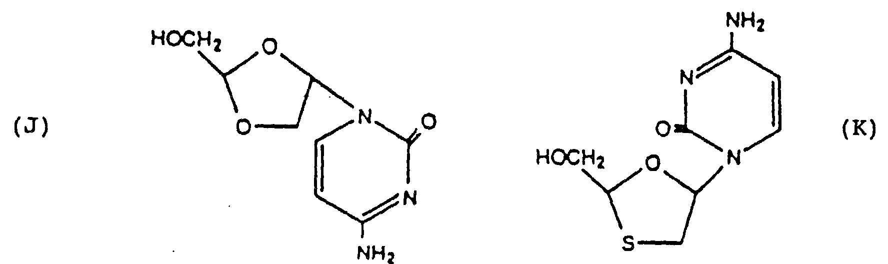

Despite these developments to date, and in view of the increasing incidence and life threatening characteristics of AIDS, there is a great need for the discovery and development of new potent and non-toxic inhibitors of HIV.

A structurally distinct class of compounds known as 2-substituted 4-substituted 1,3-oxathiolanes has been found to have potent antiretroviral activity. In particular, these compounds have been found to act as potent inhibitors of HIV-1 replication in T-lymphocytes over a prolonged period of time with less cytotoxic side effects than compounds known in the art. These compounds are also useful in prophylaxis and treatment of hepatitis B virus infections.

There are accordingly provided in a first aspect of this invention compounds of formula (I) 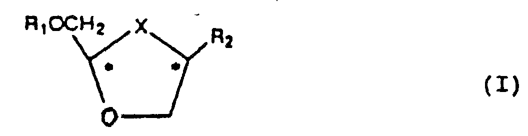
wherein X is S, S=O, or SO₂;
R₁ is hydrogen; and
R₂ is a purine or pyrimidine base or an analogue or derivative thereof as defined on pages 11 to 13; and pharmaceutically acceptable derivatives thereof.

It will be appreciated by those skilled in the art that the compounds of formula (I) contain at least two chiral centers (shown as * in formula (I)) and thus exist in the form of two pairs of optical isomers (i.e., enantiomers) and mixtures thereof including racemic mixtures. Thus the compounds of formula (I) may be either cis isomers, as represented by formula (II), or trans isomers, as represented by formula (III), or mixtures thereof. Each of the cis and trans isomers can exist as one of two enantiomers or as mixtures thereof including racemic mixtures. All such isomers and mixtures thereof including racemic mixtures are included within the scope of the invention.

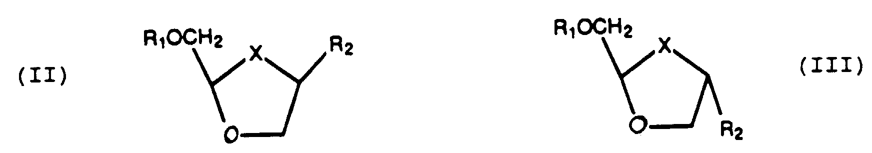

The compounds of formula (I) are preferably in the form of their cis isomers.

It will also be appreciated that when X is S=O , the compounds exist in two additional isomeric forms as shown in formulas (IIa) and (IIb). These isomers differ in the configuration of the oxide oxygen atom relative to the 2,4-substituent. 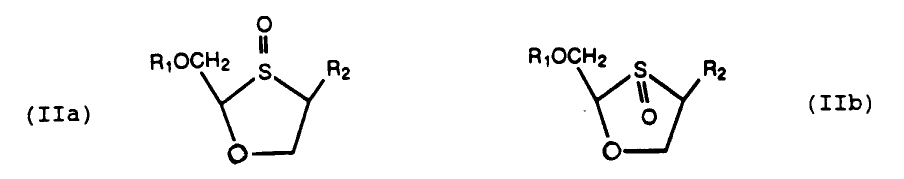 The compounds of the invention additionally embrace such isomers and mixtures thereof.

By purine or pyrimidine base or analogue or derivative thereof is generally meant a purine or pyrimidine base found in native nucleosides or an analogue thereof which mimics such bases in that their structures (the kinds of atoms and their arrangement) are similar to the native bases but may either possess additional or lack certain of the functional properties of the native bases. Such analogues include those derived by replacement of a CH moiety by a nitrogen atom (for example, 5-azapyrimidines such as 5-azacytosine) or vice verse (for example, 7-deazapurines, such as 7-deazadenine or 7 deazaguanine) or both (e.g., 7-deaza, 8-azapurines). By derivatives of such bases or analogues are meant those compounds wherein ring substituents are either incorporated, removed, or modified by conventional substituents known in the art, e.g., halogen, hydroxyl, amino, C₁₋₆ alkyl. Such purine or pyrimidine bases, analogues and derivatives will be well known to those skilled in the art.

Conveniently they are selected from: 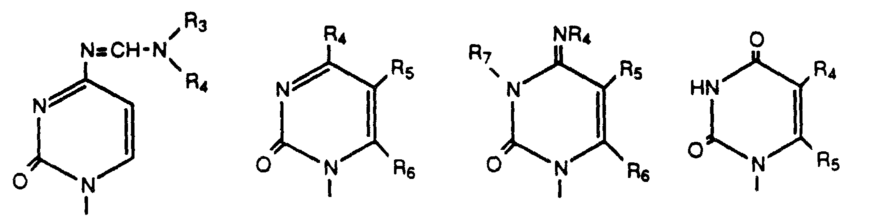 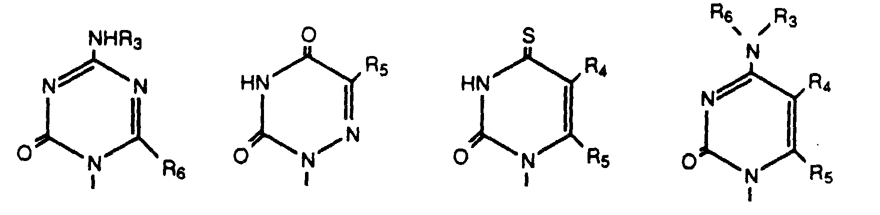 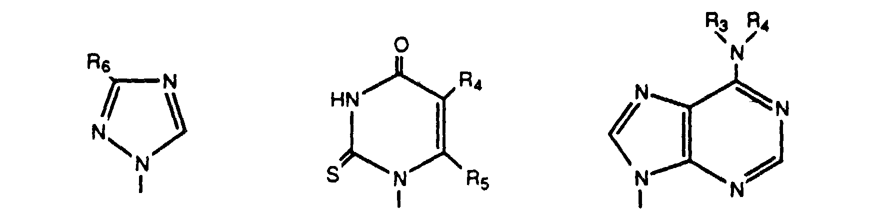 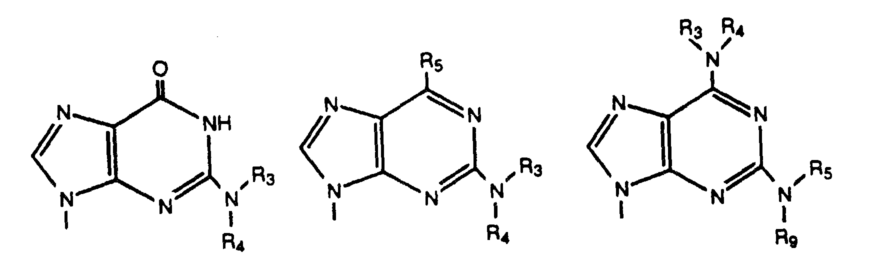 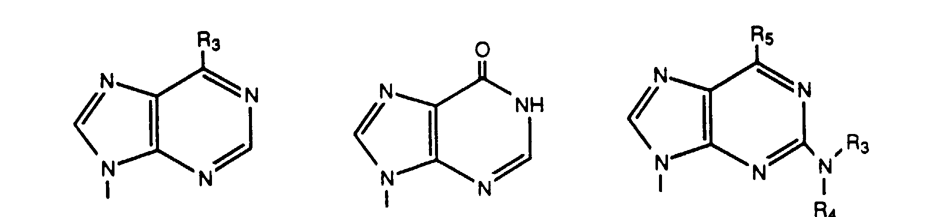 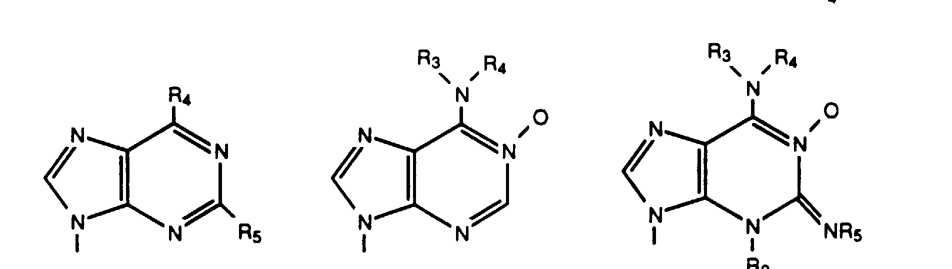 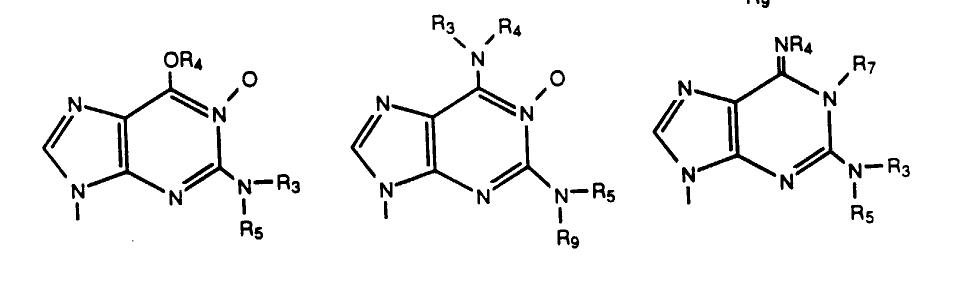 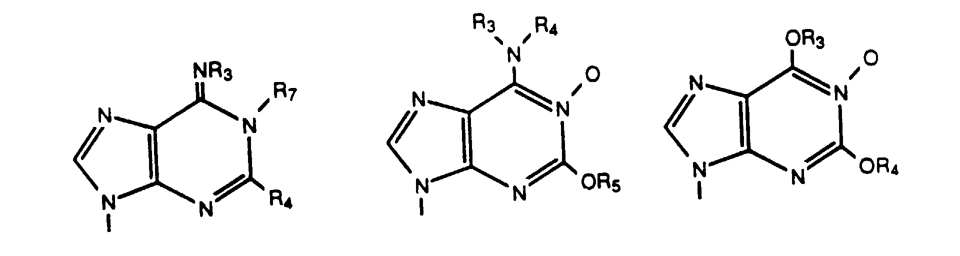 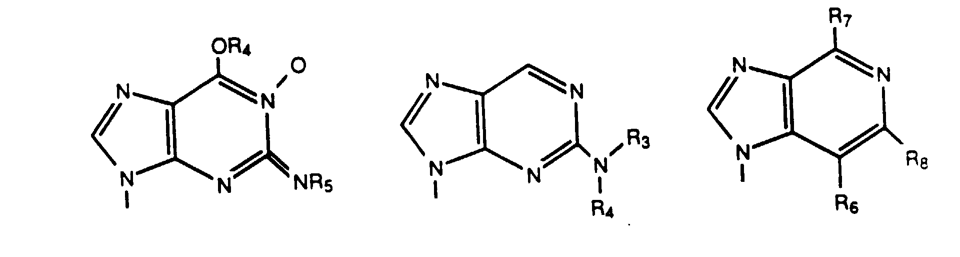 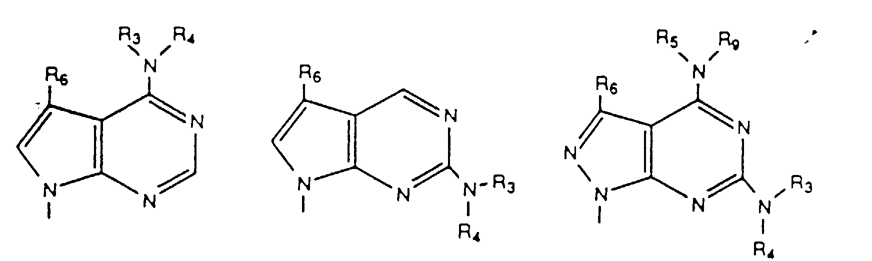 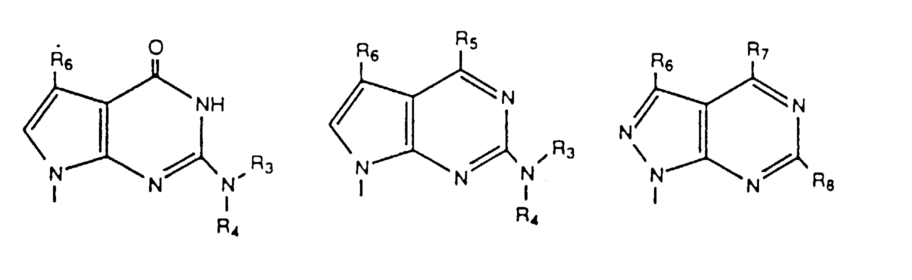 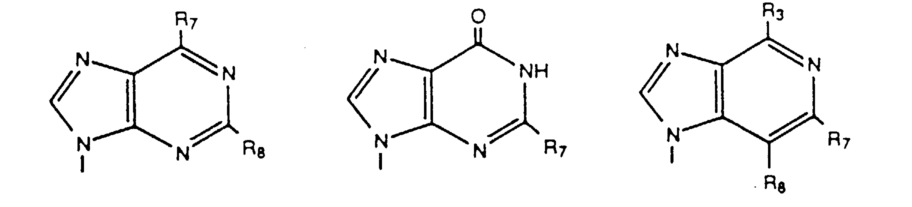
wherein R₃ is selected from the group of hydrogen, C₁₋₁₀ acyl (e.g., acetyl), hydroxyl, substituted or unsubstituted C₁₋₆ alkyl, and substituted or unsubstituted up to C₆ alkenyl or alkynyl (e.g. propynyl);
R₄ and R₅ are independently selected from the group of hydrogen, hydroxymethyl, trifluoromethyl, substituted or unsubstituted C₁₋₆ alkyl, substituted or unsubstituted up to C₆ alkenyl or alkynyl, bromine, chlorine, fluorine, iodine, and thioaryl;
R₆ is selected from the group of hydrogen, bromine, chlorine, fluorine, iodine, cyano, carboxy, carboxamide, ethoxycarbonyl, carbamoyl, and thiocarbamoyl;
R₇ and R₈ are independently selected from the group of hydrogen, bromine, chlorine, fluorine, iodine, substituted or unsubstituted amino, and hydroxy; and
R₉ is selected from the group of hydrogen, C₁₋₁₀ acyl, hydroxyl, substituted or unsubstituted C₁₋₆ alkyl, and substituted or unsubstituted up to C₆ alkenyl or alkynyl; and pharmaceutically acceptable derivatives.

The 2-substituted 4-substituted 1,3-oxathiolane of this invention is a compound of formula (I): 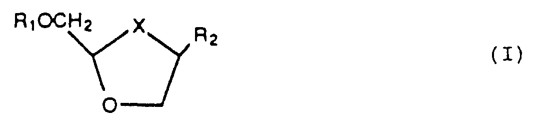
wherein X is S, S=O, or SO₂;
R₁ is hydrogen,; and
R₂ is a heterocyclic radical selected from the group consisting of: 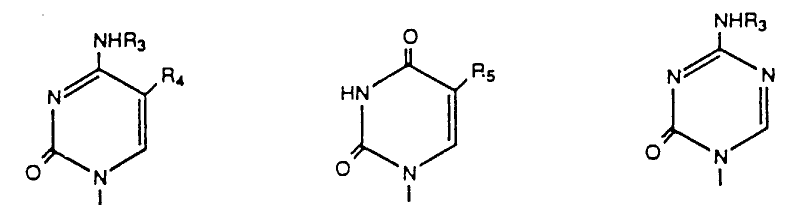 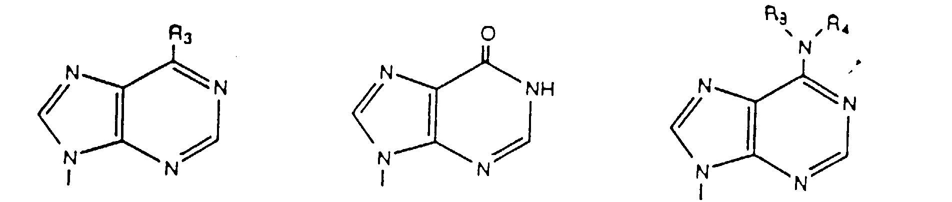 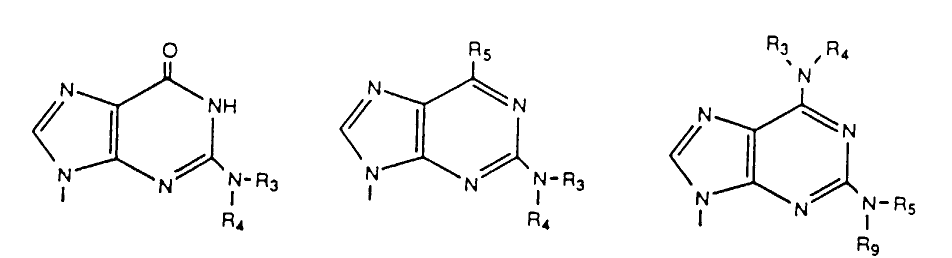 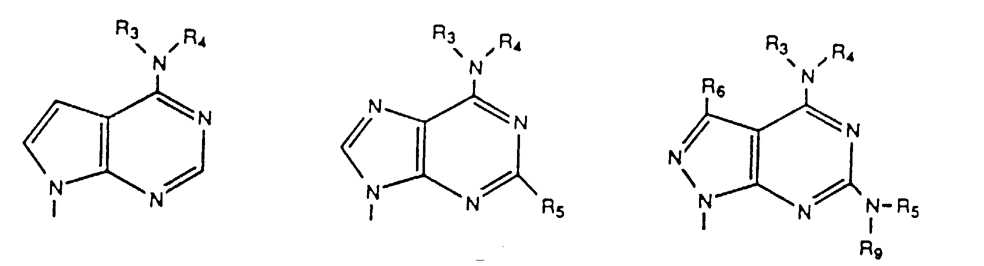 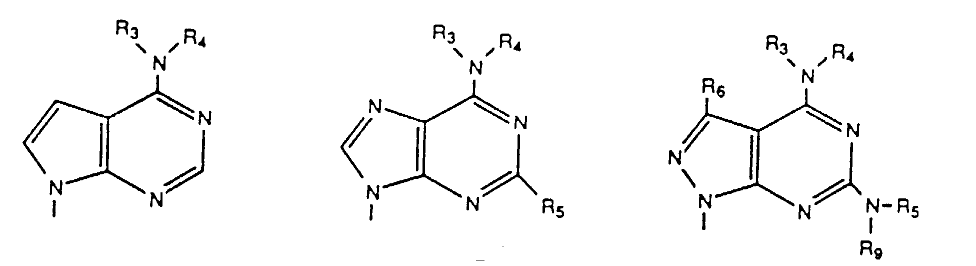 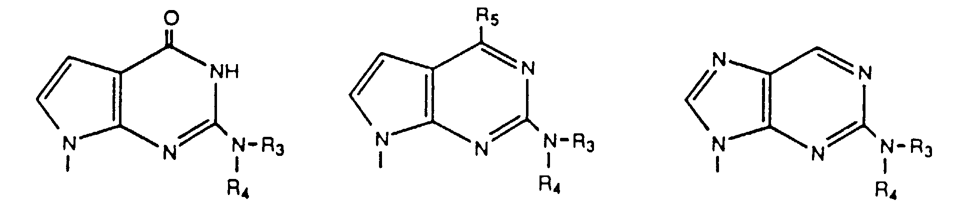 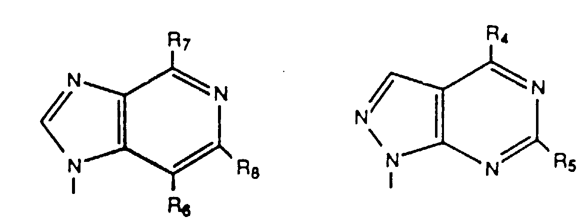
wherein R₃ is selected from the group of hydrogen, C₁₋₁₀ acyl (e.g., acetyl), hydroxyl, C₁₋₆ alkyl, and up to C₆ alkenyl or alkynyl (e.g. propynyl);
R₄ and R₅ are independently selected from the group of hydrogen, hydroxymethyl, trifluoromethyl, C₁₋₆ alkyl, up to C₆ alkenyl or alkynyl, bromine, chlorine, fluorine, and iodine;
R₆ is selected from the group of hydrogen, bromine, chlorine, fluorine, iodine, cyano, carboxy, carboxamide, ethoxycarbonyl, carbamoyl, and thiocarbamoyl;
R₇ and R₈ are independently selected from the group of hydrogen, bromine, chlorine, fluorine, iodine, amino, and hydroxy; and
R₉ is selected from the group of hydrogen, C₁₋₁₀ acyl, hydroxyl, C₁₋₆ alkyl, and up to C₆ alkenyl or alkynyl; and pharmaceutically acceptable derivatives.

As used herein, the term "acyl" refers to a radical derived from a carboxylic acid, substituted or unsubstituted, by replacement of the -OH group. Like the acid to which it is related, an acyl radical may be aliphatic or aromatic, substituted or unsubstituted, and whatever the structure of the rest of the molecule may be, the properties of the functional group remain essentially the same. The use of the term "aroyl" is meant to refer to acyl groups derived from aromatic acids and describes a preferred subset of the term "acyl". Other suitable acyl groups will include, for example: acetyl, propionyl, isobutanoyl, pivaloyl, hexanoyl, trifluoroacetyl, chloroacetyl, cyclohexanoyl, chlorobenzoyl, methoxybenzoyl, trifluoromethylbenzoyl, 1-naphthaloyl, 2-naphthaloyl, phenacyl, nitrobenzoyl, α-hydroxy-α-phenylacetyl, α-methoxy-α-phenylacetyl, aminoacetyl, α-amino-β-phenylpropionyl, and α-methoxy-α-(trifluoromethyl) phenacyl.

As used herein, "a pharmaceutically acceptable derivative" means any pharmaceutically acceptable salt, ester or salt of such ester, of a compound of formula (I) which, upon administration to the recipient, is capable of providing (directly or indirectly) a compound of formula (I) or an antivirally active metabolite or residue thereof.

Pharmaceutically acceptable salts of the compounds of formula (I) include those derived from pharmaceutically acceptable inorganic and organic acids and bases. Examples of suitable acids include hydrochloric, hydrobromic, sulfuric, nitric, perchloric, fumaric, maleic, phosphoric, glycollic, lactic, salicylic, succinic, toluene-p-sulfonic, tartaric, acetic, citric, methanesulfonic, formic, benzoic, malonic, naphthalene-2-sulfonic and benzenesulfonic acids. Other acids such as oxalic, while not in themselves pharmaceutically acceptable, may be useful in the preparation of salts useful as intermediates in obtaining the compounds of the invention and their pharmaceutically acceptable acid addition salts.

Salts derived from appropriate bases include alkali metal (e.g., sodium), alkaline earth metal (e.g., magnesium), ammonium and N-(C₁₋₄ alkyl)₄+ salts.

References hereinafter to a compound according to the invention include both compounds of formula (I) and their pharmaceutically acceptable derivatives.

It will be appreciated by those skilled in the art that the compounds of formula (I) may be modified to provide pharmaceutically acceptable derivatives thereof, at functional groups in both the base moiety, R₂, and at the hydroxymethyl group of the oxathiolane or dithiolane ring. Modification at all such functional groups is included within the scope of the invention. However, of particular interest are pharmaceutically acceptable derivatives (e.g., esters or esters of amino acids) obtained by modification of the 2-hydroxymethyl group of the oxathiolane or dithiolane ring.

Preferred esters of the compounds of formula (I) include the compounds in which R₁ is replaced by a carboxyl function 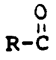 in which the non-carbonyl moiety R of the ester grouping is selected from hydrogen, straight or branched chain alkyl (e.g., methyl, ethyl, n-propyl, t-butyl, n-butyl), alkoxyalkyl (e.g., methoxymethyl), aralkyl (e.g., benzyl), aryloxyalkyl (e.g., phenoxymethyl), aryl (e.g., phenyl optionally substituted by halogen, C₁₋₄ alkyl or C₁₋₄ alkoxy); substituted dihydro pyridinyl (e.g., N-methyldihydro pyridinyl); sulphonate esters such as alkyl- or aralkylsulphonyl (e.g., methanesulphonyl); sulfate esters; amino acid esters (e.g., L-valyl or L-isoleucyl) and mono-, di- or tri-phosphate esters.

Also included within the scope of such esters are esters derived from polyfunctional acids such as carboxylic acids containing more than one carboxyl group, for example, dicarboxylic acids HO₂C(CH₂)ₙCO₂H where n is an integer of 1 to 10 (for example, succinic acid) or phosphoric acids. Methods for preparing such esters are well known. See, for example, E. Hahn et al., "Nucleotide dimers as anti-human immunodeficiency virus agents", Nucleotide Analogues As Antiviral Agents, J.C. Martin, Ed. Symposium Series #401, American Chemical Society, pp. 156-159 (1989) and M. Busso et al., "Nucleotide dimers suppress HIV expression in vitro", AIDS Research and Human Retroviruses, 4(6), pp. 449-455 (1988). Where esters are derived from such acids, each acidic group is preferably esterified by a compound of formula (I) or other nucleosides or analogues and derivatives thereof to provide esters of the formula (IV) where: 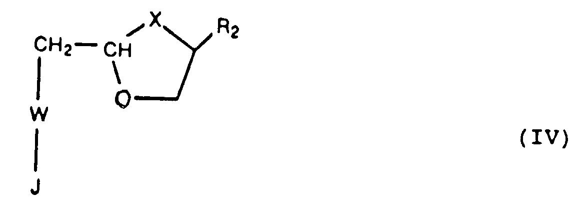 W is 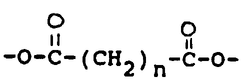 and n is an integer of 1 to 10 or 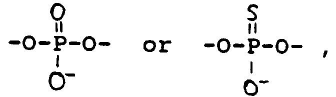 J is any nucleoside or nucleoside analogue or derivative thereof, and X and R₂ are as defined above. Among the preferred nucleosides and nucleoside analogues are 3'-azido-2',3'-dideoxythymidine, 2',3'-dideoxycytidine, 2',3'-dideoxyadenosine, 2',3'-dideoxyinosine, 2',3'-dideoxythymidine, 2',3'-dideoxy-2',3'-didehydrothymidine, and 2',3'-dideoxy-2',3'-didehydrocytidine and ribavirin and those nucleosides whose bases are depicted on pages 7-8 of this specification. The preferred ester of this invention is a homodimer consisting of two nucleosides of formula (I).

With regard to the above described esters, unless otherwise specified, any alkyl moiety present advantageously contains 1 to 16 carbon atoms, preferably 1 to 4 carbon atoms and could contain one or more double bonds. Any aryl moiety present in such esters advantageously comprises a phenyl group.

In particular the esters may be a C₁₋₁₆ alkyl ester, an unsubstituted benzoyl ester or a benzoyl ester substituted by at least one halogen (bromine, chlorine, fluorine or iodine), C₁₋₆ alkyl or alkenyl, saturated or unsaturated C₁₋₆ alkoxy, nitro or trifluoromethyl groups.

Specific compounds of formula (I) include:
cis-2-hydroxymethyl-4-(cytosin-1'-yl)-1,3-oxathiolane, trans-2-hydroxymethyl-4-(cytosin-1'-yl)-1,3-oxathiolane, and mixtures thereof;
cis-2-hydroxymethyl-4-(N₄*'*-acetyl-cytosin-1'-yl)-1,3-oxathiolane, trans-2-hydroxymethyl-4-(N₄'-acetylcytosin-1'-yl)-1,3-oxathiolane, and mixtures thereof;
cis-2-hydroxymethyl-4-(uracil-1'-yl)-1,3-oxathiolane, trans-2-hydroxymethyl-4-(uracil-1'-y1)-1,3-oxathiolane, and mixtures thereof;
cis-2-hydroxymethyl-4-(thymin-1'-yl)-1,3-oxathiolane, trans-2-hydroxymethyl-4-(thymin-1'-yl)-1,3-oxathiolane, and mixtures thereof;
cis-2-hydroxymethyl-4-(6'-chloropurin-9'-yl)-1,3-oxathiolane, trans-2-hydroxymethyl-4-(6'-chloropurin-9'yl)-1,3-oxathiolane, and mixtures thereof;
cis-2-hydroxymethyl-4-(adenin-9'-y1)-1,3-oxathiolane, trans-2-hydroxymethyl-4-(adenin-9'-yl)-1,3- oxathiolane, and mixtures thereof;
cis-2-hydroxymethyl-3-oxo-4-(adenin-9'-yl)-1,3-oxathiolane;
cis-2-hydroxymethyl-4-(6'-N-methylamino-purin-9'-yl)-1,3-oxathiolane;
cis-2-hydroxymithyl-4-(6'-N,N-dimethylamino-purin-9'-yl)-1,3-oxathiolane, trans-2-hydroxymethyl-4-(6'-N,N-dimethylamino-purin-9'-yl)-1,3-oxathiolane, and mixtures thereof;
cis-2-hydroxymethyl-4-(2'-amino-6'-chloro-purin-9'-yl)-1,3-oxathiolane, trans-2-hydroxymethyl-4-(2'-amino-6'-chloro-purin-9'-yl)-1,3-oxathiolane, and mixtures thereof;
cis-2-hydroxymethyl-4-(2',6'-diamino-purin-9'-yl)-1,3-oxathiolane, trans-2-hydroxymethyl-4-(2',6'-diamino-purin-9'-yl)-1,3-oxathiolane, and mixtures thereof;
cis-2-hydroxymethyl-4-(guanin-9'-yl)-1,3-oxathiolane;
and pharmaceutically acceptable derivatives thereof in the form of a racemic mixture or single enantiomer.

Preferred compounds of formula (I) are cis-2-hydroxymethyl-4-(adenin-9'-yl)-1,3-oxathiolane and pharmaceutically acceptable derivatives thereof in the form of a racemic mixture or single enantiomer.

In the processes for preparing the compounds of this invention, the following definitions are used:
R₂ is a purine or pyrimidine base or an analogue or derivative thereof as defined on pages 11 to 13;
R_{w} is hydrogen, trisubstituted silyl, C₁₋₆ alkyl, aralkyl such as benzyl or trityl, C₁₋₁₆ acyl, preferably a benzoyl or a benzoyl substituted in any position by at least one halogen (bromine, chlorine, fluorine or iodine), C₁₋₆ alkyl, C₁₋₆ alkoxy, nitro, or trifluoromethyl group;
Rₓ is C₁₋₆ alkyl; and
L is a "leaving group", i.e., an atom or group which is displaceable upon reaction with an appropriate base, with or without a Lewis acid. Suitable leaving groups include halogens such as iodine, bromine, chlorine, or fluorine; amido; azido; isocyanato; substituted or unsubstituted, saturated or unsaturated thiolates, such as thiomethyl or thiophenyl; substituted or unsubstituted, saturated or unsaturated selenino compounds, such as phenyl selenide or alkyl selenide; and substituted or unsubstituted, saturated or unsaturated aliphatic or aromatic ketones such as methyl ketone.

A suitable leaving group may also be -OR, where R is a substituted or unsubstituted, saturated or unsaturated alkyl group, e.g., C₁₋₆ alkyl or alkenyl group; a substituted or unsubstituted aliphatic or aromatic acyl group, e.g., a C₁₋₆ aliphatic acyl group such as acetyl and an aromatic acyl group such as benzoyl; a substituted or unsubstituted, saturated or unsaturated alkoxy or aryloxy carbonyl group, such as methyl carbonate and phenyl carbonate; substituted or unsubstituted sulphonyl imidazolide; substituted or unsubstituted aliphatic or aromatic amino carbonyl group, such as phenyl carbamate; substituted or unsubstituted alkyl imidate group such as trichloroacetamidate; substituted or unsubstituted, saturated or unsaturated phosphonates, such as diethylphosphonate; substituted or unsubstituted aliphatic or aromatic sulphonyl group, such as tosylate; or hydrogen.

Oxathiolane compounds of formula (I), 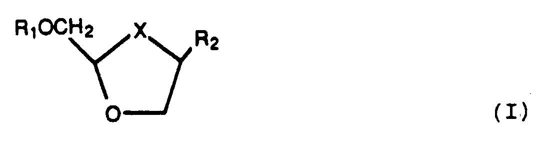 wherein X is S, S=O, or SO₂, and their pharmaceutically acceptable derivatives, may be prepared according to the processes discussed herein or by any method known in the art for the preparation of compounds of analogous structure.

In one such process for producing oxathiolanes of this invention, a compound of formula (V), 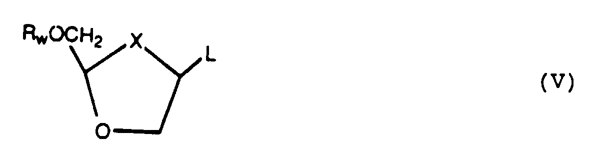 wherein R_{w} is hydrogen or a hydroxyl protecting group and L is a displaceable atom or group, i.e., a leaving group, is reacted with an appropriate base.

In a second process for producing oxathiolanes of this invention, a compound of formula (VI) 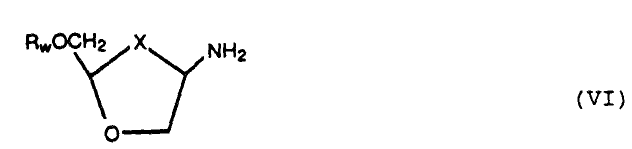 may be converted to a compound of formula (I) by conversion of the anomeric NH₂ group to the required base by methods well known in the art of nucleoside chemistry.

The 1,3-oxathiolanes of formula (I) may also be prepared, for example, by reaction of an aldehyde of formula (VII)

C₆H₅COOCH₂CHO (VII)

with 2-mercaptoethanol in a compatible organic solvent followed by Pummerer rearrangements as is know in the art (T. Durst, "Dimethylsuifoxide in Organic Synthesis", Adv. Org. Chem., E.C. Taylor and B. Wynberg, Eds., 6, pp. 356-365 (1969)) to give 1,3-oxathiolanes of formula (X), which are converted to 1,3-oxathiolanes of formula (I) by methods known in the art of nucleoside chemistry.

Another process for preparing the 1,3-oxathiolanes of formula (I) is illustrated in SCHEME 1. Although this process is illustrated using specific reagents and compounds, it will be appreciated by one of skill in the art that suitable analogous reactants may be used to prepare analogous products, as depicted, for example, in SCHEME 1A.

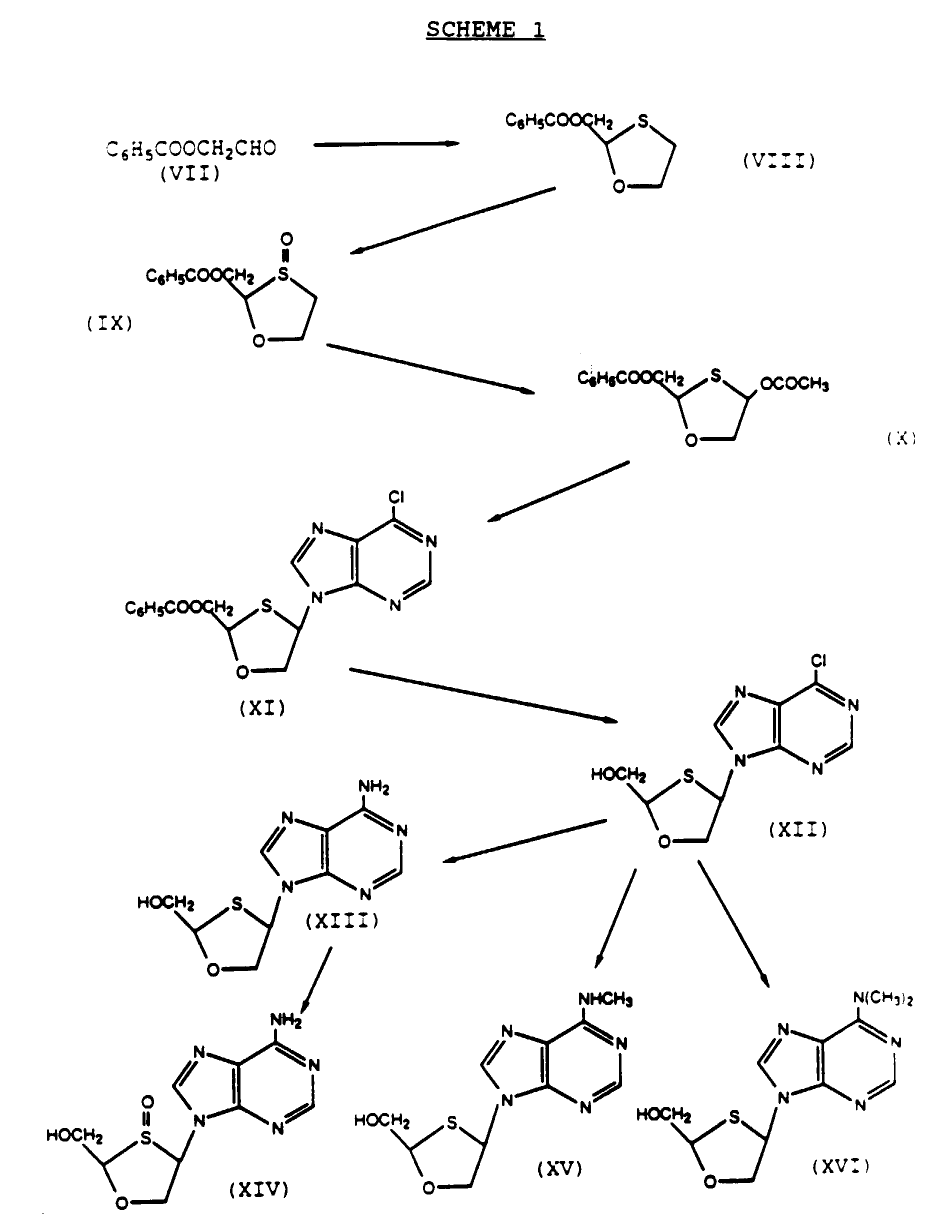

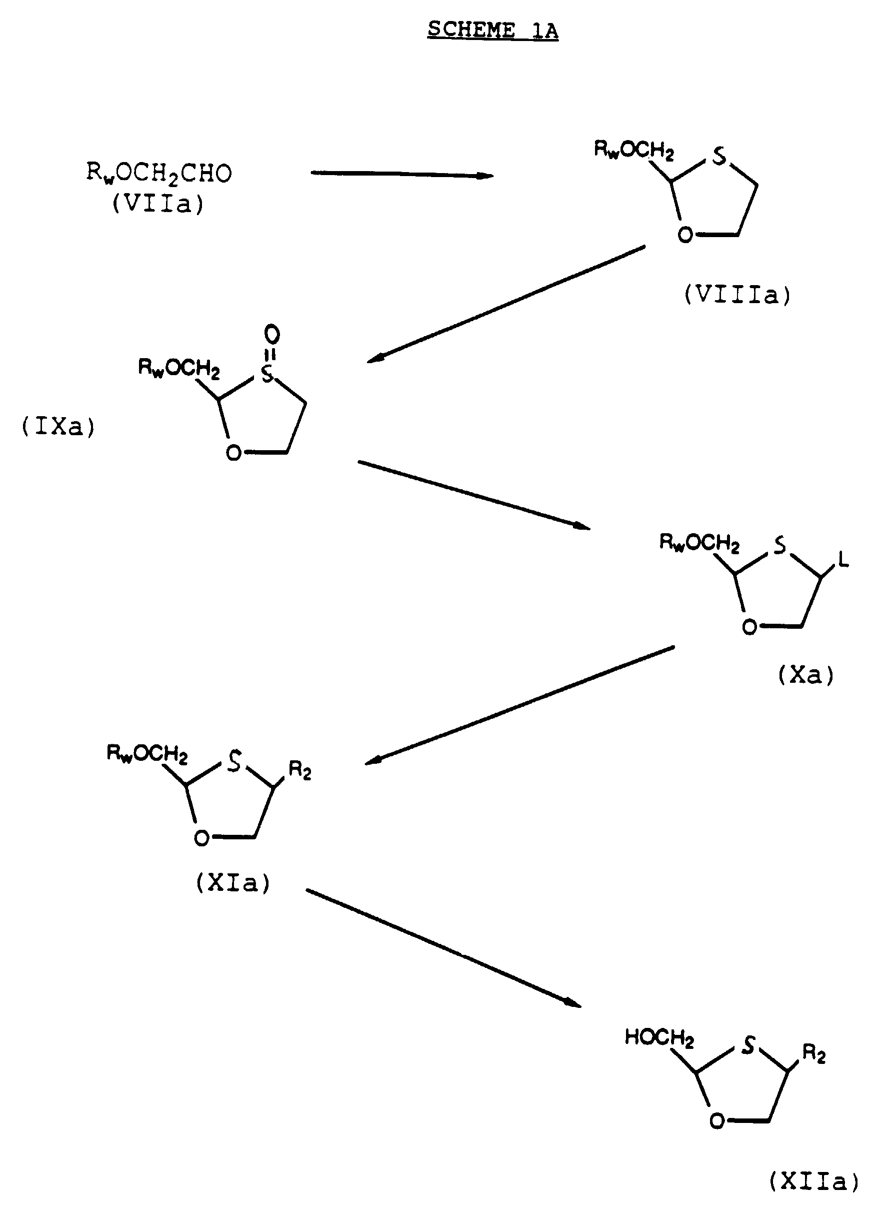

The various steps involved in the synthesis of 1,3-oxathiolanes of formula (I) as illustrated in SCHEME 1 may be briefly described as follows:

Step 1: Benzoyloxyacetaldehyde of formula (VII) or any aldehyde of the formula R_{w}OCH₂CHO (C.D. Hurd and E.M. Filiachione, "A new approach to the syntheses of aldehyde sugars", J. Am. Chem. Soc., 61, pp. 1156-1159 (1939)) is condensed with a mercaptoalcohol such as 2-mercaptoethanol in a compatible organic solvent, such as toluene, containing a catalytic amount of a strong acid to give the intermediate shown in formula (VIII).

Step 2: The 1,3-oxathiolane of formula (VIII) is then oxidized with a peracid such as magnesium monoperoxyphthalic acid in a compatible organic solvent such as methylene chloride containing a salt such as tetrabutyl ammonium bromide to give the sulfoxide intermediate shown in formula (IX).

Step 3: The sulfoxide intermediate shown in formula (IX) is treated with an acid anhydride such as acetic anhydride or any other anhydride of the formula (RₓCO)₂O in the presence of a buffer such as tetra-n-butylammonium acetate to give the 2,4-disubstituted-1,3-oxathiolane of formula (X) (T. Durst, Adv. Org. Chem., 6, pp. 356-365 (1969)).

Step 4: The 1,3-oxathiolane of formula (X) is then reacted with a pyrimidine or purine base or analogue thereof, (e.g., 6-chloropurine) previously silylated with, for example, hexamethyldisilazane in a compatible solvent using a Lewis acid or trimethylsilyl triflate to give the intermediate of formula (XI) as cis and trans isomers. The isomers may be separated, preferably by chromatography, to give pure cis (XI) and pure trans (XI).

Step 5: The benzoate function of the compound of formula (XI) (cis or trans isomer), is hydrolyzed using a base such as methanolic ammonia to obtain the compound shown in formula (XII) as cis- or trans- isomer.

Step 6: The chloro function of product of formula (XII) is displaced by methanolic ammonia preferably under pressure to give the product shown in formula (XIII) as a cis- or trans- isomer.

Step 7: The preceding isomers of formula (XIII) are treated with an oxidizing agent, e.g., a suitable peracid, in a compatible organic solvent to give the 3-oxide (sulfoxide) of formula (XIV).

Step 8: The chloro function of the compound of formula (XII) is displaced by ethanolic methylamine, preferably under pressure, to give the product shown in formula (XV) as a cis- or trans- isomer.

Step 9: The chloro function of the compound of formula (XII) is displaced by ethanolic dimethylamine, preferably under pressure, to give the product shown in formula (XVI) as a cis- or trans- isomer.

Many of the reactions in the above-described processes have been extensively reported in the context of purine nucleoside synthesis, for example, in L.B. Townsend, "Chemistry of the heterocyclic moiety of purine nucleosides and some closely related analogues", Nucleoside Analogues - Chemistry, Biology, and Medical Applications, R.T. Walker et al., Eds., Plenum Press, New York (1979) at pages 193-223, the text of which is incorporated by reference herein.

It will be appreciated that the reactions of the above-described processes may require the use of, or conveniently may be applied to, starting materials having protected functional groups, and deprotection might thus be required as an intermediate or final step to yield the desired compound. Protection and deprotection of functional groups may be effected using conventional means. Thus, for example, amino groups may be protected by a group selected from aralkyl (e.g., benzyl), acyl or aryl (e.g., 2,4-dinitrophenyl); subsequent removal of the protecting group being effected when desired by hydrolysis or hydrogenolysis as appropriate using standard conditions. Hydroxyl groups may be protected using any conventional hydroxyl protecting group, for example, as described in "Protective Groups in Organic Chemistry", Ed. J.F.W. McOmie (Plenum Press, 1973) or "Protective Groups in Organic Synthesis" by Theodora W. Greene (John Wiley and Sons, 1981). Examples of suitable hydroxyl protecting groups include groups selected from alkyl (e.g., methyl, t-butyl or methoxymethyl), aralkyl (e.g., benzyl, diphenylmethyl or triphenylmethyl), heterocyclic groups such as tetrahydropyranyl, acyl, (e.g., acetyl or benzoyl) and silyl groups such as trialkylsilyl (e.g., t-butyldimethylsilyl). The hydroxyl protecting groups may be removed by conventional techniques. Thus, for example, alkyl, silyl, acyl and heterocyclic groups may be removed by solvolysis, e.g., by hydrolysis under acidic or basic conditions. Aralkyl groups such as triphenylmethyl may similarly be removed by solvolysis, e.g., by hydrolysis under acidic conditions. Aralkyl groups such as benzyl may be cleaved, for example, by treatment with BF₃/etherate and acetic anhydride followed by removal of acetate groups so formed at an appropriate stage in the synthesis. Silyl groups may also conveniently be removed using a source of fluoride ions such as tetra-n-butylammonium fluoride.

In the above-described processes, the compounds of formula (I) are generally obtained as a mixture of the cis and trans isomers.

These isomers may be separated, for example, by acetylation, e.g., with acetic anhydride followed by separation by physical means, e.g., chromatography on silica gel and deacetylation, e.g., with methanolic ammonia or by fractional crystallization.

Pharmaceutically acceptable salts of the compounds of the invention may be prepared as described in United States Patent No. 4,383,114, the disclosure of which is incorporated by reference herein. Thus, for example, when it is desired to prepare an acid addition salt of a compound of formula (I), the product of any of the above procedures may be converted into a salt by treatment of the resulting free base with a suitable acid using conventional methods. Pharmaceutically acceptable acid addition salts may be prepared by reacting the free base with an appropriate acid optionally in the presence of a suitable solvent such as an ester (e.g., ethyl acetate) or an alcohol (e.g., methanol, ethanol or isopropanol). Inorganic basic salts may be prepared by reacting the free base with a suitable base such as an alkoxide (e.g., sodium methoxide) optionally in the presence of a solvent such as an alcohol (e.g., methanol). Pharmaceutically acceptable salts may also be prepared from other salts, including other pharmaceutically acceptable salts, of the compounds of formula (I) using conventional methods.

A compound of formula (I) may be converted into a pharmaceutically acceptable phosphate or other ester by reaction with a phosphorylating agent, such as POCl₃, or a suitable esterifying agent, such as an acid halide or anhydride, as appropriate. An ester or salt of a compound of formula (I) may be converted to the parent compound, for example, by hydrolysis.

Where the compound of formula (I) is desired as a single isomer it may be obtained either by resolution of the final product or by stereospecific synthesis from isometrically pure starting material or any convenient intermediate.

Resolution of the final product, or an intermediate or starting material therefore may be effected by any suitable method known in the art: see for example, Stereochemistry of Carbon Compounds, by E.L. Eliel (McGraw Hill, 1962) and Tables of Resolving Agents, by S.H. Wilen.

The intermediates of formulas (Ic) and (Id) are useful in the above-described processes for making the oxathiolane compounds of this invention 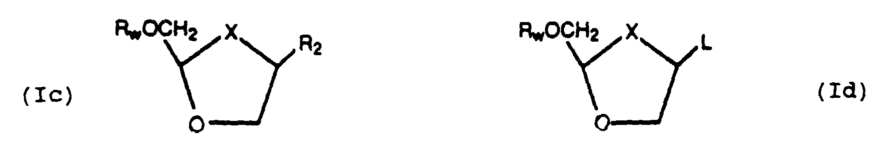
wherein R_{w} is trisubstituted silyl, C₁₋₆ alkyl, aralkyl such as benzyl or trityl, C₁₋₁₆ acyl, preferably a benzoyl or a benzoyl substituted in any position by at least one halogen (bromine, chlorine, fluorine or iodine), C₁₋₆ alkyl, C₁₋₆ alkoxy, nitro, or trifluoromethyl group; and
R₂ is a purine or pyrimidine base or an analogue or derivative thereof as defined on pages 11 to 13; and
L is a leaving group as previously defined.

The following intermediates of formula (Ic) are of particular importance:
cis-2-benzoyloxymethyl-4-(cytosin-1'-yl)-1,3-oxathiolane, trans-2-benzoyloxymethyl-4-(cytosin-1'-yl)-1,3-oxathiolane, and mixtures thereof;
cis-2-benzoyloxymethyl-4- (N₄'-acetyl-cytosin-1'-yl)-1,3-oxathiolane, trans-2-benzoyloxymethyl-4-(N₄'-acetyl-cytosin-1'-yl)-1,3-oxathiolane, and mixtures thereof;
cis-2-benzoyloxymethyl-4-(uracil-1'-yl)-1,3-oxathiolane, trans-2-benzoyloxymethyl-4-(uracil-1'-yl)-1,3-oxathiolane, and mixtures thereof;
cis-2-benzoyloxymethyl-4-(thymin-1'-yl)-1,3-oxathiolane, trans-2-benzoyloxymethyl-4-(thymin-1'-yl)-1,3-oxathiolane, and mixtures thereof;
cis-2-benzoyloxymethyl-4-(6'-chloropurin-9'-yl)-1,3-oxathiolane, trans-2-benzoyloxymethyl-4-(6'-chloropurin9'-yl)-1,'3-oxathiolane, and mixtures thereof;
cis-2-benzoyloxymethyl-4-(adenin-9'-yl)-1,3-oxathiolane, trans-2-benzoyloxymethyl-4-(adenin-9'-yl)-1,3-oxathiolane;
cis-2-benzoyloxymethyl-4-(2'-amino-6'-chloro-purin-9'-yl)-1,3-oxathiolane, trans-2-benzoyloxymethyl-4-(2'-amino-6'-chloro-purin-9'-yl)-1,3-oxathiolane, and mixtures thereof;

The following intermediates of formula (Id) are of particular importance:
2-benzoyloxymethyl-1,3-oxathiolane;
cis-2-benzoylaxymethyl-1-oxo-1,3-oxathiolane, trans-2-benzoyloxymethyl-1-oxo-1,3-oxathiolane, and mixtures thereof;
cis-2-benzoyloxymethyl-4-acetoxy-1,3-oxathiolane, trans-2-benzoyloxymethyl-4-acetoxy-1,3-oxathiolane, and mixtures thereof;

The compounds of the invention either themselves possess antiviral activity and/or are metabolizable to such compounds. In particular these compounds are effective in inhibiting the replication of hepatitis B virus and retroviruses, including human retroviruses such as human immunodeficiency viruses (HIV's), the causative agents of AIDS.

There is thus provided as a further aspect of the invention a compound formula (I) or a pharmaceutically acceptable derivative thereof for use as an active therapeutic agent in particular as an antiviral agent, for example in the treatment of hepatitis B viral and retroviral infections.

In a further or alternative aspect there is provided a method for the treatment of a viral infection, in particular an infection caused by hepatitis B virus or a retrovirus such as HIV, in a mammal, including man, comprising administration of an effective amount of an antiviral compound of formula (I) or a pharmaceutically acceptable derivative thereof.

There is also provided in a further or alternative aspect of this invention, use of a compound of formula (I) or a pharmaceutically acceptable derivative thereof for the manufacture of a medicament for the treatment of a viral infection.

The compounds of the invention are also useful in the treatment of AIDS-related conditions such as AIDS-related complex (ARC), persistent generalized lymphadenopathy (PGL), AIDS-related neurological conditions (such as dementia), anti-HIV antibody-positive and HIV-positive conditions, Kaposi's sarcoma, thrombocytopenia purpura and opportunistic infections.

The compounds of the invention are also useful in the prevention or progression to clinical illness of individuals who are anti-HIV antibody or HIV-antigen positive and in prophylaxis following exposure to HIV.

The compounds of formula (I) or the pahrmaceutically acceptable derivatives thereof, may also be used for the prevention of viral contamination of biological fluids such as blood or semen in vitro.

Certain of the compounds of formula (I) are also useful as intermediates in the preparation of other compounds of the invention.

It will be appreciated by those skilled in the art that references herein to treatment extends to prophylaxis as well as the treatment of established infections or symptoms.

It will be further appreciated that the amount of a compound of the invention required for use in treatment will vary not only with the particular compound selected but also with the route of administration, the nature of the condition being treated and the age and condition of the patient and will be ultimately at the discretion of the attendant physician or veterinarian. In general, however, a suitable dose will be in the range from about 1 to about 750 mg/kg of body weight per day, such as 3 to about 120 mg per kilogram body weight of the recipient per day, preferably in the range of 6 to 90 mg/kg/day, most preferably in the range of 15 to 60 mg/kg/day.

The desired dose may conveniently be presented in a single dose or as divided doses administered at appropriate intervals, for example as two, three, four or more sub-doses per day.

The compound is conveniently administered in unit dosage form; for example containing 10 to 1500 mg, conveniently 20 to 1000 mg, most conveniently 50 to 700 mg of active ingredient per unit dosage form.

Ideally the active ingredient should be administered to achieve peak plasma concentrations of the active compound of from about 1 to 75 µM, preferably about 2 to 50 µM, most preferably about 3 to about 30 µM. This may be achieved, for example, by the intravenous injection of a 0.1 to 5% solution of the active ingredient, optionally in saline, or administered as a bolus containing about 0.1 to about 110 mg/kg of the active ingredient. Desirable blood levels may be maintained by a continuous infusion to provide about 0.01 to about 5.0 mg/kg/hour or by intermittent infusions containing about 0.4 to about 15 mg/kg of the active ingredient.

While it is possible that, for use in therapy, a compound of the invention may be administered as the raw chemical it is preferable to present the active ingredient as a pharmaceutical formulation.

The invention thus further provides a pharmaceutical formulation comprising a compound of formula (I) or a pharmaceutically acceptable derivative thereof together with one or more pharmaceutically acceptable carriers thereof and, optionally, other therapeutic and/or prophylactic ingredients. The carrier(s) must be "acceptable" in the sense of being compatible with the other ingredients of the formulation and not deleterious to the recipient thereof.

Pharmaceutical formulations include those suitable for oral, rectal, nasal, topical (including buccal and sub-lingual), vaginal or parenteral (including intramuscular, sub-cutaneous and intravenous) administration or in a form suitable for administration by inhalation or insufflation. The formulations may, where appropriate, be conveniently presented in discrete dosage units and may be prepared by any of the methods well known in the art of pharmacy. All methods include the step of bringing into association the active compound with liquid carriers or finely divided solid carriers or both and then, if necessary, shaping the product into the desired formulation.

Pharmaceutical formulations suitable for oral administration may conveniently be presented as discrete units such as capsules, cachets or tablets each containing a predetermined amount of the active ingredient; as a powder or granules; as a solution; as a suspension; or as an emulsion. The active ingredient may also be presented as a bolus, electuary or paste. 5 Tablets and capsules for oral administration may contain conventional excipients such as binding agents, fillers, lubricants, disintegrants, or wetting agents. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, emulsifying agents, non-aqueous vehicles (which may include edible oils) or preservatives.

The compounds according to the invention may also be formulated for parenteral administration (e.g., by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulatory agents such as suspending, stabilizing and/or dispersing agents. Alternatively, the active ingredient may be in powder form, obtained by aseptic isolation of sterile solid or by lyophilization from solution, for constitution with a suitable vehicle, e.g., sterile, pyrogen-free water, before use.

For topical administration to the epidermis, the compounds according to the invention may be formulated as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents.

Formulations suitable for topical administration in the mouth include lozenges comprising active ingredient in a flavored based, usually sucrose and acacia or tragacanth; pastilles comprising the active ingredient in an inert base such as gelatin and glycerin or sucrose and acacia; and mouthwashes comprising the active ingredient in a suitable liquid carrier.

Pharmaceutically formulations suitable for rectal administration wherein the carrier is a solid, are most preferably represented as unit dose suppositories. Suitable carriers include cocoa butter and other materials commonly used in the art, and the suppositories may be conveniently formed by admixture of the active compound with the softened or melted carrier(s) followed by chilling and shaping in molds.

Formulations suitable for vaginal administration may be presented as pessaries, tampons, creams, gels, pastes, foams or sprays containing in addition to the active ingredient, such carriers as are known in the art to be appropriate.

For intra-nasal administration the compounds of the invention may be used as a liquid spray or dispersible powder or in the form of drops.

Drops may be formulated with an aqueous or non-aqueous base also comprising one or more dispersing agents, solubilizing agents or suspending agents. Liquid sprays are conveniently delivered from pressurized packs.

For administration by inhalation, the compounds according to the invention are conveniently delivered from an insufflator, nebulizer or a pressurized pack or other convenient means of delivering an aerosol spray. Pressurized packs may comprise a suitable propellant such as dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount.

Alternatively, for administration by inhalation or insufflation, the compounds according to the invention may take the form of a dry powder composition, for example a powder mix of the compound and a suitable powder base such as lactose or starch. The powder composition may be presented in unit dosage form in, for example, capsules or cartridges or, e.g., gelatin or blister packs from which the powder may be administered with the aid of an inhalator or insufflator.

When desired, the above described formulations adapted to give sustained release of the active ingredient, may be employed.

The pharmaceutical compositions according to the invention may also contain other active ingredients such as antimicrobial agents, or preservatives.

The compounds of the invention may also be used in combination with other therapeutic agents, for example, other anti-infective agents. In particular the compounds of the invention may be employed together with known antiviral agents.

The invention thus provides, in a further aspect, a combination comprising a compound of formula (I) or a physiologically acceptable derivative thereof together with another therapeutically active agent, in particular, an antiviral agent.

The combinations referred to above may conveniently be presented for use in the form of a pharmaceutical formulation and thus pharmaceutical formulations comprising a combination as defined above together with a pharmaceutically acceptable carrier thereof comprise a further aspect of the invention.

Suitable therapeutic agents for use in such combinations include acyclic nucleosides such as acyclovir, ganciclovir, interferons such as alpha-, beta-and gamma-interferon; glucuronation inhibitors such as probenecid; nucleoside transport inhibitors such as dipyridamole; nucleoside analogues such as 3'-azido-2',3'-dideoxythymidine, 2',3'-dideoxycytidine, 2',3'-dideoxyadenosine, 2',3'-dideoxyinosine, 2',3'-dideoxythymidine, 2',3'-dideoxy-2',3'-didehydro-thymidine, and 2',3'-dideoxy-2',3'-didehydrocytidine and ribavirin; immunomodulators such as interleukin II (IL2) and granulocyte macrophage colony stimulating factor (GM-CSF), erythropoietin, ampligen, thymomodulin, thymopentin, foscarnet, glycosylation inhibitors such as 2-deoxy-D-glucose, castanospermine, 1-deoxynojirimycin; and inhibitors of HIV binding to CD4 receptors such as soluble CD4, CD4 fragments and CD4-hybrid molecules.

The individual components of such combinations may be administered either sequentially or simultaneously in separate or combined pharmaceutical formulations.

When the compound of formula (I) or a pharmaceutically acceptable derivative thereof is used in combination with a second therapeutic agent active against the same virus, the dose of each compound may be either the same or differ from that when the compound is used alone. Appropriate doses will be readily appreciated by those skilled in the art.

The invention will be further described by the following examples which are not intended to limit the invention in any way. All temperatures are in degrees celsius.

### EXAMPLES

### Example 1

### Benzoyloxyacetaldehyde

C₆H₅COOCH₂CHO (VII)

This known intermediate was prepared by portionwise addition of NaIO₄ (80 g) to a mixture of 1-benzoyl glycerol (50 g), CH₂Cl₂ (500 ml), and H₂O (25 ml) under vigorous stirring at room temperature. The resulting solution was stirred for 2 hours MgSO₄ (100 g) added, and the solution stirred for an additional 30 minutes. The mixture was filtered, the filtrate evaporated in vacuo and the residue distilled in vacuo to yield 26 g of pure product.
b.p. 92-94°/0.25 mm
¹H NMR (200 MH_{Z}; TMS as internal reference):
δ(ppm in CDCl₃)
9.71 (s, lH; -CHO)
8.11 (d, 2H; aromatic)
7.60 (m, 1H; aromatic)
7.46 (m, 2H; aromatic)
4.88 (s, 2H; -CH₂CHO)

### Example 2

### 2-Benzoylozymethyl-1,3-oxathiolane

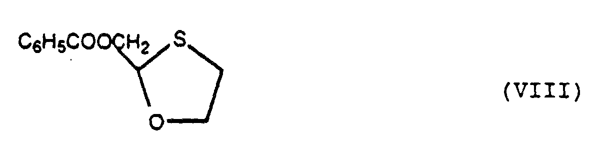

A mixture of benzoyloxyacetaldehyde (6.21 g), 2-mercaptoethanol (3 ml) and para-toluene sulfonic acid (0.2 g) in toluene (150 ml) was heated for 3 hours at refluxing under water removal conditions using a Dean Stark apparatus. The mixture was cooled to room temperature, washed first with aqueous NaHCO₃-solution (1 x 50 ml), and then with water (2.5 ml) and dried over MgSO₄. The solution was filtered and the filtrate evaporated under reduced pressure. The residue was purified on silica gel using hexane:ethyl acetate (9:1) as eluant. It yielded 7.63 g (90%) of pure product, which was identified by ¹H- and ¹³C-NMR.
R_{f}: 0.39 (hexane:ethyl acetate)
¹H-NMR: δ(ppm in CDCl₃)
8.03 (m, 2H, aromatic)
7.53 (m, 1H, aromatic)
7.39 (m, 2H, aromatic)
5.41 (dd, 1H, C₂-H)
4.43 (m, 2H, C₂-CH₂OCC₆H₅)
4.21 (m, 1H, C₅-H)
3.96 (m, 1H, C₅-H)
2.98 (m, 2H, C₄-H)
¹³C-NMR: δ(ppm in CDCl₃)
166.82, 133.74, 130.35, 128.97, 83.58, 71.87, 66.62 and 32.74

### Example 3

### 2-Benzoyloxymethyl-3-oxo-1,3-oxathiolane

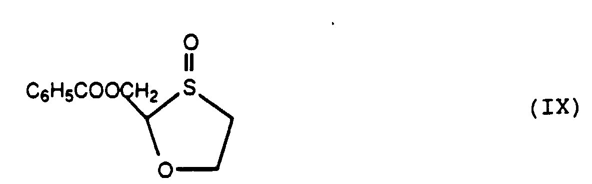

Monoperoxyphthalic acid, magnesium salt (MMPP, 28 g) was added portion wise under vigorous stirring to a mixture of 2-benzoyloxymethyl-1,3-oxathiolane (20 g), tetrabutyl ammonium bromide (0.4 g) in methylene chloride (200 ml), and water (200 ml). The mixture was stirred at room temperature for 30 minutes and the organic layer was 5 collected. The aqueous phase was extracted with methylene chloride (3 x 75 ml) and the combined organic layer was washed first with water (2 x 100 ml), then with brine solution (100 ml), dried over MgSO₄, and filtered. The filtrate was evaporated in vacuo and the residue was purified by chromatography on silica gel using ethyl acetate as eluant to give 18.5 g (86%) of pure product as a mixture of cis- and trans- isomers in a ratio of 1:2. m.p.: 70-72°
¹H-NMR: δ(ppm in CDCl₃)
8.05 (m, 2H, aromatic, cis-isomer)
7.95 (m, 2H, aromatic, trans-isomer)
7.56 (m, aromatic)
7.23 (m, aromatic)
4.77 (m, 4H, C₂-H, C₅-H, and C₂-CH₂OOCC₆H₅)
4.43 (m, 1H, C₅-H, trans-isomer)
4.09 (m, 1H, C₅-H, cis-isomer)
3.11 (m, 2H, C₄-H, trans-isomer)
2.75 (m, 2H, C₄-H, cis-isomer)
¹³C-NMR: δ(ppm in CDCl₃)
cis-isomer:
166.64, 134.02, 130.42, 129.88, 129.06, 96.16,
68.83, 59.47 and 54.30 trans-isomer:
166.36, 134.12, 130.29, 129.68, 129.15, 108.07, 70.09, 61.83 and 53.47

### Example 4

### 2-Benzoyloxymethyl-4-acetoxy-1,3-oxathiolane

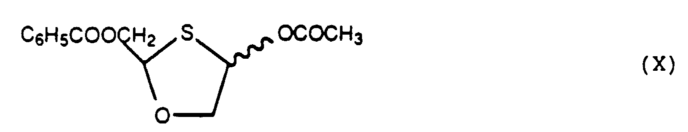

A mixture of 2-benzoyloxymethyl-3-oxo-1,3-oxathiolane (10.5 g), tetra-n-butylammonium acetate (17 g) in acetic anhydride (250 ml) was heated at 110°-120°C under argon for 14 hours and cooled to room temperature. Excess acetic anhydride was removed under reduced pressure. The residue was dissolved in methylene chloride (500 ml), washed first with saturated aqueous NaHCO₃ (2 x 200 ml), then with brine solution (200 ml), dried over MgSO₄, filtered and evaporated in vacuo. The residue was purified by chromatography on silica gel using hexane:ethyl acetate (8:1) as eluant to give 7.4 g (60% yield) of the desired product as a mixture of cis-and trans- isomers. A small quantity of each isomer was also isolated and characterized by ¹H- and ¹³C-NMR. cis-isomer:
R_{f}: 0.43 (hexane:EtOAc)
¹H-NMR: δ(ppm in CDCl₃)
8.05 (m, 2H, aromatic)
7.58 (m, 1H, aromatic)
7.45 (m, 2H, aromatic)
6.24 (d, 1H, C₄-H)
5.50 (t, 1H, C₂-H)
4.61 (d, 1H, C₂-CH₂OOCC₆H₅)
4.53 (d, 2H, C₅-H)
3.94 (dd, 1H, C₅-H)
2.05 (s, 3H, CH₃)
trans-isomer:
R_{f}: 0.43 (hexane:EtOAc 7:3)
¹H-NMR: δ(ppm in CDCl₃)
8.04 (m, 2H, aromatic)
7.58 (m, 1H, aromatic)
7.45 (m, 2H, aromatic)
6.27 (dd, 1H, C4-H)
5.73 (dd, 1H, C₂-H)
4.53 (dd, 1H, C₂-CH₂OOCC₆H₅)
4.34 (dd, 1H, C₅-H)
4.26 (dd, 1H, C₂-CH₂OCC₆H₅)
4.20 (dd, 1H, C₅-H)
2.09 (s, 3H, CH₃)
¹³C-NMR: δ(ppm in CDCl₃)
177.66, 166.37, 133.46, 129.93, 128.60, 83.76, 81.22, 74.33, 64.65 and 20.79

### Example 5

### Cis- and trans-2-benzoyloxymethyl-4-(cytosin-1'-yl)-1,3-oxathiolane

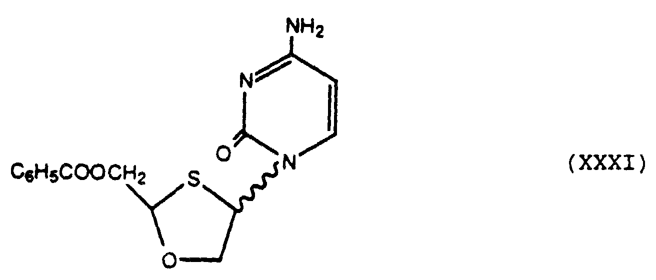

A mixture of cytosine (206 mg), ammonium sulfate (10 mg) and hexamethyldisilazane (HMDS, 10 ml) was heated at refluxing under argon until a clear solution resulted. Excess reagents were evaporated in vacuo and the remaining volatile removed under high vacuum (15 minutes). The solid residue was dissolved in dry methylene chloride (20 ml) and a solution of 2-benzoyloxymethyl-4-acetoxy-1,3-oxathiolane (350 mg) in dry methylene chloride (20 ml) was added under argon, followed by a solution of tin IV chloride (SnCl₄, 124 ml) in methylene chloride (20 ml) at 0°C. The reaction mixture was stirred under argon overnight at room temperature, then heated at refluxing for 3 hours and cooled to room temperature. The mixture was diluted with methylene chloride (100 ml) and poured while stirring into saturated aqueous NaHCO₃. The organic layer was separated by filtration over celite, washed first with water (2 x 75 ml), then with brine solution (100 ml), dried over MgSO₄ and filtered. The residue was purified by chromatography on silica gel using ethyl acetate:CH₃OH as the eluant to give 140 mg (35%) of the desired product as a mixture of cis- and trans- isomers in a 1:1 ratio as determined by 1H-NMR. These isomers were separated as the N-acetyl derivatives in the next example.

### Example 6

### Cis- and trans-2-benzoyloxymethyl-4-(N₄,-acetyl-cytosin-1'-yl)-1,3-oxathiolane

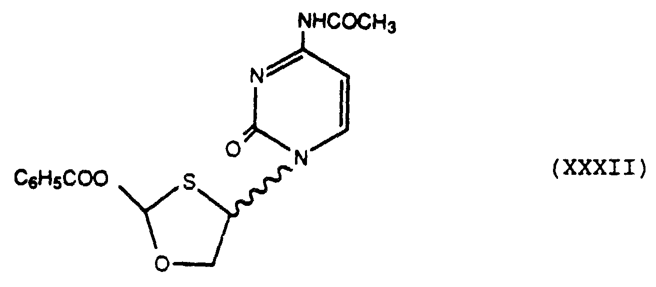

A solution of the cis- and trans- mixture of 2-benzoyloxymethyl-4-(cytosin-1'-yl)-1,3-oxathiolane (135 mg), 4-dimethylaminopyridine (DMAP, 15 mg) and acetic anhydride (44 ml) in dry pyridine (10 ml) was stirred overnight at room temperature (16 hours) and poured into cold water (100 ml) followed, by extraction with methylene chloride (3 x 50 ml). The extract was washed with water, dried over MgSO₄, filtered and evaporated in vacuo. Toluene was added to the residue, then evaporated in vacuo. Toluene was added to the residue, then evaporated in vacuo and the residual oil was purified by chromatography on silica gel using ethyl acetate as eluant to yield 65 mg of pure trans-isomer as the fast moving product and 60 mg of pure cis-isomer as the low moving product. These were characterized by ¹H and ¹³C-NMR.
cis-isomer:
¹H-NMR: δ(ppm in CDCl₃)
9.61 (b, 1H, C₄,-NHCOCH₃)
8.29 (d, 1H, C₆,-H)
8.06 (m, 2H, aromatic)
7.65 (m, 1H, aromatic)
7.51 (m, 2H, aromatic)
7.25 (d, 1H, C₅,-H)
6.61 (d, 1H, C4-H)
5.50 (t, 1H, C₂-H)
4.80 (m, 2H, C₂-CH₂OOCC₆H₅)
4.48 (d, 1H, C₅-H)
4.05 (dd, 1H, C₅-H)
2.25 (s, 3H, CH₃)
¹³C-NMR: δ(ppm in CDCl₃)
170.93, 166.28, 162.80, 155.76, 146.06, 133.91,
129.90, 128.84, 97.45, 85.88, 78.25, 64.60, 63.53 and 24.71.
trans-isomer:
¹H-NMR: δ(ppm in DMSOd₆)
10.88 (s, 1H, C4,-NHCOCH₃)
8.13 (d, 1H, C₆,-H)
7.96 (m, 2H, aromatic)
7.68 (m, 1H, aromatic)
7.52 (m, 2H, aromatic)
7.20 (d, 1H, C₅,-H)
6.35 (d, 1H, C₄-H)
5.96 (dd, 1H, C₂-H)
4.58 (dd, 1H, C₂-CH₂OOCC₆H₅)
4.44 (d, 1H, C₅-H)
4.29 (m, 2H, C₅-H and CH₂OOCC₆H₅)
2.07 (s, 3H, CH₃)
¹³C-NMR: δ(ppm in DMSOd₆)
171.53, 165.84, 162.76, 155.21, 146.59, 134.00,
129.64, 129.23, 96.54, 83.78, 74.24, 64.58, 64.01 and 24.35

### Example 7

### Cis- and trans-2-hydroxymethyl-4-(cytosin-1'-yl)-1,3-oxathiolane

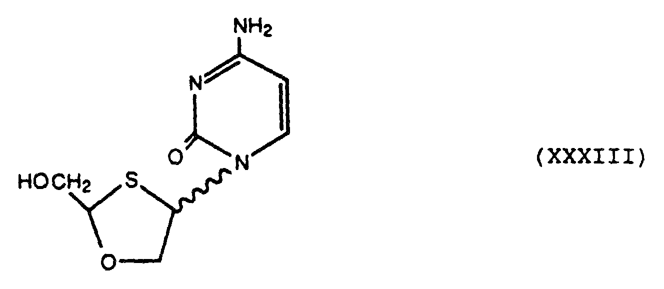

cis-isomer:

A solution of cis-2-benzoyloxymethyl-4(N₄,-acetyl-cytosin-l'-yl)-1,3-oxathiolane (54 mg) in methanolic ammonia (50 ml) was stirred overnight at room temperature (16 hours). The solvent was evaporated in vacuo and the residue treated with ether yielding 37 mg (90%) of desired product. The product was then characterized by ¹H- and ¹³C-NMR.
m.p.: 213-215°C
UV:(CH₃OH) Lamda max: 270 nm
¹H-NMR: δ(ppm in, DMSOd₆)
7.85 (d, 1H, C₆,-H)
7.16 (d, 2H, C₄,-NH₂)
6.34 (d, 1H, C₄-H)
5.76 (d, 1H, C₅,-H)
5.31 (t, 1H, C₂-CH₂OH)
5.18 (t, 1H, C₂-H)
4.40 (d, 1H, C₅-H)
3.92 (dd, 1H, C₅-H)
3.78 (m, 2H, C₂-CH₂OH)
¹³C-NMR: δ(ppm in DMSOd₆)
165.95, 155.74, 142.39, 94.98, 88.85, 77.29, 62.91 and 62.48
trans-isomer:

A solution of trans-2-benzoyloxymethyl-4-(N₄,-acetyl-cytosin-1'-yl)-1,3-oxathiolane (63 mg) in methanolic ammonia (50 ml) was stirred overnight at room temperature (16 hours). The solvent was removed in vacuo and the residue was solidified with ether to give 36 mg (93%) of the desired product which was characterized by ¹H-and ¹³C-NMR.
m.p.: 175-177°C
UV:(CH₃OH) Lamda max: 270 nm
¹H-NMR: δ(ppm in DMSOd₆)
7.67 (d, 1H, C₆,-H)
7.19 (d, 2H, C₄,-NH₂)
6.30 (d, 1H, C₄-H)
5.77 (d, 1H, C₅,-H)
5.56 (t, 1H, C₂-CH₂OH)
5.23 (t, 1H, C₂-H)
4.18 (m, 2H, C₅-H)
3.61 (m, 1H, C₂-CH₂OH)
3.36 (m, 1H, C₂-CH₂OH)
¹³C-NMR: δ(ppm in DMSOd₆)
166.00, 155.65, 142.30, 95.11, 87.52, 74.52, 63.42 and 62.86

### Example 8

### Cis- and trans-2-benzoyloxymethyl-4(uracil-1'-yl)-1,3-oxathiolane

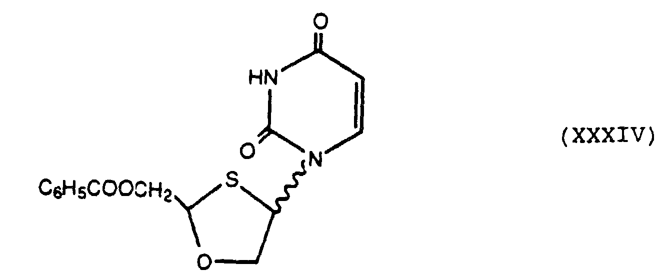

A mixture of uracil (446 mg), ammonium sulfate (20 mg) and hexamethyldisilazane (HMDS, 15 ml) was heated at refluxing under argon until the solution became clear (3 hours). Excess HMDS was removed under reduced pressure and the residue was dried under high vacuum for 3 hours. The oily residue was dissolved in dry methylene chloride (20 ml) and a solution of 2-benzoyloxymethyl-4-acetoxy-1,3-oxathiolane (748 mg) in dry methylene chloride (15 ml). The reaction mixture was stirred overnight at room temperature (20 hours) then poured into saturated aqueous NaHCO₃-solution (100 ml). The organic layer was collected and the aqueous phase was extracted with methylene chloride (2 x 50 ml). The combined organic phase was washed with water, dried over MgSO₄, filtered and evaporated under reduced pressure. The residue was purified and separated by chromatography on silica gel using hexane:ethyl acetate (1:4) as eluant. It gave 258 mg (29%) of a fast moving product, which was identified as trans-isomer and 156 mg (18%) of a low moving product, which was identified as cis-isomer. cis-isomer:
¹H-NMR: δ(ppm in DMSOd₆)
11.39 (s, 1H, N₃,-H)
8.00 (d, 2H, aromatic)
7.97 (m, 2H, C₆,-H and aromatic)
7.56 (m, 2H, aromatic)
6.31 (d, 1H, C₄-H)
5.53 (t, 1H, C₂-H)
5.41 (d, 1H, C₅,-H)
4.76 (d, 2H, C₂-CH₂OH)
4.65 (d, 1H, C₅-H)
4.01 (dd, 1H, C₅-H)
¹³C-NMR: δ(ppm in DMSOd₆).
165.83, 163.36, 151.06, 141.25, 134.15, 129.72,
129.50, 129.29, 102.83, 85.34, 76.60, 63.78 and 62.68
trans-isomer:
¹H-NMR: δ(ppm in CDCl₃)
9.10 (b, 1H, N₃,-H)
8.02 (d, 2H, aromatic)
7.53 (m, 2H, C₆,-H and aromatic)
7.43 (m, 2H, aromatic)
6.50 (d, 1H, C₄-H)
5.87 (dd, 1H, C2₅,-H)
5.76 (d, 1H, C₅,-H)
4.56 (dd, 1H, C₂-CH₂OBz)
4.30 (m, 2H, C₅,-H)
4.24 (dd, 1H, C₂-H₂OBz)
¹³C-NMR: δ(ppm in CDCl₃)
166.28, 163.49, 150.89, 140.70, 133.59, 129.87, 129.42, 128.64, 103.72, 84.18, 75.18, 64.24 and 62.23

### Example 9

### Cis-and trans-2-hydroxymethyl-4-(uracil-1'-yl)-1,3-oxathiolane

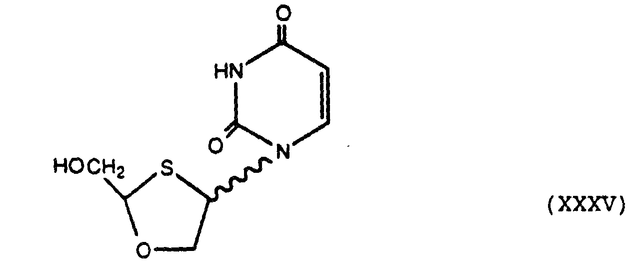

cis-isomer:

A solution of cis-2-benzoyloxymethyl-4-(uracil-1'-yl)-1,3-oxathiolane (150 mg) in methanolic ammonia (50 ml) was stirred overnight at room temperature (16 hours). Solvent was evaporated in vacuo and the residue was triturated with ether (2 x 10 ml) and crystallized in ethyl acetate to yield 77 mg (75%) of desired product which was characterized by spectroscopic methods.
m.p.: 138-140°C
UV: (CH₃OH) Lamda max: 266 nm
¹H-NMR: δ(ppm in DMSOd₆)
11.36 (s, 1H, N₃,-H)
7.88 (d, 1H, C₆,-H)
6.28 (d, 1H, C₄-H)
5.66 (d, 1H, C₅,-H)
5.39 (t, 1H, C₂-CH₂OH)
5.19 (t, 1H, C₂-H)
4.55 (d, 1H, C₅-H)
3.95 (dd, 1H, C₅-H)
3.80 (m, 2H, C₂-CH₂OH)
¹³C-NMR: δ(ppm in DMSOd₆).
163.54, 151.13, 141.87, 102.63, 89.13, 77.15, 62.45 and 62.04
trans-isomer:

A solution of trans-2-benzoyloxymethyl-4-(uracil-1'-yl)-1,3-oxathiolane (207 mg) in methanolic ammonia (50 ml) was stirred overnight at room temperature (16 hours) and then evaporated in vacuo. The residue was treated with ether (2 x 20 ml) and filtered. The solid residue was washed with cold ethyl acetate and yield 115 mg (81%) of pure product which was characterized by ¹H- and ¹³C-NMR.
m.p.: 176-178°C
UV: (CH₃OH) Lamda max: 266 nm
¹H-NMR: δ(ppm in DMSOd₆)
11.39 (s, 1H, N₃,-H)
7.66 (d, 1H, C₆,-H)
6.25 (d, 1H, C₄-H)
5.67 (d, 1H, C₅,-H)
5.62 (dd, 1H, C₂-H)
5.27 (t, 1H, C₂-CH₂OH)
4.35 (d, 1H, C₅-H)
4.16 (dd, 1H, C₅-H)
3.60 (m, 1H, C₂-CH₂OH)
3.35 (m, 1H, C₂-CH₂OH)
¹³C-NMR: δ(ppm in DMSOd₆)
163.53, 151.15, 141.77, 102.78, 87.77, 74.28, 63.25 and 62.32

### Example 10

### Cis- and trans-2-benzoyloxymethyl-4-(thymin-1'-yl)-1,3-oxathiolane

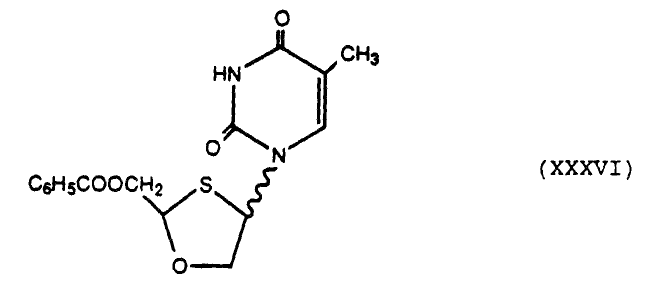

A mixture of thymine (671 mg), ammonium sulfate (20 mg) and hexamethyldisilazane (HMDS, 20 ml) was heated at refluxing until the solution became clear (3 hours). Excess reagent was evaporated in vacuo and the remaining volatile removed under high vacuum (1 hour). The oily residue was dissolved in dry methylene chloride (20 ml) and a solution of 2-benzoyloxymethyl-4-acetoxy-1,3-oxathiolane (1.05 g) in dry methylene -chloride (20 ml) was added under argon, followed by a solution of trimethylsilyl trifluoromethane sulfonate (865 ml) in methylene chloride (5 ml). The reaction mixture was stirred overnight at room temperature under argon (16 hours) and poured into saturated aqueous NaHCO₃-solution. The organic layer was separated and the aqueous phase extracted with methylene chloride (3 x 50 ml). The combined organic layer was washed with water (2 x 50 ml). The combined organic layer was washed first with water (2 x 50 ml), then with brine solution (100 ml), dried over MgSO₄, filtered and evaporated in vacuo. The residue was purified and separated by chromatography on silica gel using hexane:ethyl acetate (1:1) as eluant to give 732 mg of a fast moving product which was identified by spectroscopic methods as trans-isomer and 244 mg of a lower moving product which was identified as cis-isomer. Total yield was 976 mg (75%). cis-isomer:
¹H-NMR: δ(ppm in DMSOd₆)
11.41 (s, 1H, N₃,-H)
7.98 (d, 2H, aromatic)
7.72 (t, 1H, aromatic)
7.53 (t, 2H, aromatic)
7.47 (s, 1H, C₆,-H)
6.32 (d, 1H, C₂-H)
4.73 (m, 3H, C₅-H and C₂-CH₂OOCC₆H₅)
4.01 (dd, 1H, C₅-H)
1.58 (s, 3H, CH₃)
¹³C-NNR: δ(ppm in DMSOd₆)
165.89, 164.00, 151.08, 136.37, 134.09, 129.63,
110.73, 85.30, 75.99, 63.68, 62.58 and 12.18
trans-isomer:
¹H-NMR: δ(ppm in CDCl₃)
8.30 (s, 1H, N₃,-H)
8.04 (d, 2H, aromatic)
7.58 (t, 1H, aromatic)
7.45 (t, 2H, aromatic)
7.32 (d, 1H, C₆,-H, J = 1.3 Hz)
6.50 (dd, 1H, C₄-H)
5.90 (dd, 1H, C₂-H)
4.58 (dd, 1H C₂-CH₂OCC₆H₅)
4.30 (m, 2H, C₅-H)
4.24 (dd, 1H, C₂-CH₂OOCC₆H₅)
1.91 (d, 3H, -CH₃, J = 1.1 Hz)
¹³C-NMR: 6(ppm in CDCl₃)
166.29, 163.91, 150.95, 136.14, 133.59, 129.88, 128.58, 112.48, 84.16, 75.04, 64.29, 62.35 and 12.41

### Example 11

### Cis- and trans-2-hydroxymethyl-4-(thymin-1'-yl)-1,3-oxathiolane

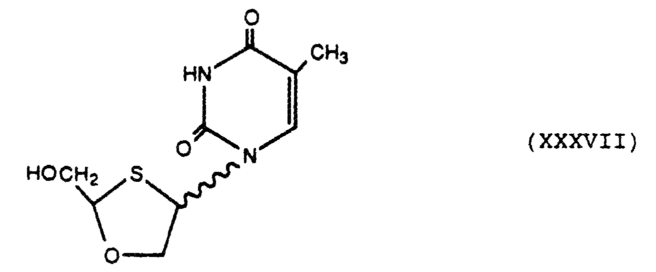

cis-isomer:

A solution of cis-2-benzoyloxymethyl-4-(thymin-1'-yl)-1,3-oxathiolane (190 mg) in saturated methanolic ammonia was stirred overnight at room temperature (16 hours). The mixture was evaporated under reduced pressure and the residue was purified by chromatography on silica gel using ethyl acetate as eluant to give 109 mg (82%) of pure product.
m.p.: 149-151°C
UV: (CH₃OH) Lamda max: 271.3 nm
¹H-NMR: δ(ppm in DMSOd₆)
11.37 (s, 1H, N₃,-H, D₂O-exchange)
7.79 (d, 1H, C₆,-H, J = 1.1 Hz)
6.30 (d, 1H, C₄-H, J = 4.4 Hz)
5.44 (t, 1H, C₂-CH₂OH, D₂O-exchange)
5.20 (t, 1H, C₂-H, J = 4.2 Hz)
4.52 (d, 1H, C₅-H, J = 10.7 Hz)
3.93 (dd, 1H, C₅-H, J = 4.90 and 10.7 Hz)
3.81 (m, 2H, C₂-CH₂OH)
1.78 (s, 3H, -CH₃)
¹³C-NMR: δ(ppm in DMSOd₆)
164.15, 151.08, 137.44, 110.14, 89.09, 67.17, 62.18, 61.89 and 12.37
trans-isomer:

A mixture of trans-2-benzoyloxymethyl-4-(thymin-1'-yl)-1,3-oxathiolane (200 mg) in saturated methanolic ammonia (50 ml) was stirred overnight at room temperature (16 hours). The mixture was evaporated under reduced pressure and the residue was triturated with diethyl ether (3 x 15 ml) and filtered. The solid residue was recrystallized in ethanol to give 136 mg (97%) of pure product.
m.p.: 202-204 °C
UV: (CH₃OH) Lamda max: 271.3 nm
¹H-NMR: δ(ppm in DMSOd₆)
11.40 (s, 1H, N₃,-H, D₂O exchange)
7.49 (s, 1H, C₆,-H)
6.26 (d, 1H, C₄-H)
5.67 (dd, 1H, C₂-H)
5.27 (t, 1H, C₂-CH₂OH, D₂O-exchange)
4.32 (d, 1H, C₅-H)
4.16 (dd, 1H, C₅-H)
3.58 (dd, 1H, C₂-CH₂OH)
3.33 (dd, 1H, C₂-CH₂OH)
1.80 (s, 3H, CH₃)
¹³C-NMR: δ(ppm in DMSOd₆)
164.04, 150.90, 136.92, 110.44, 87.68, 74.06, 63.18, 61.98 and 12.19

### Example 12

### Cis- and trans-2-benzoyloxymethyl-4-(6'-chloropurin-9'-yl)-1,3-oxathiolane

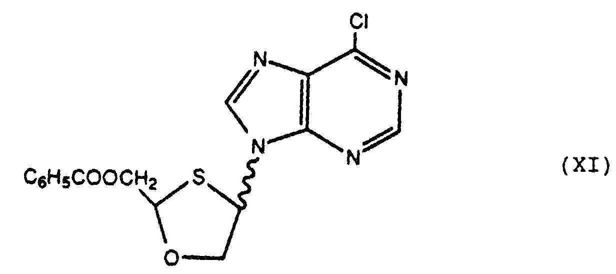

A mixture of 6-chloropurine (850 mg), ammonium sulfate (20 mg) and hexamethyldisilazane (HMDS, 20 ml) was heated at refluxing until the solution became clear. Excess reagent was evaporated in vacuo and the remaining volatile removed under high vacuum (1 hour). The oily residue was dissolved in dry dichloroethane (50 ml) and a solution of 2-benzoyloxymethyl-4-acetoxy-1,3-oxathiolane (1 g) in dry dichloroethane (20 ml) was added under argon, followed by a solution of trimethylsilyl trifluoromethane sulfonate (825 ml) in dry dichloroethane (5 ml). The mixture was heated at refluxing under argon for 2 hours, cooled to room temperature and poured into saturated aqueous NaHCO₃ solution. The organic layer was collected and the aqueous phase was extracted with methylene chloride (3 x 70 ml). The combined organic layer was washed first with water (2 x 50 ml), then with brine solution, dried over MgSO₄ and filtered. The filtrate was evaporated under reduced pressure and the residue was purified by chromatography on silica gel using hexane:ethyl acetate (1:1) as eluant to give 435 mg of fast moving product which was identified as trans-isomer and 245 mg of lower moving product which was identified as cis-isomer. The total yield was 51%. Each isomer was characterized by ¹H- and ¹³C-NMR spectroscopy.
cis-isomer:
¹H-NMR: δ(ppm in CDCl₃)
8.73 (s, 1H, C₈,-H)
8.53 (s, 1H, C₂,-H)
8.02 (d, 2H, aromatic)
7.56 (m, 1H, aromatic)
7.45 (m, 2H, aromatic)
6.51 (d, 1H, C₄-H, J = 3.8 Hz)
5.62 (t, 1H, C₂-H, J = 4.8 Hz)
4.78 (m, 2H, C₂-CH₂OOCC₆H₅)
4.69 (d, 1H, C₅-H, J = 10.4 Hz)
4.18 (dd, 1H, C₅-H, J= 4.0 and 10.6 Hz)
¹³C-NMR: δ(ppm in CDCl₃)
165.83, 152.24, 151.80, 149.63, 145.48, 134.07, 129.58, 129.21, 85.42, 76.84, 64.48, and 61.75
trans-isomer:
¹H-NMR: δ(ppm in CDCl₃)
8.74 (s, 1H, C₈,-H)
8.38 (s, 1H, C₂,-H)
8.05 (d, 2H, aromatic)
7.58 (m, 1H, aromatic)
7.45 (m, 2H, aromatic)
6.50 (d, 1H, C₄-H, J = 3.7 Hz)
6.03 (dd, 1H, C₂-H)
4.70 (dd, 1H, C₂-CH₂OOCC₆H₅)
4.50 (m, 2H, C₅-H)
4.32 (dd, lH, C₂-CH₂OOCC₆H₅)

### Example 13

### Cis-2-hydroxymethyl-4-(6'-chloropurin-9'-yl)-1,3-oxathiolane

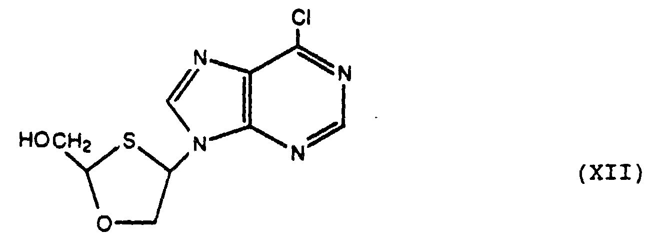

A solution of cis-2-benzoyloxymethyl-4-(6'-chloropurin-9'-yl)-1,3-oxathiolane (300 mg) in methanolic ammonia (40 ml) was stirred overnight at room temperature (16 hours). The solvent was evaporated under reduced pressure and the residue was purified by chromatography on silica gel using EtOAc:MeOH (95:5) as eluant to give 189 mg (88%) of the desired product.
m.p.: 175-176°C
R_{f}: 0.68(EtOAc:MeOH)
UV: (H₂O) Lamda Max 263.5 nm
H-NMR: δ(ppm in DMSOd₆)
9.07 (s, 1H, C₈,-H)
8.98 (s, 1H, C₂,-H)
6.70 (d, 1H, C₄-H)
5.61 (t, 1H, C₂-CH₂OH, D₂O-exchange)
5.53 (t, 1H, C₂-H)
4.90 (d, 1H, C₅-H)
4.27 (dd, 1H, C₅-H)
4.02 (m, 2H, C₂-CH₂OH)
¹³C-NMR: δ(ppm in DMSOd₆)
152.17, 151.59, 149.58, 146.07, 131.17, 80.04, 77.37, 62.02, and 61.31

### Example 14

### Cis- and trans-2-hydroxymethyl-4-(adenin-9'-yl)-1,3-oxathiolane

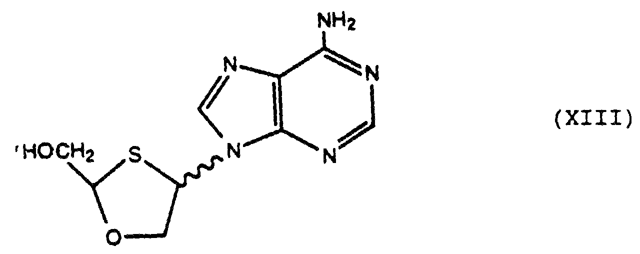

cis-isomer:

A mixture of cis-2-benzoyloxymethyl-4-(6-chloropurin-9'-yl)-1,3-oxathiolane (140 mg) in saturated ethanolic ammonia (50 ml) was placed in a steel bomb and heated overnight at 100-110°C (16 hours). The bomb was cooled to room temperature and emptied. The mixture was evaporated under reduced pressure and the residue was purified by chromatography on silica get using ethyl acetate:methanol (85:5) as eluant to give 70 mg (71%) of pure product.
m.p.: 200-202°C
UV: (CH₂OH) Lamda max: 260 nm
¹³H-NMR: δ(ppm in DMSOd₆)
8.31 (s, 1H, C₈,-H)
8.16 (s, 1H, C₂,-H)
7.31 (s, 2H, C₆-NH₂, D₂O-exchange)
6.36 (d, 1H, C₄-H, J = 3.8 Hz)
5.41 (t, 1H, C₂-H, J = 3.8 Hz)
5.34 (t, 1H, C₂-CH₂OH, D₂O-exchange)
4.66 (d, 1H, C₅-H, J = 11.4 Hz)
4.09 (dd, 1H, C₅-H, J = 4.1 and 10.4 and 10.4 Hz)
3.83 (m, 2H, C₂-CH₂OH)
¹³C-NMR: δ(ppm in DMSOd₆)
156.51, 153.09, 149.45, 139.19, 118.76, 89.06, 77.65, 62.79 and 60.13
trans-isomer:

A mixture of trans-2-benzoyloxymethyl-4-(6'-chloropurin-9'-yl)-1,3-oxathiolane (220 mg) in saturated ethanolic ammonia (50 ml) was placed in a steel bomb and heated overnight at 110°C for overnight (16 hours). The bomb was cooled to room temperature and emptied. The mixture was evaporated under reduced pressure and the residue was purified by chromatography on silica gel using EtOAc:MeOH (95:5) as eluant to give 76 mg (50%) the desired product.
m.p.: 185-187°C
UV: (CH₃OH) Lamda max: 260 nm
¹³H-NMR: δ(ppm in DMSOd₆)
8.23 (s, 1H, C₈,-H)
8.17 (s, 1H, C₂,-H)
7.32 (s, 2H, C₆-NH₂, D₂O-eXchange)
6.36 (d, 1H, C₄-H, J = 3.8 Hz)
5.75 (t, 1H, C₂-H, J = 5.7 Hz)
5.32 (t, 1H, C₂-CH₂OH, D₂O-exchange)
4.50 (d, 1H, C₅-H, J = 10.2 Hz)
4.35 (dd, 1H, C₅-H, J = 4.1 Hz and 10.0 Hz) 3.73 (m, 1H, C₂-CH₂OH)
3.43 (m, 1H, C₂-CH₂OH)
¹³C-NMR: δ(ppm in DMSOd₆)
165.53, 153.12, 149.40, 139.30, 119.00, 87.99, 74.70, 63.29 and 60.51

### Example 15

### Cis-2-hydroxymethyl-4-(adenin-9'-yl)-3-oxo-1,3-oxathiolane

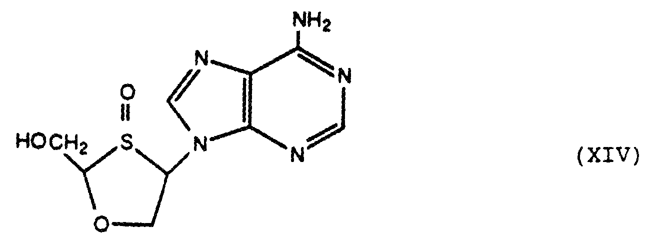

A solution of 2-hydroxymethyl-4-(adenin-9'-yl)-1,3-oxathiolane (60 mg) in methanol (30 ml) was cooled at 0°C in an ice bath and meta-chloroperbenzoic acid (49 mg) was added slowly under stirring. The mixture was kept at this temperature for and solvent was evaporated under reduced pressure. The residue was triturated with diethyl either (2 x 15 ml) and purified on silica gel using EtOAc:MeOH (9:1) as eluant to give 49 mg of the product (77% yield). Surprisingly, only one diastereomer was formed. The product was characterized by H-and ¹³C-NMR.
UV: (H₂O) Lamda max: 258 nm
m.p.: Dec >192°C
¹H-NMR: δ(ppm in DMSOd₆)
8.24 (s, 1H, C₈,-H)
8.08 (s, 1H, C₂,-H)
7.41 (s, 2H, C₄,-NH₂, D₂O-exchange)
5.85 (d, 1H, C₄-H, J = 3.8 Hz)
5.52 (t, 1H, C₂-CH₂OH, D₂O-exchange)
5.17 (d, 1H, C₅-H, J = 11.6 Hz)
4.62 (dd, 1H, C₅-H, J = 4.1 and 11.8 Hz)
4.60 (t, 1H, C₂-H, J = 3.6 Hz)
4.02 (m, 1H, C₂-CH₂OH)
3.82 (m, 1H, C₂-CH₂OH)
¹³C-NMR: δ(ppm in DMSOd₆)
156.66, 153.51, 149.83, 138.56, 118.61, 111.84, 75.99, 71.91 and 58.29

### Example 16

### Cis-2-hydroxymethyl-4-(6'-N-methylamino-purin-9'-yl)-1,3-oxathiolane

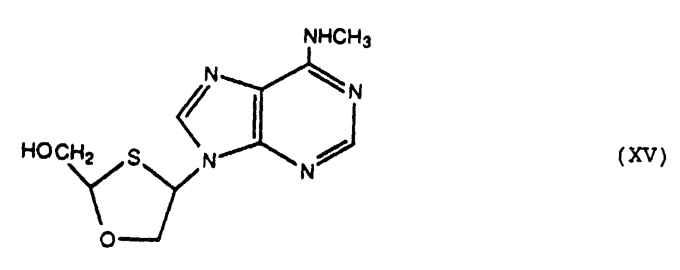

A solution of cis-2-hydroxymethyl-4-(6'-chloropurin-9'-yl)-1,3-oxathiolane (144 mg) in ethanol (30 ml) was cooled at 0°C in an ice bath and methylamine gas was bubbled through the solution for 20 minutes. The mixture was placed in steel bomb and heated overnight at 110-115°C (16 hours). The bomb was cooled to room temperature and emptied. The mixture was evaporated in vacuo and the residue was purified by chromatography on silica gel using EtOAc:MeOH (9:1) as eluant to give 102 mg (72%) of the desired product.
m.p.: 190-192°C
R_{f}: 0.43 (EtOAc:MeoH)
UV: (H₂O) Lamda max: 266 nm
¹H-NMR: δ(ppm in DMSOd₆)
8.31 (s, 1H, C₈,-H)
8.25 (s, 1H, C₂,-H)
7.79 (b, 1H, C₄,-NH)
6.38 (d, 1H, C₄-H, J = 3.8 Hz)
5.40 (t, 1H, C₂-CH₂OH)
5.33 (t, 1H, C₂-H, J = 4.3 Hz)
4.64 (d, 1H, C₅-H, J = 10.5 Hz)
4.10 (dd, 1H, C₅-H, J = 4.3 and 10.5 Hz)
3.82 (m, 2H, C₂-CH₂-OH)
2.95 (s, 3H, C₄,-NCH₃)

### Example 17

### Cis- and trans-2-hydroxymethyl-4-(6'-dimethylamino purin-9'-yl)-1,3-oxathiolane

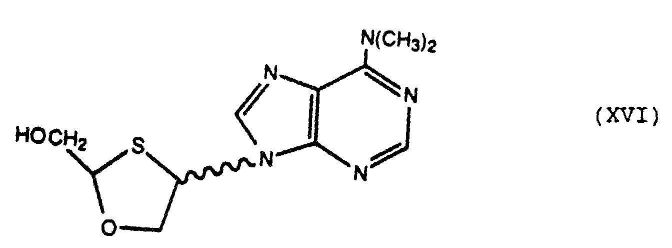

cis-isomer:

A solution of cis-2-benzoyloxymethyl-4-(6'-chloropurin-9'-yl)-1,3-oxathiolane (300 mg) in ethanol (60 ml) was cooled at 0°C in an ice bath and dimethylamine gas was bubbled through the solution for 20 minutes. The mixture was placed in a steel bomb and heated overnight at 110-115°C (16 hours). The bomb was cooled to room temperature and emptied. Solvent was removed in vacuo and the residue was purified by chromatography on silica gel using ethyl acetate as eluant to give 170 mg (76%) of the desired product. m.p.: 124-126°C
R_{f}: 0.32 (EtOAc)
UV: (CH₃OH) Lamda max: 273 nm
¹H-NMR: δ(ppm in DMSOd₆)
8.32 (s, 1H, C₈,-H)
8.22 (s, 1H, C₂,-H)
6.37 (d, 1H, C₄,-H, J = 4.0 Hz)
5.38 (t, 1H, C₂-CH₂OH, D₂O-exchange)
5.32 (t, 1H, C₂-H, J = 4.3 Hz)
4.63 (d, 1H, C₅-H, J = 11.5 Hz)
4.08 (dd, 1H, C₅-H, J = 4.0 and 10.5 Hz)
3.80 (m, 2H , C₂-CH₂OH)
3.43 (b, 6H, C₆,-N(CH₃)₂)
trans-isomer:

A solution of trans-2-benzoyloxymethyl-4-(6'-chloropurin-9'-yl)-1,3-oxathiolane (500 mg) in ethanol (70 ml) was cooled at 0°C in an ice bath and dimethylamine gas was bubbled through the solution for 20 minutes. The mixture was placed in a steel bomb and heated overnight at 110°-115°C (16 hours). The bomb was cooled to room temperature and emptied. Solvent was removed in vacuo and the solid residue triturated with diethylether (2 x 30 ml) and recrystallized in ethanol to give 268 mg of the desired product (72% yield).
m.p.: 174-176°C
R_{f}: 0.30(EtOAc)
UV: (CH₃OH) Lamda max: 271 nm
¹H-NMR: δ(ppm in DMSOd₆)
8.23 (s, 1H, C₈,-H)
8.22 (S, 1H, C₂,-H)
6.36 (d, 1H, C₄-H, J = 3.0 Hz)
5.71 (dd, 1H, C₂H, J = 4.9 and 6.8.Hz)
5.30 (t, 1H, C₂-CH₂OH, D₂O-exchange)
4.46 (dd, 1H, C₅-H, J = 3.3 and 12.4 Hz)
4.32 (dd, 1H, C₅-H, J = 4.3 and 10.4 Hz)
3.68 (m, 1H, C₂-CH₂OH)
3.43 (m, 7H, C₂-CH₂OH and C₆,-N(CH₃)₂)

### Example 18

### Cis- and trans-2-benzoyloxymethyl-4-(2'-amino-6'-chloro-purin-9'-yl)-1,3-oxathiolane

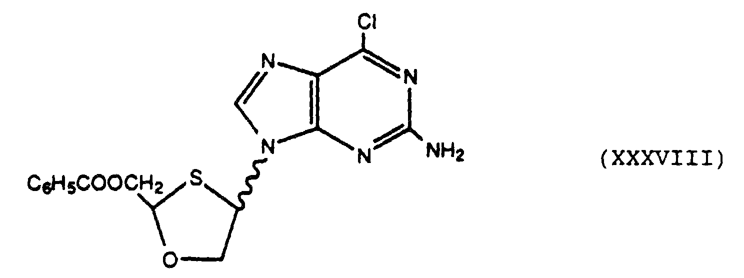

A mixture of 2-amino-6-chloropurine (6-chloroguanine, 1.3 g), ammonium sulfate (50 mg), and hexamethyldisilazane (HMDS, 30 ml) became clear (3 hours). Excess reagent was evaporated in vacuo and the remaining volatile removed under high vacuum (1 hour). The residue was dissolved in dry dichloroethane (75 ml) and a solution of 2-benzoyloxymethyl-4-acetoxy-1,3-oxathiolane (1.1 g), in dry dichloroethane (20 ml) was added under argon, followed by a solution of trimethylsilyl trifluoromethane sulfonate (1.5 ml) in dry dichloroethane (15 ml). The mixture was heated at refluxing under argon for 2 hours, cooled to room temperature and poured into saturated aqueous NaHCO₃ solution. After stirring for 15 minutes the organic layer was collected and the aqueous phase was extracted with methylene chloride (3 x 75 ml). The combined organic layer was washed with water (2 x 100 ml), then with brine solution, dried over MgSO₄, and filtered. The filtrate was evaporated in vacuo and the residue was purified by chromatography on silica gel using hexane:ethyl acetate (1:1) as eluant to give 460 mg of a fast moving product which was identified as trans-isomer and 340 mg of low moving product which was identified as cis-isomer. The total yield was 52%.
cis-isomer:
m.p.: 192-194°C
R_{f}: 0.50 (hexane:ethyl acetate 3:7)
¹H-NMR: δ(ppm in DMSOd₆)
8.20 (s, 1H, C₈,-H)
7.89 (m, 2H, aromatic)
7.69 (m, 1H, aromatic)
7.52 (m, 2H, aromatic)
7.03 (s, 2H, C₂,-NH₂)
6.26 (d, 1H, C₄-H, J = 3.9 Hz)
5.66 (t, 1H, C₂-H, J = 6.00 Hz)
4.88 (d, 1H, C₅-H, J = 10.8 Hz)
4.73 (m, 2H, C₂-CH₂OOCC₆H₅)
4.16 (dd, 1H, C₅-H, J = 4.2 and 10.7 Hz)
trans-isomer:
m.p.: 186-188°C
R_{f}: 963 (hexane:ethyl acetate)
¹H-NMR: δ(ppm in DMSOd₆)
8.21 (s, 1N, C₈,-H)
8.01 (m, 2H, aromatic)
7.60 (m, 1H, aromatic)
7.57 (m, 2H, aromatic)
7.04 (s, 2H, C₂,-NH₂)
6.29 (d, 1H, C4-H, J = 4.0 Hz)
6.11 (dd, 1H, C₂-H, J = 3.2 and 8.2 Hz)
4.63 (m, 2H, C₅-H and C₂-CH₂OOCC₆H₅)
4.38 (m, 2H, C₅-H and C₂-CH₂OOCC₆H₅)

### Example 19

### Cis- and trans-2-hydroxymethyl-4-(2'-amino-6'-chloropurin-9'-yl)-1,3-oxathiolane

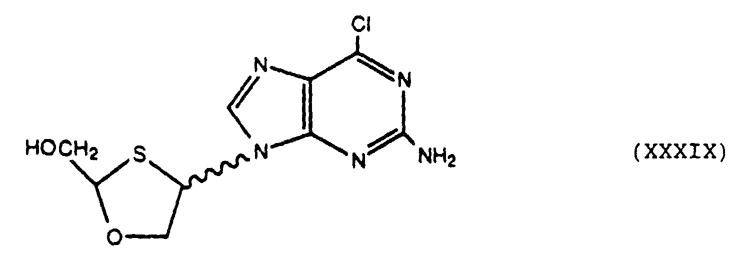

cis-isomer:

A solution of cis-2-benzoyloxymethyl-4-(2'-amino-6'-chloropurin-9'-yl)-1,3-oxathiolane (305 mg) in methanolic ammonia (100 ml) was stirred overnight at room temperature (16 hours). The mixture was evaporated under reduced pressure and the residue was triturated with diethyl ether (2 x 30 ml). The solid residue was recrystallized in ethanol to give pure 184 mg of product (82% yield).
m.p.: 194-196°C
R_{f}: 0.58 (EtOAc)
UV: (CH₃OH) Lamda max: 309 and 248 nm
¹H-NMR: δ(ppm in DMSOd₆)
8.30 (s, 1H, C₈,-H)
7.03 (s, 2H, C₂,-NH₂)
6.19 (d, 1H, C₄-H, J = 3.9 Hz)
5.41 (t, 1H, C₂-CH₂OH, D₂O-exchange)
5.33 (t, 1H, C₂-H, J = 4.1 Hz)
4.70 (d, 1H, C₅-H, J = 10.4 Hz)
4.06 (dd, 1H, C₅-H, J = 4.1 and 10.5 Hz)
3.82 (t, 2H, C₂-CH₂OH)
trans-isomer:

A solution of trans-2-benzoyloxymethyl-4-(2'-amino-6'-chloropurin-9'-yl)-1,3-oxathiolane (150 mg) in methanolic ammonia (50 ml) was stirred overnight at room temperature (16 hours). Solvent was evaporated under reduced pressure and the residue purified by chromatography on silica gel using ethyl acetate as eluant to give 83 mg (76%) of pure product.
m.p.: 180-182°C
R_{f}: 0.50 (ethyl acetate)
U.V. (CH₃OH) Lamda max: 309:5 and 248 nm
¹H-NMR: δ(ppm in DMSOd₆)
8.21 (s, 1H, C₈,-H)
7.03 (s, 2H, C₂,-NH₂)
6.20 (dd, 1H, C₄-H, J = 1.6 and 4.3 Hz)
5.74 (dd, 1H, C₂-H, J = 4.7 and 8.9 Hz)
5.31 (t, 1H C₂-CH₂OH, D₂O-exchange)
4.52 (dd, 1H, C₅-H, J = 1.7 and 10.4 Hz)
4.31 (dd, 1H, C₅-H, J = 4.4 and 10.4 Hz)
3.68 (g, 1H, C₂-CH₂OH)
3.44 (g, 1H, C₂-CH₂OH)

### Example 20

### Cis- and trans-2-hydroxymethyl-4-(2',6'-diaminopurin-9'-yl)-1,3-oxathiolane

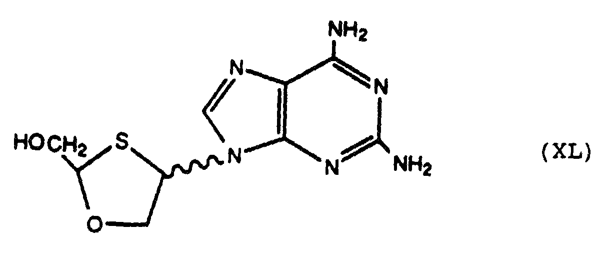

cis-isomer:

A mixture of cis-2-benzoyloxymethyl-4-(2'-amino-6'-chloropurin-9'-yl)-1,3-oxathiolane (175 mg) in saturated methanolic ammonia (50 ml) was placed in a steel bomb and heated overnight at 110-115°C (16 hours). The bomb was cooled to room temperature and emptied. The mixture was evaporated to dryness and the solid residue triturated with diethyl ether (2 x 30 ml) and dissolved in methanol (100 ml). The solution was boiled with charcoal (2 g) for 5 minutes and filtered. The filtrate was evaporated in vacuo and the residue recrystallized in ethanol to give 90 mg of the product (75% yield).
m.p.: Dec.>250°C
R_{f}: 0.27 (EtOAc:MeOH 9:1)
UV: (CH₃OH) Lamda max: 281 and 258 nm
¹H-NMR: δ(ppm in DMSOd₆)
7.80 (s, 1H, C₈,-H)
6.94 (b, 2H, C₂,-NH₂, D₂O-exchange)
6.12 (d, 1H, C₄-H, J = 3.9 Hz)
6.00 (b, 2H, C₆,-NH₂, D₂O-exchange)
5.40 (b, 1H, C₂-CH₂OH, D₂O-exchange)
5.30 (t, 1H, C₂-H, J = 4.5 Hz)
4.61 (d, 1H, C₅-H, J = 10.4 Hz)
4.05 (dd, 1H, C₅-H, J = 4.2 and 10.4 Hz)
3.78 (m, 2H, C₂-CH₂OH)
trans-isomer:

A mixture of trans-2-benzoyloxymethyl-4-(2'-amino-6'-chloropurin-9'-yl)-1,3-oxathiolane (300 mg) in saturated methanolic ammonia (60 ml) was placed in a steel bomb and heated overnight at 110-115°C. The bomb was cooled to room temperature and emptied. The mixture was evaporated to dryness and the solid residue was triturated with diethyl ether (2 x 30 ml) and dissolved in methanol (100 ml). The solution was boiled with charcoal for 5 minutes and filtered. The filtrate was evaporated in vacuo and the residue was recrystallized in ethanol to give 149 mg (72%) of the desired product. m.p.: 242-244°C
R_{f}: 0.23 (EtOAc:MeOH 9:1)
UV: (CH₃OH) Lamda max: 281 and 259 nm
¹H-NMR: δ(ppm in DMSOd₆)
7.81 (s, 1H, C₈,-H)
6.78 (s, 2H, C₂,-NH₂, D₂O-exchange)
6.12 (d, 1H, C₄-H, J = 4.4 Hz)
5.90 (s, 2H, C₆,-NH₂,, D₂O-exchange)
5.70 (dd, 1H, C₂-H, J = 4.5 and 6.7 Hz)
5.31 (t, 1H, C₂-CH₂OH, D₂O-exchange)
4.44 (d, 1H, C₅,-H, J = 10.2 Hz)
4.29 (dd, 1H, C₅-H, J = 4.6 and 10.2 Hz)
3.67 (m, 1H, C₂-CH₂OH)
3.43 (m, 1H, C₂-CH₂OH)

### Example 21

### Cis-2-hydroxymethyl-4-(guanin-9'-yl)-1,3-oxathiolane

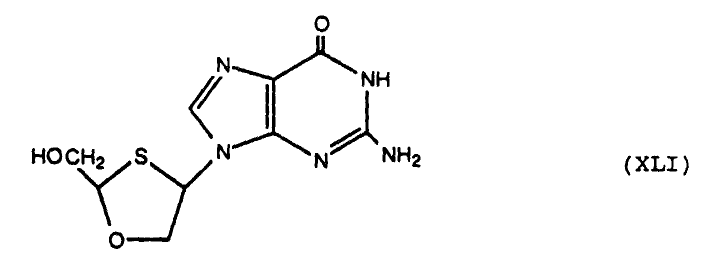

A mixture of cis-2-hydroxymethyl-4-(2'-amino-6'-chloropurin-9'-yl)-1,3-oxathiolane (100 mg), sodium hydroxide (3 g), water (5 ml) and methanol (20 ml) was heated overnight at refluxing (16 hours) and cooled to room temperature. The mixture was diluted with water (100 ml), neutralized with ion-exchange resin (in pyridinium form) and filtered. The residue was washed with water (100 ml), the combined aqueous solution evaporated in vacuo and the residue recrystallized in water ethanol to give 48 g (51%) the desired product. m.p.: Dec>280°C
UV: (H₂O) Lamda max: 270 and 250 nm
¹H-NMR: δ(ppm in DMSOd₆)
10.67 (b, 1H, -NH)
7.86 (s, 1H, C₈,-H)
6.55 (b, 2H, C₂,-NH₂, D₂O-exchange)
6.06 (d, 1H, C₄-H, J = 3.9 Hz)
5.40 (b, 1H, C₂-CH₂OH, D₂O-exchange)
5.29 (t, 1H, C₂-H, J = 4.6 Hz)
4.61 (d, 1H, C₅-H, J = 10.4 Hz)
4.05 (dd, 1H, C₅-H, J = 4.4 and 10.8 Hz)
3.79 (m, 2H, C₂-CH₂OH)

### Example 22

### Antiviral Activity: MT-4 Formazan Assay

Anti-HIV-1 antiviral activity was determined in MT-4 cells. A suspension of cells (approximately 10⁶ cells/ml) in RPMI 1640 growth medium was infected with HIV-1 strain RF at a M.O.I. of 10⁻³ infectious units/cell. An uninfected cell suspension was prepared in parallel to evaluate drug-induced cytotoxicity. The two suspensions were incubated for 90 minutes at room temperature. Test compounds were serially diluted in 10-fold decrements from 100 µg/ml to 0.01 µg/ml (final concentrations in two 96 well microtitre plates. 20 µl of infected cell suspension were inoculated into each well of one of the plates (anti-viral), while 20 µl of uninfected cell suspension were added to each well of the second plate (cytotoxicity). The plates were then incubated for 7 days at 37°C. After incubation, 10 µl of 3-(4,5-dimethylthiazol-2-yl)-2,5-diphenyltetrazolium bromide (MTT) at 20 mg/ml was added to all wells and the plates incubated for a further 90 minutes at 37°C.

150 µl of 10% (v/v) alcoholic Triton X-100 was then added and the cells resuspended. After 15 minutes at room temperature, the plates were analyzed in a Multiskan MC reader at 405 nm. Conversion of yellow MMT to its formazan derivative is maximum in uninfected cells, and absent in untreated infected cells. The optical density values for the cytotoxicity controls and the antiviral test wells were graphically plotted and the dose of compounds required to inhibit the conversion of MMT to 50% of the untreated uninfected controls was calculated. In this way, both the 50% cytotoxic dose (CD 50%) and the 50% anti-viral dose (ID 50%) can be calculated. Table 1 shows CD 50% and ID 50% values obtained for cis-2-hydroxymethyl-4-(adenin-9'-yl) -1,3-oxathiolane and 2',3'-dideoxyinosine.

**Table 1**

| Compound | CD 50% | ID 50% |
|---|---|---|
| cis-XIII | 100 µg/ml | 2.1 µg/ml |
| ddI | 100 µg/ml | 2.3 µg/ml |

## Claims (Claims for the following Contracting State(s): AT, BE, CH, DE, DK, FR, GB, IT, LI, LU, NL, SE)

1. A compound of formula (I), the geometric and optical isomers thereof, and mixtures of those isomers: 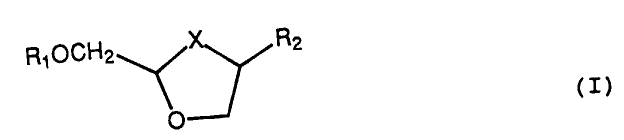 wherein:
X is selected from the group consisting of S, S=O, and SO₂;
R₁ is hydrogen; and
R₂ is selected from the group consisting of 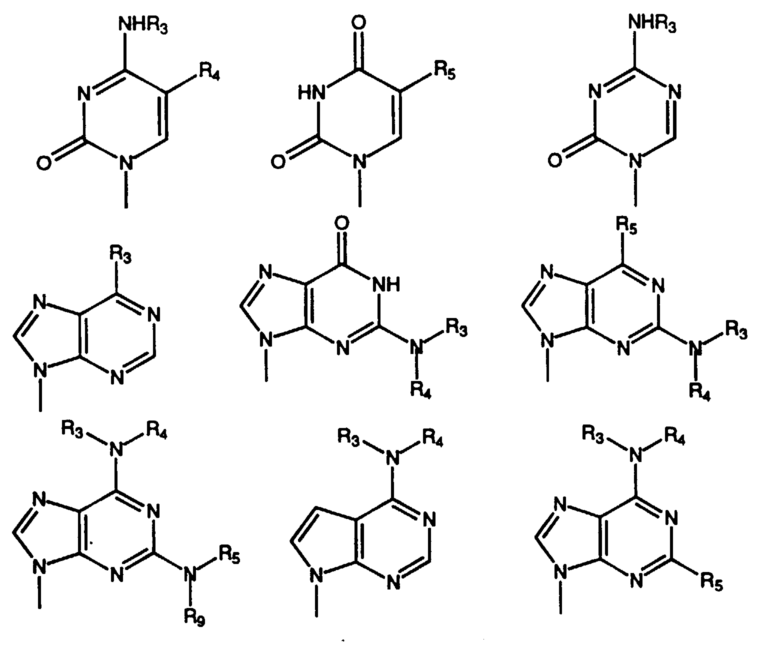 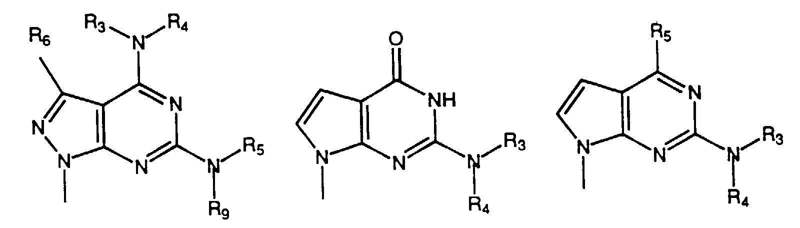 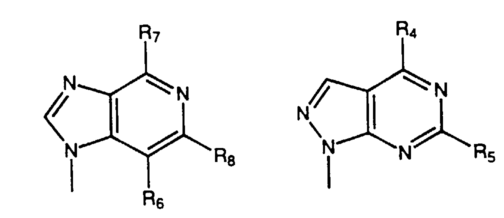
wherein:
R₃ is selected from the group consisting of hydrogen, C₁₋₁₀ acyl, hydroxyl, C₁₋₆ alkyl, and up to C₆ alkenyl or alkynyl;
R₄ and R₅ are independently selected from the group consisting of hydrogen, hydroxymethyl, trifluoromethyl, C₁₋ ₆ alkyl, up to C₆ alkenyl or alkynyl, bromine, chlorine, fluorine, and iodine;
R₆ is selected from the group consisting of hydrogen, bromine, chlorine, fluorine, iodine, cyano, carboxy, carboxamide, ethoxycarbonyl, carbamoyl, and thiocarbamoyl;
R₇ and R₈ are independently selected from the group consisting of hydrogen, bromine, chlorine, fluorine, iodine, amino, and hydroxy; and
R₉ is selected from the group consisting of hydrogen, C₁₋₁₀ acyl, hydroxyl, C₁₋₆ alkyl, and up to C₆ alkenyl or alkynyl; and pharmaceutically acceptable salts, esters or salts of such esters thereof which, upon administration to the recipient, are capable of providing (directly or indirectly) a compound of formula (I) or an antivirally active metabolite or residue thereof.

2. A compound according to claim 1 wherein R₂ is selected from the group consisting of:

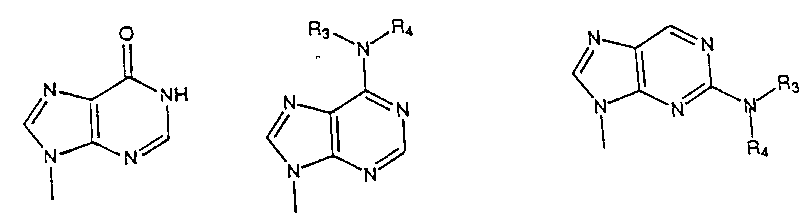

3. The compound according to claims 1 or 2 in the form of its cis isomer.

4. The compound according to claims 1 or 2 selected from the group consisting of:
cis-2-hydroxymethyl-4-(cytosin-1'-yl)-1,3-oxathiolane, trans-2-hydroxymethyl-4-(cytosin-1'-yl)-1,3-oxathiolane, and mixtures thereof;
cis-2-hydroxymethyl-4-(N₄'-acetyl-cytosin-1'-yl)-1,3-oxathiolane, trans-2-hydroxymethyl-4-(N₄'-acetyl-cytosin-1'-yl)-1,3-oxathiolane, and mixtures thereof;
cis-2-hydroxymethyl-4-(uracil-1'-yl)-1,3-oxathiolane, trans-2-hydroxymethyl-4-(uracil-1'-yl)-1,3-oxathiolane, and mixtures thereof;
cis-2-hydroxymethyl-4-(thymin-1'-yl)-1,3-oxathiolane, trans-2-hydroxymethyl-4-(thymin-1'-yl)-1,3-oxathiolane, and mixtures thereof;
cis-2-hydroxymethyl-4-(6'-chloropurin-9'-yl)-1,3-oxathiolane, trans-2-hydroxymethyl-4-(6'-chloropurin-9'-yl)-1,3-oxathiolane, and mixtures thereof;
cis-2-hydroxymethyl-4-(adenin-9'-yl)-1,3-oxathiolane, trans-2-hydroxymethyl-4-(adenin-9'-yl)-1,3-oxathiolane, and mixtures thereof;
cis-2-hydroxymethyl-3-oxo-4-(adenin-9'-yl)-1,3-oxathiolane;
cis-2-hydroxymethyl-4-(6'-N-methylamino-purin-9'-yl)-1,3-oxathiolane;
cis-2-hydroxymethyl-4-(6'-N,N-dimethylamino-purin-9'-yl)-1,3-oxathiolane, trans-2-hydroxymethyl-4-(6'-N,N-dimethylamino-purin-9'-yl)-1,3-oxathiolane, and mixtures thereof;
cis-2-hydroxymethyl-4-(2'-amino-6'-chloro-purin-9'-yl)-1,3-oxathiolane, trans-2-hydroxymethyl-4-(2'-amino-6-chloro-purin-9'-yl)-1,3-oxathiolane, and mixtures thereof;
cis-2-hydroxymethyl-4-(2',6'-diamino-purin-9'-yl)-1,3-oxathiolane, trans-2-hydroxymethyl-4-(2',6'-diamino-purin-9'-yl)-1,3-oxathiolane, and mixtures thereof;
cis-2-hydroxymethyl-4-(guanin-9'-yl)-1,3-oxathiolane; and pharmaceutically acceptable derivatives thereof in the form of a racemic mixture or single enantiomer.

5. The compound according to claims 1 or 2 in the form of a racemic mixture.

6. The compound according to claims 1 or 2 substantially in the form of a single enantiomer.

7. A pharmaceutical formulation comprising a pharmaceutically effective amount of a compound according to claims 1 or 2 and a pharmaceutically acceptable carrier.

8. The pharmaceutical formulation according to claim 7 additionally comprising a further therapeutic agent.

9. An ester of formula (IV) the geometric and optical isomers thereof, and mixtures of those isomers: 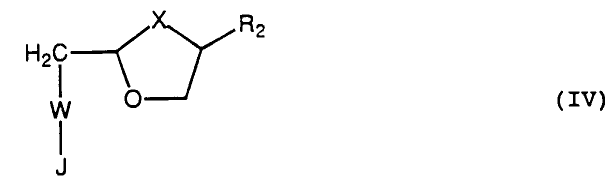 wherein:
W is selected from the group consisting of PO₄⁻, SPO₃⁻, and -O-C(O)-(CH₂)ₙ-C(O)-O- where n is an integer of 1 to 10;
J is any nucleoside or nucleoside analog or derivative thereof;
X is selected from the group consisting of S, S=O and SO₂,
R₂ is as defined in claims 1 or 2.

10. The ester according to claim 9 wherein J is:

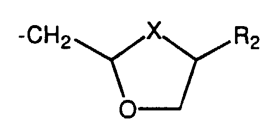

11. An intermediate of formula (Ic), the geometric and optical isomers thereof, and mixtures of those isomers, useful for the production of 1,3-oxathiolanes with antiviral properties: 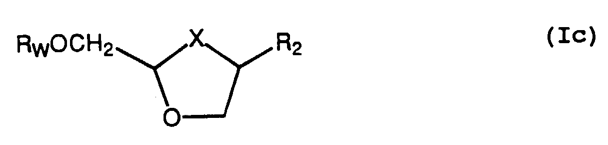 wherein:
X is selected from the group consisting of S, S=O, and SO₂;
R_{w} is selected from the group consisting of trisubstituted silyl, C₁₋₆ alkyl, aralkyl, and C₁₋₁₆ acyl; and
R₂ is as defined in claims 1 or 2.

12. The intermediate according to claim 11 wherein R_{w} is selected from the group consisting of benzyl, trityl, benzoyl and a benzoyl which may be substituted in any position by at least one group selected from the group consisting of bromine, chlorine, fluorine, iodine, C₁₋₆ alkyl, C₁₋₆ alkoxy, nitro and trifluoromethyl.

13. The intermediate according to claim 11 useful for the production of 1,3-oxathiolanes with antiviral properties selected from the group consisting of:
cis-2-benzoyloxymethyl-4-(cytosin-1'-yl)-1,3-oxathiolane, trans-2-benzoyloxymethyl-4-(cytosin-1'-yl)-1,3-oxathiolane, and mixtures thereof;
cis-2-benzoyloxymethyl-4-(N₄'-acetyl-cytosin-1'-yl)-1,3-oxathiolane, trans-2-benzoyloxymethyl-4-(N₄'-acetyl-cytosin-1'-yl)-1,3-oxathiolane, and mixtures thereof;
cis-2-benzoyloxymethyl-4-(uracil-1'-yl)-1,3-oxathiolane, trans-2-benzoyloxymethyl-4-(uracil-1'-yl)-1,3-oxathiolane, and mixtures thereof;
cis-2-benzoyloxymethyl-4-(thymin-1'-yl)-1,3-oxathiolane, trans-2-benzoyloxymethyl-4-(thymin-1'-yl)-1,3-oxathiolane, and mixtures thereof;
cis-2-benzoyloxymethyl-4-(6'-chloropurin-9'-yl)-1,3-oxathiolane, trans-2-benzoyloxymethyl-4-(6'-chloropurin-9'-yl)-1,3-oxathiolane, and mixtures thereof;
cis-2-benzoyloxymethyl-4-(adenin-9'-yl)-1,3-oxathiolane, trans-2-benzoyloxymethyl-4-(adenin-9'-yl)-1,3-oxathiolane;
cis-2-benzoyloxymethyl-4-(2'-amino-6'-chloro-purin-9'-yl)-1,3-oxathiolane, trans-2-benzoyloxymethyl-4-(2'-amino-6'-chloro-purin-9'-yl)-1,3-oxathiolane, and mixtures thereof.

14. An intermediate of formula (Id), the geometric and optical isomers thereof, and mixtures of those isomers, useful for the production of 1,3-oxathiolanes with antiviral properties: 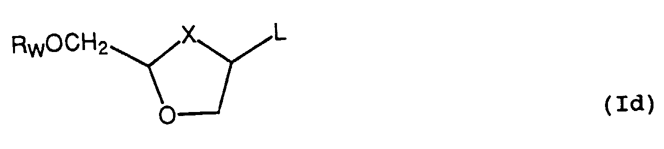 wherein:
X is selected from the group consisting of S, S=O, and SO₂;
R_{w} is selected from the group consisting of trisubstituted silyl, C₁₋₆ alkyl, aralkyl, and C₁₋₁₆ acyl; and
L is a leaving group or hydrogen.

15. The intermediate according to claim 14 wherein R_{w} is selected from the group consisting of benzyl, trityl, benzoyl and a benzoyl which may be substituted in any position by at least one group selected from the group consisting of bromine, chlorine, fluorine, iodine, C₁₋₆ alkyl, C₁₋₆ alkoxy, nitro and trifluoromethyl.

16. The intermediate according to claim 14 useful for the production of 1,3-oxathiolanes with antiviral properties selected from the group consisting of:
2-benzoyloxymethyl-1,3-oxathiolane;
cis-2-benzoyloxymethyl-1-oxo-1,3-oxathiolane, trans-2-benzoyloxymethyl-1-oxo-1,3-oxathiolane, and mixtures thereof;
cis-2-benzoyloxymethyl-4-acetoxy-1,3-oxathiolane, trans-2-benzoyloxymethyl-4-acetoxy-1,3-oxathiolane, and mixtures thereof;

17. A process for preparing a 1,3-oxathiolane compound of formula (I), the geometric and optical isomers thereof, and mixtures of those isomers: 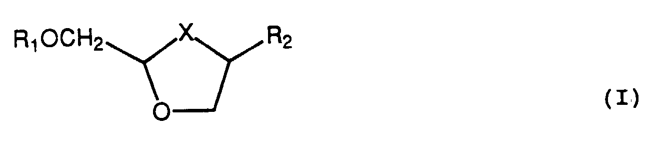 wherein:
X is selected from the group consisting of S, S=O, and SO2;
R₁ is hydrogen; and
R₂ is as defined in claims 1 or 2;
the process comprising the steps of:
a) condensing an aldehyde having of the formula R_{w}OCH₂CHO, wherein R_{w} is selected from the group consisting of trisubstituted silyl, C₁₋₆ alkyl, aralkyl, and C₁₋₁₆ acyl, with 2-mercaptoethanol in an organic solvent containing an acid catalyst to produce an intermediate of the formula: 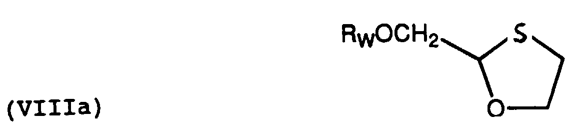
b) treating the intermediate of step (a) with a peracid to give a corresponding sulfoxide;
c) treating the intermediate of step (b) with an anhydride of the formula (RₓCO)₂O, wherein Rₓ is C₁₋₆ alkyl, in the presence of a buffer to produce an intermediate of the formula: 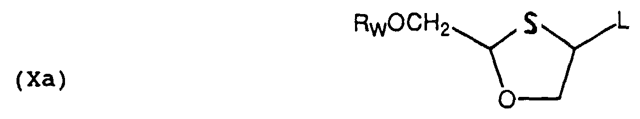 wherein L is OCORₓ or OH;
d) treating the intermediate of step (c) with an appropriate silylated pyrimidine or purine base or analogue thereof corresponding to R₂, in the presence of a Lewis acid and hydrolyzing the R_{w} function to produce a compound of the formula (I) wherein X=S; and
e) optionally oxidizing the X=S group of the compound of formula (I) obtained in step (d) to obtain another compound of formula (I) wherein X=SO or SO₂.

## Claims (Claims for the following Contracting State(s): ES, GR)

1. A process for preparing an intermediate of formula (Xa), the geometric and optical isomers thereof, and mixtures of those isomers, useful for the production of 1,3-oxathiolanes with antiviral properties: 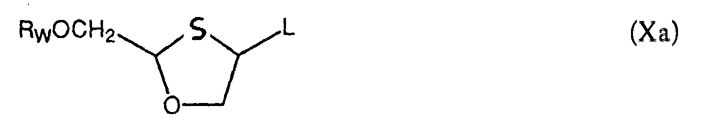 wherein:
R_{w} is selected from the group consisting of trisubstituted silyl, C₁₋₆ alkyl, aralkyl, and C₁₋₁₆ acyl; and
L is OCORₓ or OH;
the process comprising the steps of:
a) condensing an aldehyde having of the formula R_{w}OCH₂CHO with 2-mercaptoethanol in an organic solvent containing an acid catalyst to produce an intermediate of the formula: 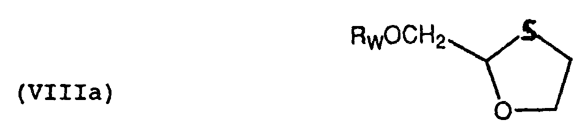
b) treating the intermediate of step (a) with a peracid to give a corresponding sulfoxide;
c) treating the intermediate of step (b) with an anhydride of the formula (RₓCO)₂O, wherein Rₓ is C₁₋₆ alkyl, in the presence of a buffer.

2. A process according to claim 1 for the preparation of an intermediate wherein R_{w} is selected from the group consisting of benzyl, trityl, benzoyl and a benzoyl which may be substituted in any position by at least one group selected from the group consisting of bromine, chlorine, fluorine, iodine, C₁₋₆ alkyl, C₁₋₆ alkoxy, nitro and trifluoromethyl.

3. A process according to claim 1 for the preparation of an intermediate useful for the production of 1,3-oxathiolanes with antiviral properties selected from the group consisting of:
2-benzoyloxymethyl-1,3-oxathiolane;
cis-2-benzoyloxymethyl-1-oxo-1,3-oxathiolane, trans-2-benzoyloxymethyl-1-oxo-1,3-oxathiolane, and mixtures thereof;
cis-2-benzoyloxymethyl-4-acetoxy-1,3-oxathiolane, trans-2-benzoyloxymethyl-4-acetoxy-1,3-oxathiolane, and mixtures thereof.

4. A process for preparing an intermediate of formula (Ic'), the geometric and optical isomers thereof, and mixtures of those isomers, useful for the production of 1,3-oxathiolanes with antiviral properties: 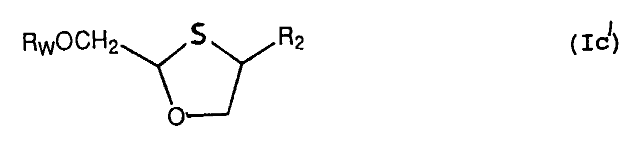 wherein:
R_{w} is as defined in claim 1;
R₂ is selected from the group consisting of 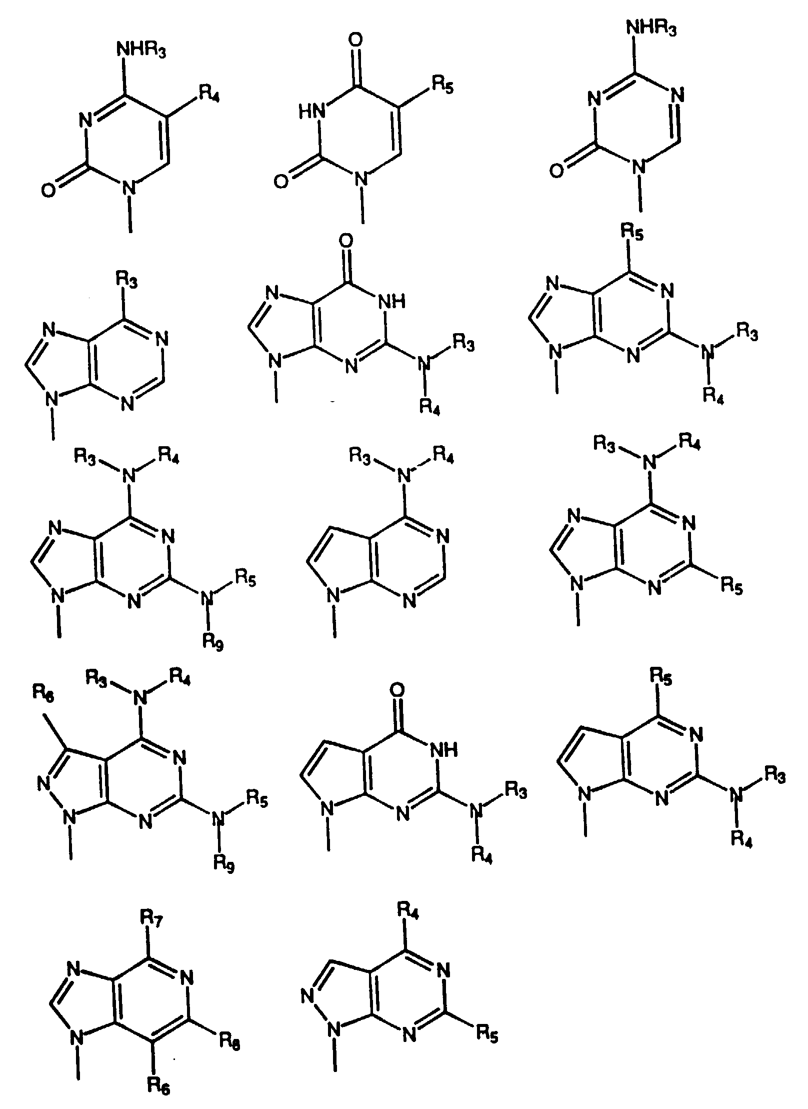
wherein:
R₃ is selected from the group consisting of hydrogen, c_{1-10,} acyl, hydroxyl, C₁₋₆ alkyl, and up to C₆ alkenyl or alkynyl;
R₄ and R₅ are independently selected from the group consisting of hydrogen, hydroxymethyl, trifluoromethyl, C₁₋ 6 alkyl, up to C₆ alkenyl or alkynyl, bromine, chlorine, fluorine, and iodine;
R₆ is selected from the group consisting of hydrogen, bromine, chlorine, fluorine, iodine, cyano, carboxy, carboxamide, ethoxycarbonyl, carbamoyl, and thiocarbamoyl;
R₇ and R₈ are independently selected from the group consisting of hydrogen, bromine, chlorine, fluorine, iodine, amino, and hydroxy; and
R₉ is selected from the group consisting of hydrogen, C₁₋₁₀ acyl, hydroxyl, C₁₋₆ alkyl, and up to C₆ alkenyl or alkynyl;
the process comprising a process according to claim 1 and further comprising the step of:
d1) treating the intermediate of step (c) with an appropriate silylated pyrimidine or purine base or analogue thereof corresponding to R₂, in the presence of a Lewis acid.

5. A process according to claim 4 wherein R₂ is selected from the group consisting of:

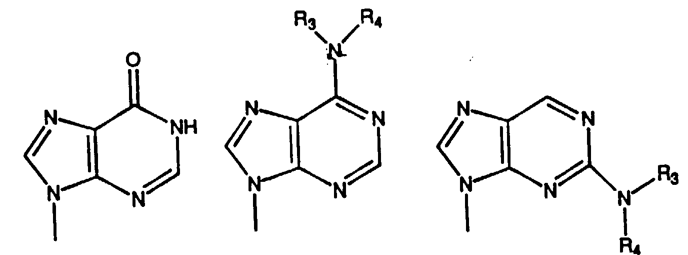

6. A process according to claims 4 or 5 for preparation of an intermediate wherein R_{w} is selected from the group consisting of benzyl, trityl, benzoyl and a benzoyl which may be substituted in any position by at least one group selected from the group consisting of bromine, chlorine, fluorine, iodine, C₁₋₆ alkyl, C₁₋₆ alkoxy, nitro and trifluoromethyl.

7. A process according to claims 4 or 5 for the preparation of an intermediate useful for the production of 1,3-oxathiolanes with antiviral properties selected from the group consisting of:
cis-2-benzoyloxymethyl-4-(cytosin-1'-yl)-1,3-oxathiolane, trans-2-benzoyloxymethyl-4-(cytosin-1'-yl)-1,3-oxathiolane, and mixtures thereof;
cis-2-benzoyloxymethyl-4-(N₄'**-**acetyl-cytosin-1'-yl)-1,3-oxathiolane, trans-2-benzoyloxymethyl-4-(N₄'-acetyl-cytosin-1'-yl)-1,3-oxathiolane, and mixtures thereof;
cis-2-benzoyloxymethyl-4-(uracil-1'-yl)-1,3-oxathiolane, trans-2-benzoyloxymethyl-4-(uracil-1'-yl)-1,3-oxathiolane, and mixtures thereof;
cis-2-benzoyloxymethyl-4-(thymin-1'-yl)-1,3-oxathiolane, trans-2-benzoyloxymethyl-4-(thymin-1'-yl)-1,3-oxathiolane, and mixtures thereof;
cis-2-benzoyloxymethyl-4-(6'-chloropurin-9'-yl)-1,3-oxathiolane, trans-2-benzoyloxymethyl-4-(6'-chloropurin-9'-yl)-1,3-oxathiolane, and mixtures thereof;
cis-2-benzoyloxymethyl-4 (adenin-9'-yl)-1,3-oxathiolane, trans-2-benzoyloxymethyl-4-(adenin-9'-yl)-1,3-oxathiolane;
cis-2-benzoyloxymethyl-4-(2'-amino-6'-chloro-purin-9'-yl)-1,3-oxathiolane, trans-2-benzoyloxymethyl-4-(2'-amino-6'-chloro-purin-9'-yl)-1,3-oxathiolane, and mixtures thereof.

8. A process for preparing a 1,3-oxathiolane compound of formula (I), the geometric and optical isomers thereof, mixtures of those isomers, and pharmaceutically acceptable salts, esters or salts of such esters thereof which, upon administration to the recipient, are capable of providing (directly or indirectly) a compound of formula (I) or an antivirally active metabolite or residue thereof: 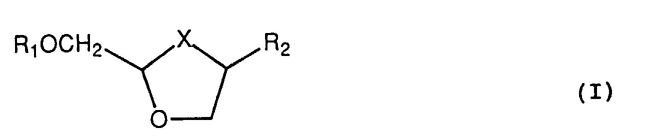 wherein:
R₁ is hydrogen;
X is selected from the group consisting of S, SO and SO₂;
R₂ is as defined in claims 4 or 5;
the process comprising a process according to claims 4 or 5 and further comprising the steps of:
d2) hydrolyzing the R_{w} function to produce a compound of the formula (I) wherein X=S; and
e) optionally oxidizing the X=S group of the compound of formula (I) obtained in step (d2) to obtain another compound of formula (I) wherein X=SO or SO₂.

9. A process according to claim 8 where the obtained compound is in the form of its cis isomer.

10. A process according to claim 8 wherein the obtained compound is selected from the group consisting of:
cis-2-hydroxymethyl-4-(cytosin-1'-yl)-1,3-oxathiolane, trans-2-hydroxymethyl-4-(cytosin-1'-yl)-1,3-oxathiolane, and mixtures thereof;
cis-2-hydroxymethyl-4-(N₄'-acetyl-cytosin-1'-yl)-1,3-oxathiolane, trans-2-hydroxymethyl-4-(N₄'-acetyl-cytosin-1'-yl)-1,3-oxathiolane, and mixtures thereof;
cis-2-hydroxymethyl-4- (uracil-1'-yl)-1,3-oxathiolane, trans-2-hydroxymethyl-4-(uracil-1'-yl)-1,3-oxathiolane, and mixtures thereof;
cis-2-hydroxymethyl-4-(thymin-1'-yl)-1,3-oxathiolane, trans-2-hydroxymethyl-4-(thymin-1'-yl)-1,3-oxathiolane, and mixtures thereof;
cis-2-hydroxymethyl-4-(6'-chloropurin-9'-yl)-1,3-oxathiolane, trans-2-hydroxymethyl-4-(6'-chloropurin-9'-yl)-1,3-oxathiolane, and mixtures thereof;
cis-2-hydroxymethyl-4-(adenin-9'-yl)-1,3-oxathiolane, trans-2-hydroxymethyl-4-(adenin-9'-yl)-1,3-oxathiolane, and mixtures thereof;
cis-2-hydroxymethyl-3-oxo-4-(adenin-9'-yl)-1,3-oxathiolane;
cis-2-hydroxymethyl-4-(6'-N-methylamino-purin-9'-yl)-1,3-oxathiolane;
cis-2-hydroxymethyl-4-(6'-N,N-dimethylamino-purin-9'-yl)-1,3-oxathiolane, trans-2-hydroxymethyl-4-(6'-N,N-dimethylamino-purin-9'-yl)-1,3-oxathiolane, and mixtures thereof;
cis-2-hydroxymethyl-4-(2'-amino-6'-chloro-purin-9'-yl)-1,3-oxathiolane, trans-2-hydroxymethyl-4-(2'-amino-6'-chloro-purin-9'-yl)-1,3-oxathiolane, and mixtures thereof;
cis-2-hydroxymethyl-4 (2',6'-diamino-purin-9'-yl)-1,3-oxathiolane, trans-2-hydroxymethyl-4-(2',6'-diamino-purin-9'-yl)-1,3-oxathiolane, and mixtures thereof;
cis-2 hydroxymethyl-4-(guanin-9'-yl)-1,3-oxathiolane; and pharmaceutically acceptable derivatives thereof in the form of a racemic mixture or single enantiomer.

11. A process according to claim 8 wherein the obtained compound is in the form of a racemic mixture.

12. A process according to claim 8 wherein the compound is substantially in the form of a single enantiomer.

13. A process according to claim 8 for preparing an ester of formula (IV), the geometric and optical isomers thereof, and mixtures of those isomers: 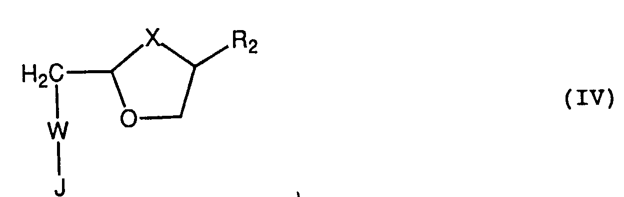
wherein: X and R₂ are as defined in claim 8;
W is selected from the group consisting of PO₄⁻, SPO₃⁻, and -O-C(O)-(CH₂)ₙ-C(O)-O- where n is an integer of 1 to 10;
J is any nucleoside or nucleoside analog or derivative thereof;
the process further comprising the step of:
f) esterifying the compound of formula (I).

14. A process according to claim 13 for preparing an ester wherein J is:

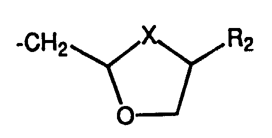

## Patentansprüche (Patentansprüche für folgende(n) Vertragsstaat(en): AT, BE, CH, DE, DK, FR, GB, IT, LI, LU, NL,SE)

1. Verbindung der Formel (I), deren geometrische und optische Isomere sowie Gemische dieser Isomeren 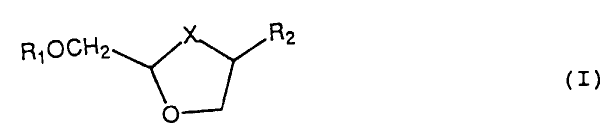 in der
X aus der aus S, S=O und SO₂ bestehenden Gruppe gewählt ist,
R₁ Wasserstoff bedeutet und
R₂ aus der Gruppe ausgewählt ist, die besteht aus: 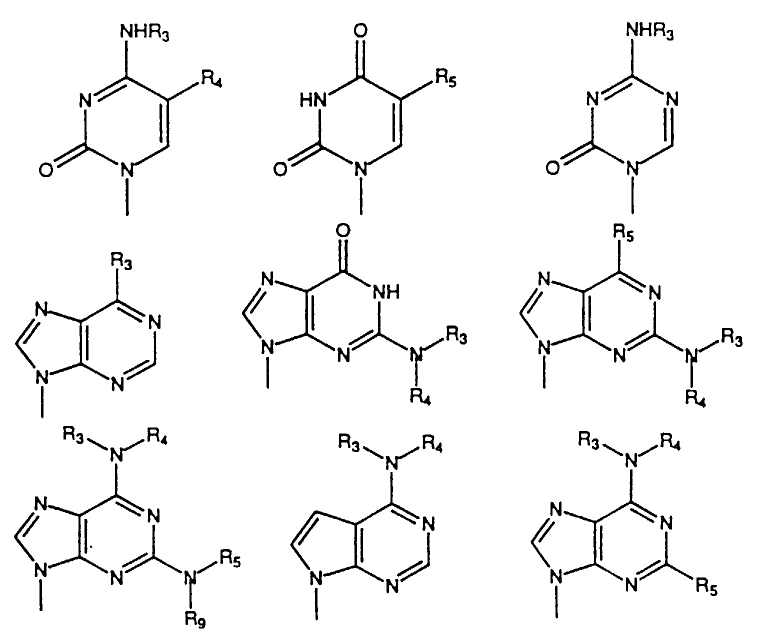 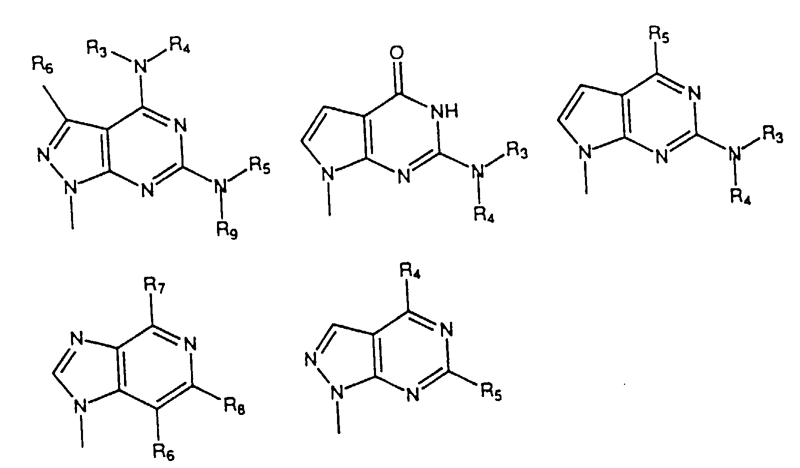
worin
R₃ aus der aus Wasserstoff, C1-10-Acyl, Hydroxyl, C₁₋₆-Alkyl sowie einem Alkenyl oder Alkinyl mit jeweils bis zu 6 Kohlenstoffatomen bestehenden Gruppe ausgewählt ist,
R₄ und R₅ unabhängig voneinander aus der aus Wasserstoff, Hydroxymethyl, Trifluormethyl, C₁₋₆-Alkyl, Alkenyl oder Alkinyl mit jeweils bis zu 6 Kohlenstoffatomen, Brom, Chlor, Fluor und Jod bestehenden Gruppe ausgewählt sind,
R₆ aus der aus Wasserstoff, Brom, Chlor, Fluor, Jod, Cyano, Carboxy, Carboxamid, Ethoxycarbonyl, Carbamoyl und Thiocarbamoyl bestehenden Gruppe ausgewählt ist,
R₇ und R₈ unabhängig voneinander aus der aus Wasserstoff, Brom, Chlor, Fluor, Jod, Amino und Hydroxy bestehenden Gruppe gewählt sind und
R₉ aus der aus Wasserstoff, C1-10-Acyl, Hydroxyl, C₁₋₆-Alkyl sowie Alkenyl und Alkinyl mit jeweils bis zu 6 Kohlenstoffatomen bestehenden Gruppe ausgewählt ist, sowie deren pharmazeutisch annehmbare Salze, Ester oder Salze dieser Ester, die bei Verabreichung an den Empfänger (direkt oder indirekt) eine Verbindung der Formel (1) oder einen antiviral wirkenden Metaboliten oder Rest derselben zur Verfügung stellen können.

2. Verbindung gemäß Anspruch 1, in der R₁ aus der Gruppe gewählt ist, die besteht aus:

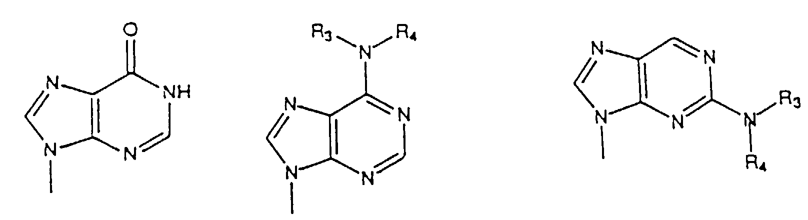

3. Verbindung gemäß Anspruch 1 oder 2 in der Form des cis-Isomeren.

4. Verbindung gemäß Anspruch 1 oder 2, ausgewählt aus der Gruppe, bestehend aus:
cis-2-Hydroxymethyl-4-(cytosin-1'-yl)-1,3-oxathiolan,
trans-2-Hydroxymethyl-4-(cytosin-1'-yl)-1,3-oxathiolan und deren Gemischen,
cis-2-Hydroxymethyl-4-(N₄'-acetylcytosin-1'-yl)-1,3-oxathiolan,
trans-2-Hydroxymethyl-4-(N₄'-acetylcytosin-1'-yl)-1,3-oxathiolan und deren Gemischen,
cis-2-Hydroxymethyl-4-(uracil-1'-yl)-1,3-oxathiolan,
trans-2-Hydroxymethyl-4-(uracil-1'-yl)-1,3-oxathiolan und deren Gemischen,
cis-2-Hydroxymethyl-4-(thymin-1'-yl)-1,3-oxathiolan,
trans-2-Hydroxymethyl-4-(thymin-1'-yl)-1,3-oxathiolan und deren Gemischen,
cis-2-Hydroxymethyl-4-(6'-chlorpurin-9'-yl)-1,3-oxathiolan,
trans-2-Hydroxymethyl-4-(6'-chlorpurin-9'-yl)-1,3-oxathiolan und deren Gemischen,
cis-2-Hydroxymethyl-4-(adenin-9'-yl)-1,3-oxathiolan,
trans-2-Hydroxymethyl-4-(adenin-9'-yl)-1,3-oxathiolan und deren Gemischen,
cis-2-Hydroxymethyl-3-oxo-4-(adenin-9'-yl)-1,3-oxathiolan,
cis-2-Hydroxymethyl-4-(6'-N-methylaminopurin-9'-yl)-1,3-oxathiolan,
cis-2-Hydroxymethyl-4-(6'-N,N-dimethylaminopurin-9'-yl)-1,3-oxathiolan,
trans-2-Hydroxymethyl-4-(6'-N,N-dimethylaminopurin-9'-yl)-1,3-oxathiolan und deren Gemischen,
cis-2-Hydroxymethyl-4-(2'-amino-6'-chlorpurin-9'-yl)-1,3-oxathiolan,
trans-2-Hydroxymethyl-4-(2'-amino-6'-chlorpurin-9'-yl)-1,3-oxathiolan und deren Gemischen,
cis-2-Hydroxymethyl-4-(2',6'-diaminopurin-9'-yl)-1,3-oxathiolan,
trans-2-Hydroxymethy1-4-(2',6'-diaminopurin-9'-yl)-1,3-oxathiolan und deren Gemischen,
cis-2-Hydroxymethyl-4-(guanin-9'-yl)-1,3-oxathiolan sowie deren pharmazeutisch annehmbaren Derivaten entweder in Form der Racemate oder eines einzelnen Enantiomeren.

5. Verbindung gemäß Anspruch 1 oder 2 in Form des Racemats.

6. Verbindung gemäß Anspruch 1 oder 2 im wesentlichen in Form eines einzelnen Enantiomeren.

7. Pharmazeutische Zubereitung, die eine pharmazeutisch wirksame Menge einer Verbindung gemäß Anspruch 1 oder 2 und einen pharmazeutisch annehmbaren Träger enthält.

8. Pharmazeutische Zubereitung gemäß Anspruch 7, die zusätzlich einen weiteren therapeutischen Wirkstoff enthält.

9. Ester der Formel (IV), dessen geometrische und optische Isomere sowie Gemische dieser Isomeren: 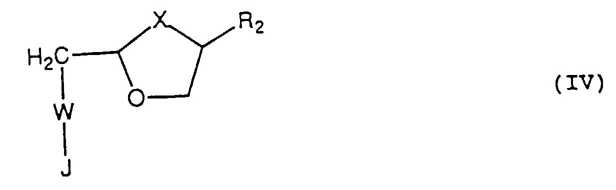 in der
W aus der aus PO₄⁻ SPO₃⁻ und -O-C(O)-(CH₂)ₙ-C(O)-O- bestehenden Gruppe ausgewählt ist, wobei n eine ganze Zahl von 1 bis 10 ist,
J ein beliebiges Nukleosid, Analogon eines Nukleosids oder ein Derivat derselben darstellt
X aus der aus S, S=O und SO₂ bestehenden Gruppe gewählt ist und
R₂ wie in Anspruch 1 oder 2 definiert ist.

10. Ester gemäß Anspruch 9, wobei J 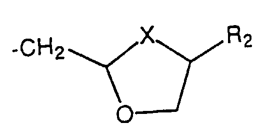 bedeutet.

11. Zwischenprodukt der Formel (Ic), dessen geometrische und optische Isomere sowie Gemische dieser Isomeren, das für die Herstellung der 1,3-Oxolane mit antiviralen Eigenschaften geeignet ist: 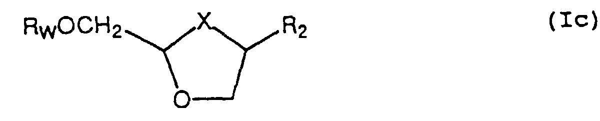 in der
X aus der aus S, S=O und SO₂ bestehenden Gruppe und
R_{w} aus der aus trisubstituiertem Silyl, C₁₋₆-Alkyl, Aralkyl und C₁₋₁₆-Acyl bestehenden Gruppe gewählt ist und
R₂ wie in den Ansprüchen 1 oder 2 definiert ist.

12. Zwischenprodukt gemäß Anspruch 11, in dem R_{W} aus der Gruppe gewählt ist, die aus Benzyl, Trityl, Benzoyl und gegebenenfalls in beliebiger Position mit einem aus der aus Brom, Chlor, Fluor, Jod, C₁₋₆-Alkyl, C₁₋₆-Alkoxy, Nitro und Trifluormethyl bestehenden Gruppe ausgewählten Substituenten substituiertem Benzoyl besteht.

13. Zwischenprodukt gemäß Anspruch 11, das zur Herstellung der 1,3-Oxathiolane mit antiviralen Eigenschaften geeignet ist und aus der aus
cis-2-Benzoyloxymethyl-4-(cytosin-1'-yl)-1,3-oxathiolan,
trans-2-Benzoyloxymethyl-4-(cytosin-1'-yl)-1,3-oxathiolan und deren Gemischen,
cis-2-Benzoyloxymethyl-4-(N₄'-acetycetylcytosin-1'-yl)-1,3-oxathiolan,
trans-2-Benzoyloxymethyl-4-(N₄'-acetylcytosin-1'-yl)-1,3-oxathiolan und deren Gemischen,
cis-2-Benzoyloxymethyl-4-(uracil-1'-yl)-1,3-oxathiolan,
trans-2-Benzoyloxymethyl-4-(uracil-1'-yl)-1,3-oxathiolan und deren Gemischen,
cis-2-Benzoyloxymethyl-4-(thymin-1'-yl)-1,3-oxathiolan,
trans-2-Benzoyloxymethyl-4-(thymin-1'-yl)-1,3-oxathiolan und deren Gemischen,
cis-2-Benzoyloxymethyl-4-(6'-chlorpurin- 9'-yl)-1,3-oxathiolan,
trans-2-Benzoyloxymethyl-4-(6'-chlorpurin-9'-yl)-1,3-oxathiolan und deren Gemischen,
cis-2-Benzoyloxymethyl-4-(adenin-9'-yl)-1,3-oxathiolan,
trans-2-Benzoyloxymethyl-4-(adenin-9'-yl)-1,3-oxathiolan und deren Gemischen und
cis-2-Benzoyloxymethyl-4-(2'-amino-6'-chlorpurin-9'-yl)-1,3-oxathiolan,
trans-2-Benzoyloxymethyl-4-(2'-amino-6'-chlorpurin-9'-yl)-1,3-oxathiolan und deren
Gemischen bestehenden Gruppe gewählt ist.

14. Zwischenprodukt der Formel (Id), dessen geometrische und optische Isomere sowie Gemische dieser Isomeren, das für die Herstellung der 1,3-Oxolane mit antiviralen Eigenschaften geeignet ist: 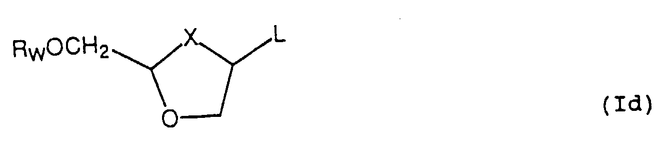 in der
X aus der aus S, S=O und SO₂ bestehenden Gruppe und
R_{w} aus der aus trisubstituiertem Silyl, C₁₋₆-Alkyl, Aralkyl und C₁₋₁₆-Acyl bestehenden Gruppe gewählt ist und
L eine Fluchtgruppe oder Wasserstoff darstellt.

15. Zwischenprodukt gemäß Anspruch 14, in dem R_{W} aus der Gruppe gewählt ist, die aus Benzyl, Trityl, Benzoyl und gegebenenfalls in beliebiger Position mit einen aus der aus Brom, Chlor, Fluor, Jod, C₁₋₆-Alkyl, C₁₋₆-Alkoxy, Nitro und Trifluormethyl bestehenden Gruppe ausgewählten Rest substituiertem Benzoyl besteht.

16. Zwischenprodukt gemäß Anspruch 14, das zur Herstellung der 1,3-Oxathiolane mit antiviralen Eigenschaften geeignet ist und aus der aus
2-Benzoyloxymethyl-1,3-oxathiolan,
cis-2-Benzoyloxymethyl-1-oxo-1,3-oxathiolan,
trans-2-Benzoyloxymethyl-1-oxo-1,3-oxathiolan und deren Gemischen sowie
cis-2-Benzoyloxymethyl-4-acetoxy-1,3-oxathiolan,
trans-2-Benzoyloxymethyl-4-acetoxy-1,3-oxathiolan und deren Gemischen
bestehenden Gruppe gewählt ist.

17. Verfahren zur Herstellung einer 1,3-Oxathiolanverbindung der Formel (1), von deren geometrischen und optischen Isomeren sowie Gemischen dieser Isomeren 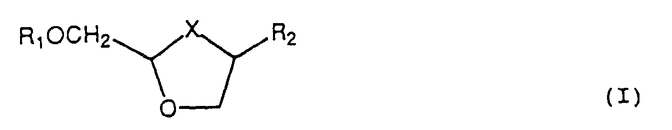 in der
aus der aus S, S=O und SO₂ bestehenden Gruppe gewählt ist,
R₁ Wasserstoff bedeutet und
R₂ wie in Anspruch 1 oder 2 definiert ist,
welches die folgenden Schritte umfaßt:
a) Kondensieren eines Aldehyds der Formel R_{W}OCH₂CHO, in der Rw aus der aus trisubstituiertem Silyl, C₁₋₆-Alkyl, Aralkyl und C₁₋₁₆-Acyl bestehenden Gruppe gewählt ist, mit 2-Mercaptoethanol in einem einen Säurekatalysator enthaltenden, organischen Lösungsmittel, um ein Zwischenprodukt der Formel 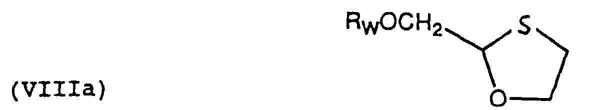 zu erzeugen,
b) Behandlung des Zwischenprodukts aus Schritt (a) mit einer Persäure zum Erhalt des entsprechenden Sulfoxids,
c) Behandlung des Zwischenprodukts aus Schritt (b) mit einem Anhydrid der Formel (R_{X}CO)₂O, in der R_{X} C₁₋₆-Alkyl bedeutet, und zwar in Anwesenheit eines Puffers, um ein Zwischenprodukt der Formel 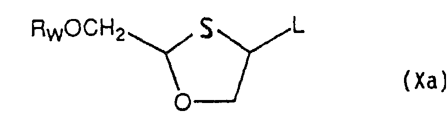 zu erzeugen, in der L OCOR_{X} oder OH darstellt,
d) Behandlung des Zwischenprodukts aus Schritt (c) mit einem passen silylierten Pyrimidin oder Purin oder einem R₂ entsprechenden Analogon derselben in Anwesenheit einer Lewis-Säure und Hydrolyse der funktionellen Gruppe R_{W} zum Erhalt einer Verbindung der Formel (I), in der X = S ist, und
e) gegebenenfalls Oxidation der Gruppe X (=S) der in Schritt (d) erhaltenen Verbindung der Formel (I), um eine andere Verbindung der Formel (I) zu erhalten, in der X SO oder SO₂ bedeutet.

## Patentansprüche (Patentansprüche für folgende(n) Vertragsstaat(en): ES, GR)

1. Verfahren zur Herstellung eines Zwischenprodukts der Formel (Xa), dessen geometrischer und optischer Isomere sowie von Gemischen dieser Isomeren, das für die Herstellung von 1,3-Oxathiolanen mit antiviralen Eigenschaften geeignet ist: 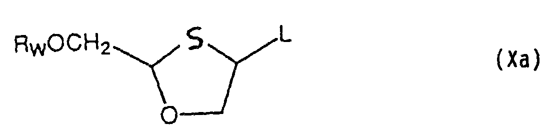 in der
R_{w} aus der aus trisubstituiertem Silyl, C₁₋₆-Alkyl, Aralkyl und C₁₋₁₆-Acyl bestehenden Gruppe gewählt ist und
L die Gruppe OCOR_{X} oder OH darstellt,
welches die folgenden Schritte umfaßt:
a) Kondensieren eines Aldehyds der Formel R_{W}OCH₂CHO mit 2-Mercaptoethanol in einem einen Säurekatalysator enthaltenden, organischen Lösungsmittel, um ein Zwischenprodukt der Formel 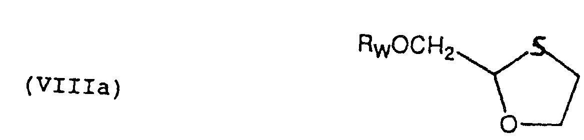 zu erzeugen,
b) Behandlung des Zwischenprodukts aus Schritt (a) mit einer Persäure zum Erhalt des entsprechenden Sulfoxids,
c) Behandlung des Zwischenprodukts aus Schritt (b) mit einem Anhydrid der Formel (R_{X}CO)₂O, in der R_{X} C₁₋₆-Alkyl bedeutet, und zwar in Anwesenheit eines Puffers.

2. Verfahren gemäß Anspruch 1 zur Herstellung eines Zwischenprodukts, bei dem R_{W} aus der Gruppe gewählt ist, die aus Benzyl, Trityl, Benzoyl und gegebenenfalls in beliebiger Position mit einem aus der aus Brom, Chlor, Fluor, Jod, C₁₋₆-Alkyl, C₁₋₆-Alkoxy, Nitro und Trifluormethyl bestehenden Gruppe ausgewählten Rest substituiertem Benzoyl besteht.

3. Verfahren gemäß Anspruch 1 zur Herstellung eines für die Produktion von 1,3-Oxathiolanen mit antiviralen Eigenschaften geeigneten Zwischenprodukts, das aus der aus
2-Benzoyloxymethyl-1,3-oxathiolan,
cis-2-Benzoyloxymethyl-1-oxo-1,3-oxathiolan, Gemischen sowie
trans-2-Benzoyloxymethyl-1-oxo-1,3-oxathiolan und deren Gemischen sowie
cis-2-Benzoyloxymethyl-4-acetoxy-1,3-oxathiolan,
trans-2-Benzoyloxymethyl-4-acetoxy-1,3-oxathiolan und deren Gemischen bestehenden Gruppe gewählt ist.

4. Verfahren zur Herstellung eines Zwischenprodukts der Formel (Ic), dessen geometrischer und optischer Isomere sowie von Gemischen dieser Isomeren, das für die Herstellung von 1,3-Oxathiolanen mit antiviralen Eigenschaften geeignet ist: 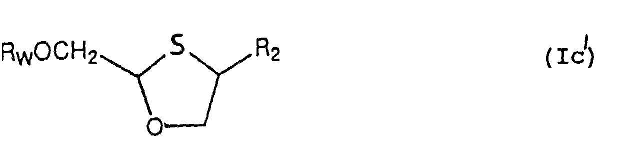 in der
R_{W} wie in Anspruch 1 definiert ist,
R₂ aus der aus 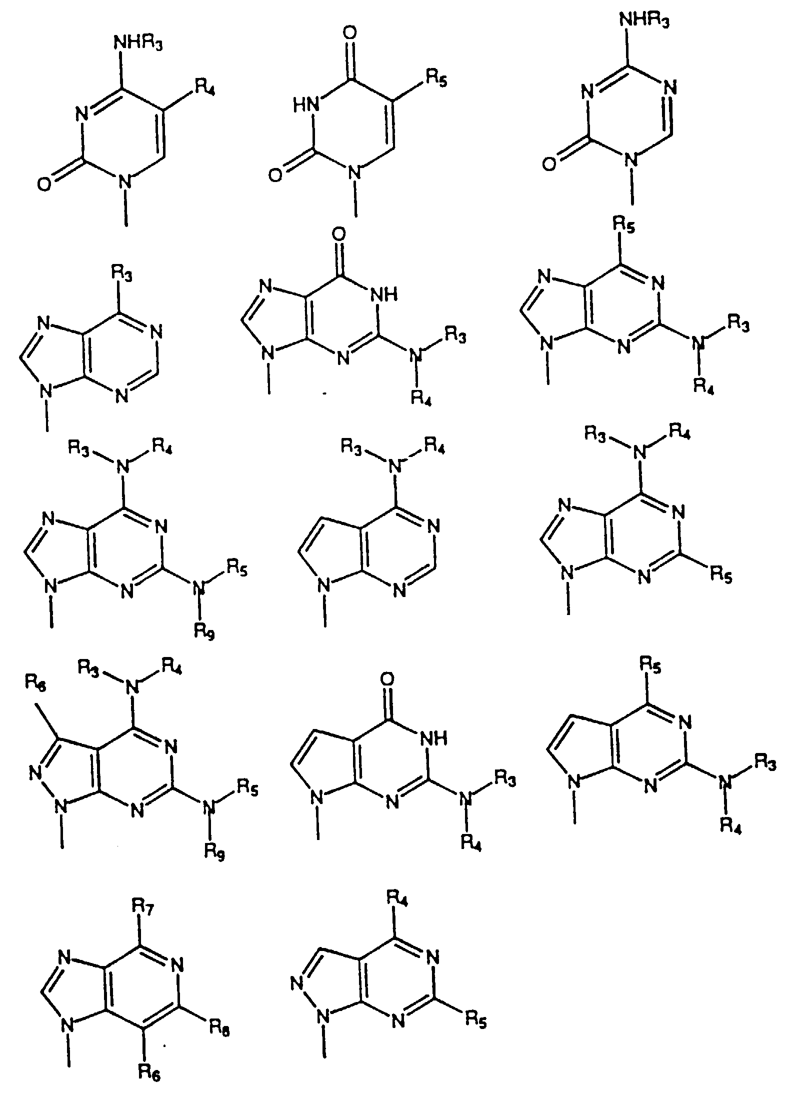 bestehenden Gruppe gewählt ist, worin
R₃ aus der aus Wasserstoff, C₁₋₁₀-Acyl, Hydroxyl, C₁₋₆-Alkyl sowie Alkenyl oder Alkinyl mit jeweils bis zu 6 Kohlenstoffatomen bestehenden Gruppe ausgewählt ist,
R₄ und R₅ unabhängig voneinander aus der aus Wasserstoff, Hydroxymethyl, Trifluormethyl, C₁₋₆-Alkyl, Alkenyl oder Alkinyl mit jeweils bis zu 6 Kohlenstoffatomen, Brom, Chlor, Fluor und Jod bestehenden Gruppe ausgewählt sind,
R₆ aus der aus Wasserstoff, Brom, Chlor, Fluor, Jod, Cyano, Carboxy, Carboxamid, Ethoxycarbonyl, Carbamoyl und Thiocarbamoyl bestehenden Gruppe ausgewählt ist,
R₇ und R₈ unabhängig voneinander aus der aus Wasserstoff, Brom, Chlor, Fluor, Jod, Amino und Hydroxy bestehenden Gruppe gewählt sind und
R₉ aus der aus Wasserstoff, C₁₋₁₀-Acyl, Hydroxyl, C₁₋₆-Alkyl sowie Alkenyl und Alkinyl mit jeweils bis zu 6 Kohlenstoffatomen bestehenden Gruppe ausgewählt ist und das Verfahren ein Verfahren gemäß Anspruch 1 umfaßt und zusätzlich folgenden Schritt d1) enthält:
d1) Behandlung des Zwischenprodukts aus Schritt (c) mit einem passend silylierten Pyrimidin oder Purin oder einem R₂ entsprechenden Analogon derselben in Anwesenheit einer Lewis-Säure.

5. Verfahren gemäß Anspruch 4, bei dem R₂ ausgewählt ist aus:

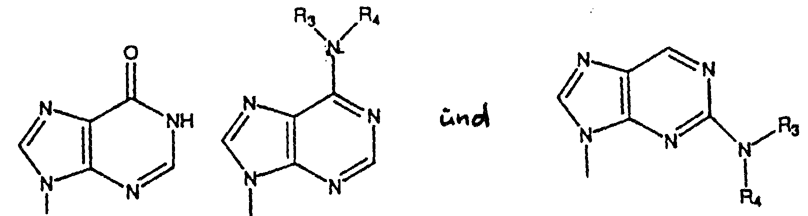

6. Verfahren gemäß Anspruch 4 oder 5 zur Herstellung eines Zwischenprodukts, bei dem R_{W} aus der Gruppe gewählt ist, die aus Benzyl, Trityl, Benzoyl und gegebenenfalls in beliebiger Position mit einem aus der aus Brom, Chlor, Fluor, Jod, C₁₋₆-Alkyl, C₁₋₆-Alkoxy, Nitro und Trifluormethyl bestehenden Gruppe ausgewählten Rest substituiertem Benzoyl besteht.

7. Verfahren gemäß Anspruch 4 oder 5 zur Herstellung eines für die Produktion von 1,3-Oxathiolanen mit antiviralen Eigenschaften geeigneten Zwischenprodukts, das aus der aus
cis-2-Benzoyloxymethyl-4-(cytosin-1'-yl)-1,3-oxathiolan,
trans-2-Benzoyloxymethyl-4-(cytosin-1'-yl)-1,3-oxathiolan und deren Gemischen,
cis-2-Benzoyloxymethyl-4-(N₄'-acetylcytosin-1'-yl)-1,3-oxathiolan,
trans-2-Benzoyloxymethyl-4-(N₄'-acetylcytosin-1'-yl)-1,3-oxathiolan und deren Gemischen,
cis-2-Benzoyloxymethyl-4-(uracil-1'-yl)-1,3-oxathiolan,
trans-2-Benzoyloxymethyl-4-(uracil-1'-yl)-1,3-oxathiolan und deren Gemischen,
cis-2-Benzoyloxymethyl-4-(thymin-1'-yl)-1,3-oxathiolan,
trans-2-Benzoyloxymethyl-4-(thymin-1'-yl)-1,3-oxathiolan und deren Gemischen,
cis-2-Benzoyloxymethyl-4-(6'-chlorpurin-9'-yl)-1,3-oxathiolan,
trans-2-Benzoyloxymethyl-4-(6'-chlorpurin-9'-yl)-1,3-oxathiolan und deren Gemischen,
cis-2-Benzoyloxymethyl-4-(adenin-9'-yl)-1,3-oxathiolan,
trans-2-Benzoyloxymethyl-4-(adenin-9'-yl)-1,3-oxathiolan und deren Gemischen und
cis-2-Benzoyloxymethyl-4-(2'-amino-6'-chlorpurin-9'-yl)-1,3-oxathiolan,
trans-2-Benzoyloxymethyl-4-(2'-amino-6'-chlorpurin-9'-yl)-1,3-oxathiolan und deren Gemischen bestehenden Gruppe gewählt ist.

8. Verfahren zur Herstellung einer 1,3-Oxathiolanverbindung der Formel (I), von deren geometrischen und optischen Isomeren sowie Gemischen dieser Isomeren und deren pharmazeutisch annehmbaren Salzen, Estern oder Salzen dieser Ester, die bei der Verabreichung an einen Empfänger (direkt oder indirekt) eine Verbindung der Formel (1) oder einen antiviral wirksamen Metaboliten oder Rest derselben zur Verfügung stellen können: 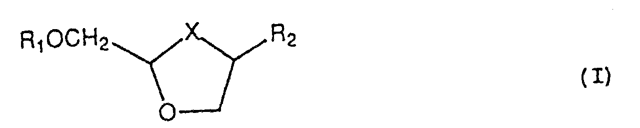 in der
R₁ Wasserstoff bedeutet,
X aus der aus S, SO und SO₂ bestehenden Gruppe gewählt ist,
R₂ wie in Anspruch 4 und 5 definiert ist und das Verfahren das Verfahren gemäß Anspruch 4 oder 5 umfaßt und zusätzlich folgende Schritte beinhaltet:
d2) Hydrolyse der funktionellen Grruppe R_{w}, zum Erhalt einer Verbindung der Formel (I), in der X S ist, und
e) gegebenenfalls Oxidation der Gruppe X (=S) der in Schritt (d) erhaltenen Verbindung der Formel (I), um eine andere Verbindung der Formel (I) zu erhalten, in der X SO oder SO₂ bedeutet.

9. Verfahren gemäß Anspruch 8, bei dem die erhaltene Verbindung in Form des cis-Isomeren vorliegt.

10. Verfahren gemäß Anspruch 8, bei dem die erhaltene Verbindung aus der Gruppe ausgewählt ist, die besteht aus:
cis-2-Hydroxymethyl-4-(cytosin-1'-yl)-1,3-oxathiolan,
trans-2-Hydroxymethyl-4-(cytosin-1'-yl)-1,3-oxathiolan und deren Gemischen,
cis-2-Hydroxymethyl-4-(N₄'-acetylcytosin-1'-yl)-1,3-oxathiolan,
trans-2-Hydroxymethyl-4-(N₄'-acetylcytosin-1'-yl)-1,3-oxathiolan und deren Gemischen,
cis-2-Hydroxymethyl-4-(uracil-1'-yl)-1,3-oxathiolan,
trans-2-Hydroxymethyl-4-(uracil-1'-yl)-1,3-oxathiolan und deren Gemischen,
cis-2-Hydroxymethyl-4-(thymin-1'-yl)-1,3-oxathiolan,
trans-2-Hydroxymethyl-4-(thymin-1'-yl)-1,3-oxathiolan und deren Gemischen,
cis-2-Hydroxymethyl-4-(6'-chlorpurin-9'-yl)-1,3-oxathiolan,
trans-2-Hydroxymethyl-4-(6'-chlorpurin-9'-yl)-1,3-oxathiolan und deren Gemischen,
cis-2-Hydroxymethyl-4-(adenin-9'-yl)-1,3-oxathiolan,
trans-2-Hydroxymethyl-4-(adenin-9'-yl)1,3-oxathiolan und deren Gemischen,
cis-2-Hydroxymethyl-3-oxo-4-(adenin-9'-yl)-1,3-oxathiolan,
cis-2-Hydroxymethyl4-(6'-N-methylaminopurin-9'-yl)**-**1,3-oxathiolan,
cis-2-Hydroxymethyl-4-(6'-N,N-dimethylaminopurin-9'-yl)-1,3-oxathiolan,
trans-2-Hydroxymethyl-4-(6'-N,N-dimethylaminopurin-9'-yl)-1,3-oxathiolan und deren Gemischen,
cis-2-Hydroxymethyl-4-(2'-amino-6'-chlorpurin-9'-yl)-1,3-oxathiolan,
trans-2-Hydroxymethyl-4-(2'-amino-6'-chlorpurin-9'-yl)-1,3-oxathiolan und deren Gemischen,
cis-2-Hydroxymethyl-4-(2',6'-diaminopurin-9'-yl)-1,3-oxathiolan,
trans-2-Hydroxymethyl-4-(2',6'-diaminopurin-9'-yl)-1,3-oxathiolan und deren Gemischen,
cis-2-Hydroxymethyl-4-(guanin-9'-yl)-1,3-oxathiolan sowie deren pharmazeutisch annehmbaren Derivaten entweder in Form der Racemate oder eines einzelnen Enantiomeren.

11. Verfahren gemäß Anspruch 8, bei dem die erhaltene Verbindung in Form eines Racemats vorliegt.

12. Verfahren gemäß Anspruch 8, bei dem die Verbindung im wesentlichen in Form eines einzelnen Isomeren vorliegt.

13. Verfahren gemäß Anspruch 8 zur Herstellung eines Esters der Formel (IV), von dessen geometrischen und optischen Isomeren sowie von Gemischen dieser Isomeren: 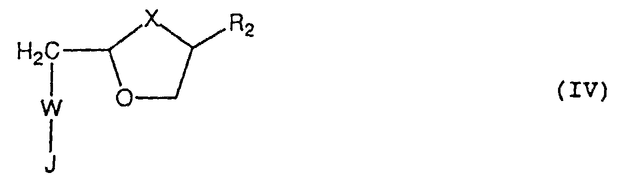 in der J
X und R₂ wie in Anspruch 8 definiert sind,
W aus der aus PO₄⁻, SPO₃⁻ und -O-C(O)-(CH₂)ₙ-C(O)-O- bestehenden Gruppe ausgewählt ist, wobei n eine ganze Zahl von 1 bis 10 ist und
J ein beliebiges Nukleosid, Analogon eines Nukleosids oder ein Derivat derselben darstellt,
welches zusätzlich den Schritt f) umfaßt:
f) Veresterung einer Verbindung der Formel (I).

14. Verfahren gemäß Anspruch 13 zur Herstellung eines Esters, in dem J bedeutet:

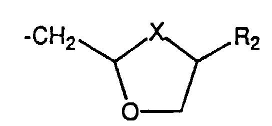

## Revendications (Revendications pour l'(les) Etat(s) contractant(s) suivant(s): AT, BE, CH, DE, DK, FR, GB, IT, LI, LU, NL, SE)

1. Composé de formule (I), isomères géométriques et optiques de celui-ci et mélanges de ces isomères : 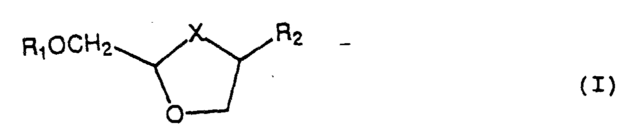 dans laquelle
X est choisi dans le groupe consistant en S, S=O et SO₂ ;
R₁ est un atome d'hydrogène, et
R₂ est choisi dans le groupe consistant en 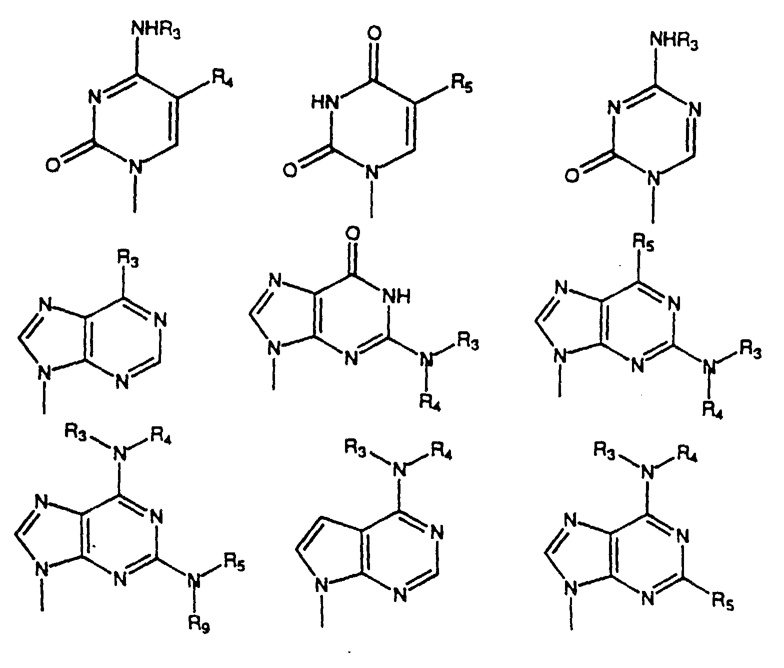 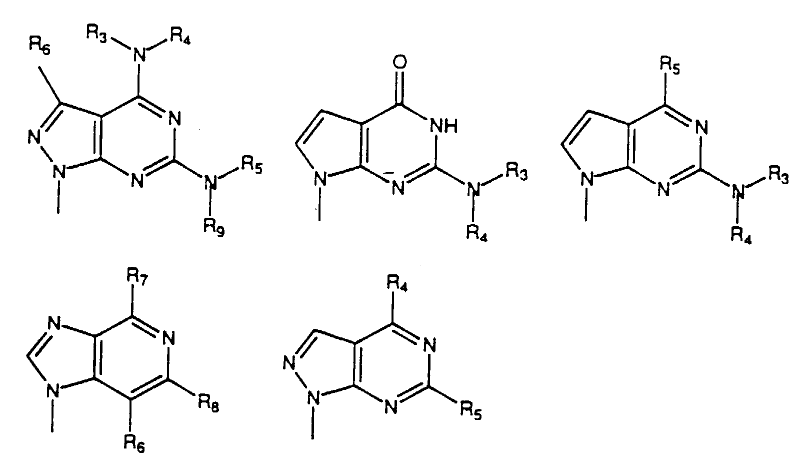
dans laquelle
R₃ est choisi dans le groupe consistant en atome d'hydrogène, groupe acyle en C₁₋₁₀, hydroxy, alkyle en C₁₋₆, alcényle ou alcynyle ayant jusqu'à 6 atomes de carbone ;
R₄ et R₅ sont indépendamment choisis dans le groupe consistant en atome d'hydrogène, groupe hydroxyméthyle, trifluorométhyle, alkyle en C₁₋₆, alcényle ou alcynyle ayant jusqu'à 6 atomes de carbone, atome de brome, de chlore, de fluor et d'iode ;
R₆ est choisi dans le groupe consistant en atome d'hydrogène, de brome, de chlore, de fluor, d'iode, groupe cyano, carboxy, carboxamide, étoxycarbonyle, carbamoyle et thiocarbamoyle ;
R₇ et R₈ sont indépendamment choisis dans le groupe consistant en atome d'hydrogène, de brome, de chlore, de fluor, d'iode, groupe amino et hydroxy ; et
R₉ est choisi dans le groupe consistant en atome d'hydrogène, groupe acyle en C₁₋₁₀, hydroxy, alkyle en C₁₋₆, alcényle ou alcynyle ayant jusqu'à 6 atomes de carbone ; et sels, esters ou sels de ces esters de ceux-ci acceptables du point de vue pharmaceutique qui, lorsqu'ils sont administrés au receveur, sont capables de fournir (directement ou indirectement) un composé de formule (I) ou un métabolite ou un résidu de celui-ci actif du point de vue anti-viral.

2. Composé suivant la revendication 1 dans lequel R₂ est choisi dans le groupe consistant en :

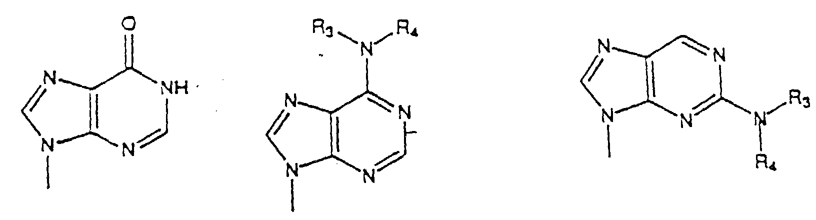

3. Composé suivant les revendications 1 ou 2, sous la forme de son isomère cis.

4. Composé suivant les revendications 1 ou 2, choisi dans le groupe consistant en :
cis-2-hydroxyméthyl-4-(cytosin-1'-yl)-1,3-oxathiolane, trans-2-hydroxy-méthyl-4-(cytosin-1'-yl)-1,3-oxathiolane et leurs mélanges ;
cis-2-hydroxyméthyl-4-(N₄'-acétyl-cytosin-1'-yl)-1,3-oxathiolane, trans-2-hydroxyméthyl-4-(N₄'-acétyl-cytosin-1'-yl)-1,3-oxathiolane, et leurs mélanges ;
cis-2-hydroxyméthyl-4-(uracyl-1'-yl)-1,3-oxathiolane, trans-2-hydroxyméthyl-4-(uracil-1'-yl)-1,3-oxathiolane, et leurs mélanges ;
cis-2-hydroxyméthyl-4-(thymin-1'-yl)-1,3-oxathiolane, trans-2-hydroxyméthyl-4-(thymin-1'-yl)-1,3-oxathiolane, et leurs mélanges ;
cis-2-hydroxyméthyl-4-(6'-chlompurin-9'-yl)-1,3-oxathiolane, trans-2-hydroxyméthyl-4-(6'-chloropufin-9'-yl)-1,3-oxathiolane, et leurs mélange ;
cis-2-hydroxyméthyl-4-(adenin-9'-yl)-1,3-oxathiolane, trans-2-hydroxyméthyl-4-(adenin-9'-yl)-1,3-oxathiolane, et leurs mélanges;
cis-2-hydroxyméthyl-3-oxo-4-(adenin-9'-yl)-1,3oxathiolane ;
cis-2-hydroxyméthyl-4-(6'-N-méthylamino-purin-9'-yl)-1,3oxathiolane;
cis-2-hydroxyméthyl-4-(6'-N,N-diméthylamino-purin-9'-yl)-1,3-oxathiolane, trans-2-hydroxyméthyl-4-(6'-N,N-diméthylamino-purin-9'-yl)-1,3-oxathiolane, et leurs mélanges ;
cis-2-hydroxyméthyl-4-(2'-amino-6'-chloro-purin-9'-yl)-1,3-oxathiolane, trans-2-hydroxyméthyl-4-(2'-amino-6'-chloro--purin-9'-yl)-1,3-oxathiolane, et leurs mélanges ;
cis-2-hydroxyméthyl-4-(2'-6'-diamino-purin-9'-yl)-1,3-oxathiolane, trans-2-hydroxyméthyl-4-(2'-6'-diamino-purin-9'-yl)-1,3-oxathiolane, et leurs mélanges ;
cis-2-hydroxyméthyl-4-(guanin-9'-yl)-1,3-oxathiolane; et dérivés de ceux-ci acceptables du point de vue pharmaceutique sous la forme d'un mélange racémique ou d'un énantiomère unique.

5. Composé suivant les revendications 1 ou 2, sous la forme d'un mélange racémique.

6. Composé suivant les revendications 1 ou 2, pratiquement sous la forme d'un énantiomère unique.

7. Formulation pharmaceutique comprenant une quantité efficace du point de vue pharmaceutique d'un composé suivant les revendications 1 ou 2 et un support acceptable du point de vue pharmaceutique.

8. Formulation pharmaceutique suivant la revendication 7, comprenant de plus un autre thérapeutique.

9. Ester de formule (IV), isomères géométriques et optiques de celui-ci et mélanges de ces isomères : 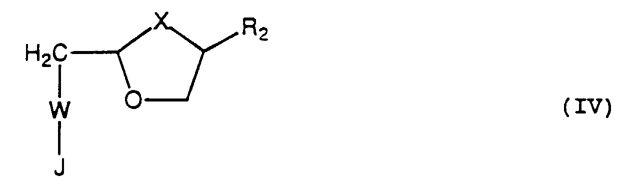 dans laquelle :
W est choisi dans le groupe consistant en PO₄⁻, SPO₃⁻ et O-C(O)-(CH₂)ₙ-C(O)-O- dans lequel n est en entier de 1 à 10 ;
J est un quelconque nucléoside ou un analogue de nucléoside ou dérivé de ceux-ci ;
X est choisi dans le groupe consistant en C, S=O et SO₂.
R₂ est tel que défini dans les revendications 1 ou 2.

10. Ester suivant la revendication 9, dans J est représenté par la formule :

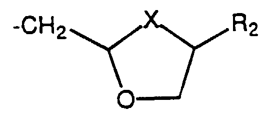

11. Intermédiaire de formule (Ic), isomères géométriques et optiques de celui-ci et mélanges de ces isomères utiles pour la production de 1,3-oxathiolane dotés de propriétés anti-virales : 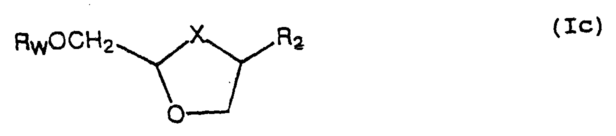 dans laquelle :
X est choisi dans le groupe consistant en S, S=O et SO₂.
R_{w} est choisi dans le groupe consistant en groupe silyle trisubstitué, alkyle en C₁₋₆, aralkyle et acyle en C₁₋₁₆ ; et
R₂ est tel que défini dans les revendications 1 ou 2.

12. Intermédiaire suivant la revendication 11, dans lequel R_{w} est choisi dans le groupe consistant en groupe benzyle, trityle, benzoyle et un groupe benzoyle qui peut être substitué en une quelconque position par au moins un groupe choisi dans le groupe consistant en atome de brome, de chlore, de fluor, d'iode, groupe alkyle en C₁₋₆ , alcoxy en C₁₋₆, nitro et trifluorométhyle.

13. Intermédiaire suivant la revendication 11, utile pour la production de 1,3-oxathiolanes dotés de propriétés anti-virales, choisi dans le groupe consistant en :
cis-2-benzoyloxyméthyl-4-(cytosin-1'-yl)-1,3-oxathiolane, trans-2-benzoyloxy-méthyl-4-(cytosin-1'-yl)-1,3-oxathiolane et leurs mélanges ;
cis-2-benzoyloxyméthyl-4-(N₄'-acétyl-cytosin-1'-yl)-1,3-oxathiolane, trans-2-benzoyloxyméthyl-4-(N₄'-acétyl-cytosin-1'-yl)-1,3-oxathiolane, et leurs mélanges ;
cis-2-benzoyloxyméthyl-4-(uracyl-1'-yl)-1,3-oxathiolane, trans-2-benzoyloxyméthyl-4-(uracil-1'-yl)-1,3-oxathiolane, et leurs mélanges ;
cis-2-benzoyloxyméthyl-4-(thymin-1'-yl)-1,3-oxathiolane, trans-2-benzoyloxyméthyl-4-(thymin-1'-yl)-1,3-oxathiolane, et leurs mélanges ;
cis-2-benzoyloxméthyl-4-(6'-chlorupurin-9'-yl)-1,3-oxathiolane, trans-2-benzoyloxyméthyl-4-(6'-chloropurin-9'-yl)-1,3-oxathiolane, et leurs mélange ;
cis-2-benzoyloxyméthyl-4-(adenin-9'-yl)-1,3-oxathiolane, trans-2-benzoyloxyméthyl-4-(adenin-9'-yl)-1,3-oxathiolane, et leurs mélanges;
cis-2-benzoyloxyméthyl-4-(2'-amino-6'-chloro-purin-9,-yl)-1,3-oxathiolane, trans-2-benzoyloxyméthyl-4-(2'-amino-6'-chloro--purin-9'-yl)-1,3-oxathiolane, et leurs mélanges.

14. Intermédiaire de formule (Id), isomères géométriques et optiques de celui-ci et mélanges de ces isomères utiles pour la production de 1,3-oxathiolane doté de propriétés anti-virales : 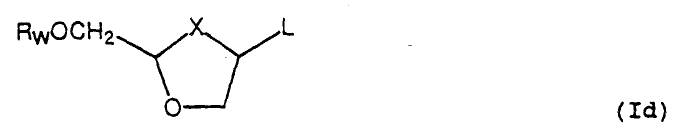 dans laquelle :
X est choisi dans le groupe consistant en S, S=O et SO₂.
R_{w} est choisi dans le groupe consistant en groupe silyle trisubstitué, groupe alkyle en C₁₋₆, aralkyle et acyle en C₁₋₁₆ ; et
L est un groupe mobile ou un atome d'hydrogène.

15. Intermédiaire suivant la revendication 14, dans laquelle R_{w} est choisi dans le groupe consistant en groupe benzyle, trityle, benzoyle et un groupe benzoyle qui peut être substitué dans une quelconque position par un groupe choisi dans le groupe consistant en atome de brome, de chlore, de fluor, d'iode, groupe alkyle en C₁₋₆, alcoxy en C₁₋₆, nitro et trifluorométhyle.

16. Intermédiaire suivant la revendication 14, utile pour la production de 1,.3-oxathiolane doté de propriétés anti-virales choisi dans le groupe consistant en :
2-benzoyloxyméthyl- 1,3-oxathiolane;
cis-2-benzoyloxyméthyl-1-oxo-1,3-oxathiolane, trans-2-benzoyloxyméthyl-1-oxo-1,3-oxathiolane, et leurs mélanges ;
cis-2-benzoyloxyméthyl-4-acétoxy-1,3-oxathiolane, trans-2-benzoyloxyméthyl-4-acétoxy-1,3-oxathiolane, et leurs mélanges.

17. Procédé pour la préparation d'un 1,3-oxathiolane de formule (I), des isomères géométriques et optiques de celui-ci et de mélanges de ces isomères : 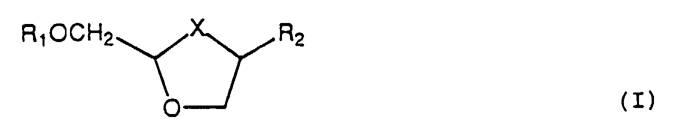 dans laquelle :
X est choisi dans le groupe consistant en S, S=O et SO₂.
R₁ est un atome d'hydrogène, et
R₂ est tel que défini dans les revendications 1 ou 2;
le procédé comprenant les étapes de :
(a) condensation d'un aldéhyde représenté par la formule
R_{w}OCH₂CHO
dans laquelle R_{w} est choisi dans le groupe consistant en groupe silyle trisubstitué, un groupe alkyle en C₁₋₆, aryle et acyle en C₁₋₁₆ ; avec du mercaptoéthanol dans un solvant organique contenant un catalyseur acide pour produire un intermédiaire de formule : 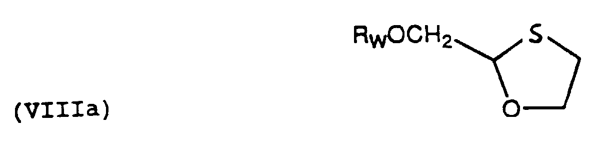
(b) traitement de l'intermédiaire de l'étape (a) avec un peracide pour donner un sulfoxyde correspondant ;
(c) traitement l'intermédiaire de l'étape (b) avec un anhydride de formule
(RₓCO)₂O
dans laquelle Rₓ est un groupe alkyle en C₁₋₆, en présence d'un tampon pour produire un intermédiaire de formule : 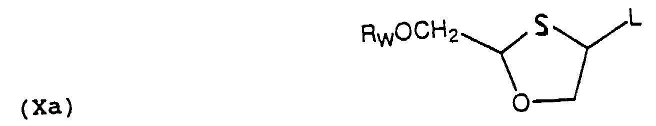 dans laquelle L est OCORₓ ou OH;
(d) traitement de l'intermédiaire de l'étape (c) avec une pyrimidine ou une purine base ou un analogue de celle-ci silylé approprié correspondant à R₂, en présence d'un acide de Lewis et hydrolyse de la fonction R_{w} pour produire un composé de formule (I) dans laquelle X est S ; et
(e) oxydation éventuelle du groupe X = S du composé de formule (I) obtenu à l'étape (d) pour obtenir un autre composé de formule (I) dans laquelle X est SO ou SO₂.

## Revendications (Revendications pour l'(les) Etat(s) contractant(s) suivant(s): ES, GR)

1. Procédé pour la préparation d'un intermédiaire de formule (Xa), des isomères géométriques et optiques de celui-ci et des mélanges de ces isomères, utiles pour la production de 1,3-oxathiolane doté de propriétés anti-virales : 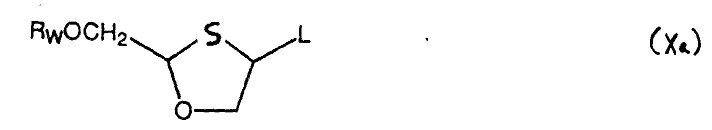 dans laquelle :
R_{w} est choisi dans le groupe consistant en groupe silyle trisubstitué, groupe alkyle en C₁₋₆, aralkyle et acyle en C₁₋₁₆ ; et
L est OCORₓ ou OH ;
Le procédé comprenant les étapes de :
(a) condensation d'un aldéhyde représenté par la formule
R_{w}OCH₂CHO
avec du mercaptoéthanol dans un solvant organique contenant un catalyseur acide pour produire un intermédiaire de formule : 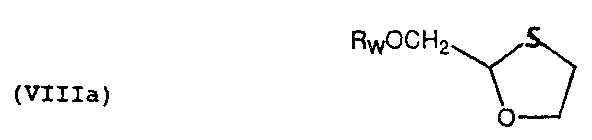
(b) traitement de l'intermédiaire de l'étape (a) avec un peracide pour donner un sulfoxyde correspondant ;
(c) traitement de l'intermédiaire de l'étape (b) avec un anhydride de formule
(RₓCO)₂O
dans laquelle Rₓ est un groupe alkyle en C₁₋₆, en présence d'un tampon.

2. Procédé suivant la revendication 1, pour la préparation d'un intermédiaire dans lequel R_{w} est choisi dans le groupe consistant en groupe benzyle, trityle, benzoyle et un groupe benzoyle qui peut être substitué en une position quelconque par au moins un groupe choisi dans le groupe consistant en atome de brome, de chlore, de fluor, d'iode, groupe alkyle en C₁₋₆, alcoxy en C₁₋₆, nitro et trifluorométhyle.

3. Procédé suivant la revendication 1, pour la préparation d'un intermédiaire utile pour la production de 1,3-oxathiolanes dotés de propriétés anti-virales, choisi dans le groupe consistant en :
2-benzoyloxyméthyl-1,3-oxathiolane ;
cis-2-benzoyloxyméthyl-1-oxo-1,3-oxathiolane, trans-2-benzoyloxyméthyl-1-oxo-1,3-oxathiolane, et leurs mélanges ;
cis-2-benzoyloxyméthyl-4-acétoxy-1,3-oxathiolane, trans-2-benzoyloxyméthyl-4-acétoxy-1,3-oxathiolane, et leurs mélanges.

4. Procédé pour la préparation d'un intermédiaire de formule (Ic'), ses isomères géométriques et optiques et des mélanges de ces isomères utiles pour la production de 1,3-oxathiolanes dotés de propriétés anti-virales : 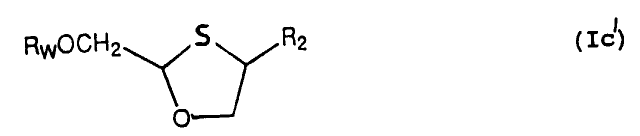 dans laquelle :
R_{w} est tel que défini dans la revendication 1,
R₂ est choisi dans le groupe consistant en : 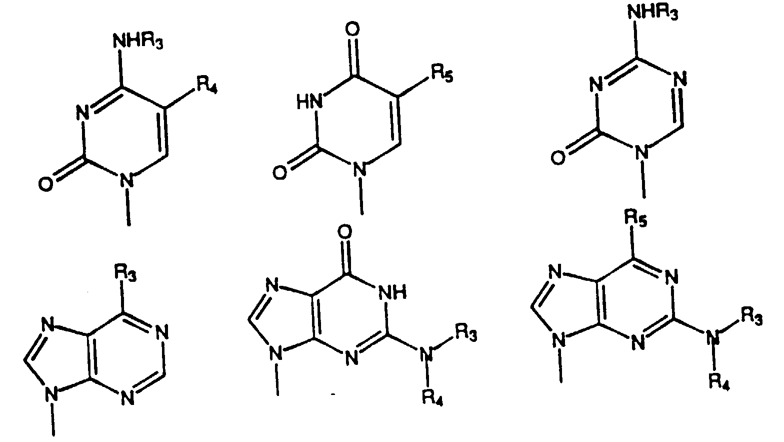 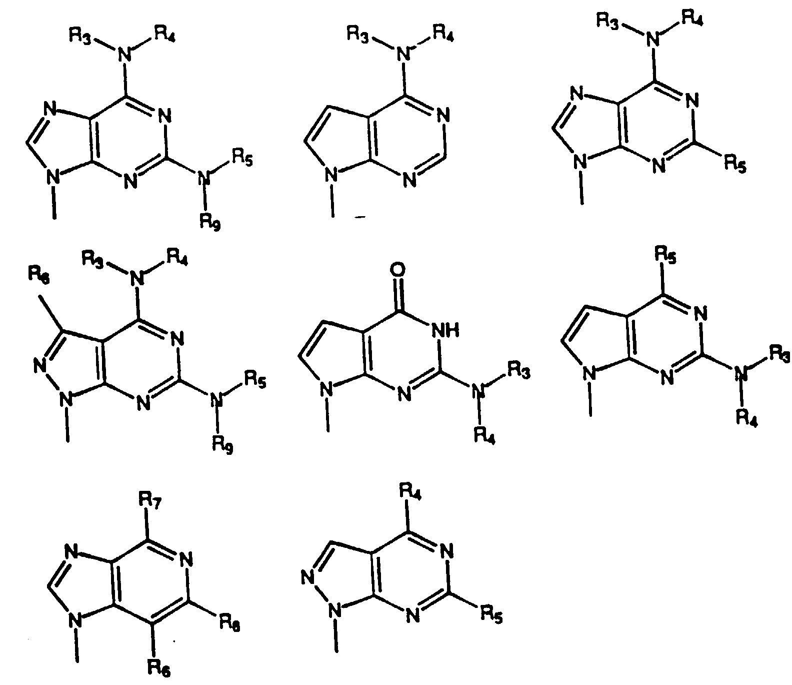
dans lesquelles :
R₃ est choisi dans le groupe consistant en atome d'hydrogène, groupe acyle en C₁₋₁₀, hydroxy, alkyle en C₁₋₆, alcényle ou alcynyle ayant jusqu'à 6 atomes de carbone ;
R₄ et R₅ sont indépendamment choisis dans le groupe consistant en atome d'hydrogène, groupe hydroxyméthyle, trifluorométhyle, alkyle en C₁₋₆, alcényle ou alcynyle ayant jusqu'à 6 atomes de carbone, atome de brome, de chlore, de fluor et d'iode ;
R₆ est choisi dans le groupe consistant en atome d'hydrogène, de brome, de chlore, de fluor, d'iode, groupe cyano, carboxy, carboxamide, éthoxycarbonyle, carbamoyle et thiocarbamoyle ;
R₇ et R₈ sont indépendamment choisis dans le groupe consistant en atome d'hydrogène, de brome, de chlore, de fluor, d'iode, groupe amino et hydroxy ; et
R₉ est choisi dans le groupe consistant en atome d'hydrogène, groupe acyle en C₁₋₁₀, hydroxy, alkyle en C₁₋₆, alcényle ou alcynyle ayant jusqu'à 6 atomes de carbone ;
le procédé comprenant un procédé suivant la revendication 1 et comprenant de plus l'étape de :
(d1) traitement de l'intermédiaire de l'étape (c) avec une pyrimidine ou une purine base ou un analogue de celle-ci silylé approprié correspondant à R₂ en présence d'un acide de Lewis.

5. Procédé suivant la revendication 4, dans lequel R₂ est choisi dans le groupe consistant en :

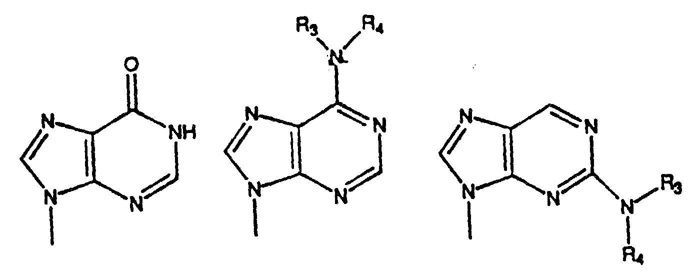

6. Procédé suivant les revendications 4 ou 5, pour la préparation d'un intermédiaire dans lequel R_{w} est choisi dans le groupe consistant en groupe benzyle, trityle, benzoyle et un groupe benzoyle qui peut être substitué en une quelconque position par au moins un groupe choisi dans le groupe consistant en atome de brome, de chlore, de fluor, d'iode, de groupe alkyle en C₁₋₆, alcoxy en C₁₋₆, nitro et trifluorométhyle.

7. Procédé suivant les revendications 4 ou 5, pour la préparation d'un intermédiaire utile pour la production de 1,3-oxathiolanes dotés de propriétés anti-virales choisi dans le groupe consistant en :
cis-2-benzoyloxyméthyl-4-(cytosin-1'-yl)-1,3 -oxathiolane, trans-2-benzoyloxy-méthyl-4-(cytosin-1'-yl)-1,3-oxathiolane et leurs mélanges ;
cis-2-benzoyloxyméthyl-4-(N₄'-acétyl-cytosin-1'-yl)-1,3-oxathiolane, trans-2-benzoyloxyméthyl-4-(N₄'-acétyl-cytosin-1'-yl)-1,3-oxathiolane, et leurs mélanges ;
cis-2-benzoyloxyméthyl-4-(uracyl-1'-yl)-1,3-oxathiolane, trans-2-benzoyloxyméthyl-4-(uracil-1'-yl)-1,3-oxathiolane, et leurs mélanges ;
cis-2-benzoyloxyméthyl-4-(thymin-1'-yl)-1,3-oxathiolane, trans-2-benzoyloxyméthyl-4-(thymin-1'-yl)-1,3-oxathiolane, et leurs mélanges ;
cis-2-benzoyloxyméthyl-4-(6'-chlorupurin-9'-yl)-1,3-oxathiolane, trans-2-benzoyloxyméthyl-4-(6'-chloropurin-9'-yl)-1,3-oxathiolane, et leurs mélange ;
cis-2-benzoyloxyméthyl-4-(adenin-9'-yl)-1,3-oxathiolane, trans-2-benzoyloxyméthyl-4-(adenin-9'-yl)-1,3-oxathiolane, et leurs mélanges;
cis-2-benzoyloxyméthyl-4-(2'-amino-6'-chloro-purin-9'-yl)-1,3-oxathiolane, trans-2-benzoyloxyméthyl-4-(2'-amino-6'-chloro--purin-9'-yl)-1,3-oxathiolane, et leurs mélanges.

8. Procédé pour la préparation d'un 1,3-oxathiolane de formule (I), de ses isomères géométriques et optiques, de mélanges de ces isomères et de sels, esters ou sels de ces esters de ceux-ci acceptables du point de vue pharmaceutique, qui lorsqu'ils sont administrés aux receveurs, sont capables de fournir (directement ou indirectement) un composé de formule (I) ou un métabolite ou un résidu de celui-ci actif du point de vue anti-viral : 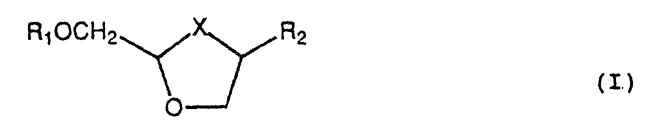 dans laquelle :
R₁ est un atome d'hydrogène;
X est choisi dans le groupe consistant en S, SO et SO₂ ;
R₂ est tel que défini dans les revendications 4 ou 5 ;
le procédé comprenant un procédé suivant les revendications 4 ou 5, et comprenant de plus les étapes de :
(d2) hydrolyse de la fonction R_{w} pour produire un composé de formule (I) dans laquelle X est S ; et
(e) oxydation éventuelle du groupe X = S du composé de formule (I) obtenu dans l'étape (D2) pour obtenir un autre composé de formule (I) dans laquelle X est SO ou SO₂.

9. Procédé suivant la revendication 8, dans lequel le composé obtenu est sous la forme de son isomère cis.

10. Procédé suivant la revendication 8, dans lequel le composé obtenu est choisi dans le groupe consistant en :
cis-2-hydroxyméthyl-4-(cytosin-1'-yl)-1,3-oxathiolane, trans-2-hydroxyméthyl-4-(cytosin-1'-yl)-1,3-oxathiolane et leurs mélanges ;
cis-2-hydroxyméthyl-4-(N₄'-acétyl-cytosin-1'-yl)-1,3-oxathiolane, trans-2-hydroxyméthyl-4-(N₄'-acétyl-cytosin-1'-yl)-1,3-oxathiolane, et leurs mélanges ;
cis-2-hydroxyméthyl-4-(uracyl-1'-yl)-1,3-oxathiolane, trans-2-hydroxyméthyl-4-(uracil-1'-yl)-1,3-oxathiolane, et leurs mélanges ;
cis-2-hydroxyméthyl-4-(thymin-1'-yl)-1,3-oxathiolane, trans-2-hydroxyméthyl-4-(thymin-1'-yl)-1,3-oxathiolane, et leurs mélanges ;
cis-2-hydroxyméthyl-4-(6'-chlorupurin-9'-yl)-1,3-oxathiolane, trans-2-hydroxyméthyl-4-(6'-chloropurin-9'-yl)-1,3-oxathiolane, et leurs mélange ;
cis-2-hydroxyméthyl-4-(adenin-9'-yl)-1,3-oxathiolane, trans-2-hydroxyméthyl-4-(adenin-9'-yl)-1,3-oxathiolane, et leurs mélanges;
cis-2-hydroxyméthyl-3-oxo-4-(adenin-9'-yl)-1,3oxathiolane;
cis-2-hydroxyméthyl-4-(6'-N-méthylamino-purin-9'-yl)-1,3oxathiolane;
cis-2-hydroxyméthyl-4-(6'-N,N-diméthylamino-purin-9'-yl)-1,3-oxathiolane, trans-2-hydroxyméthyl-4-(6'-N,N-diméthylamino-purin-9'-yl)-1,3-oxathiolane, et leurs mélanges ;
cis-2-hydroxyrnéthyl-4-(2'-amino-6'-chloro-purin-9'-yl)-1,3-oxathiolane, trans-2-hydroxyméthyl4-(2'-amino-6'-chloro--purin-9'-yl)-1,3-oxathiolane, et leurs mélanges ;
cis-2-hydroxyméthyl-4-(2'-6'-diamino-purin-9'-yl)-1,3-oxathiolane, trans-2-hydroxyméthyl-4-(2'-6'-diamino-purin-9'-yl)-1,3-oxathiolane, et leurs mélanges ;
cis-2-hydroxyméthyl-4-(guanin-9'-yl)-1,3-oxathiolane; et dérivés de ceux-ci acceptables du point de vue pharmaceutique sous la forme d'un mélange racémique ou d'un énantiomère unique.

11. Procédé suivant la revendication 8, dans lequel le composé obtenu est sous la forme d'un mélange racémique.

12. Procédé suivant la revendication 8, dans lequel le composé est pratiquement sous la forme d'un énantiomère unique.

13. Procédé suivant la revendication 8, pour la préparation d'un ester de formule (IV), des isomères géométriques et optiques de celui-ci et des mélanges de ces isomères : 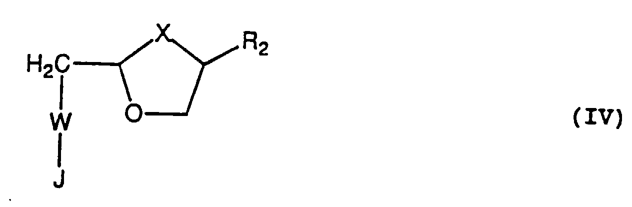 dans laquelle :
X et R₂ sont tels que définis dans la revendication 8,
W est choisi dans le groupe consistant en PO₄⁻, SPO₃⁻ et O-C(O)-(CH₂)ₙ-C(O)-O- dans lequel n est en entier de 1 à 10 ;
J est un quelconque nucléoside ou un analogue de nucléoside ou dérivé de ceux-ci ;
le procédé comprenant de plus l'étape de :
(f) estérification du composé de formule (I).

14. Procédé suivant la revendication 13, pour la préparation d'un ester dans lequel J est représenté par la formule :
